# EUROPEAN PATENT SPECIFICATION

(11) **EP 3 387 134 B1**
(45) Date of publication and mention of the grant of the patent: **14.10.2020**
(21) Application number: 16819766.3
(22) Date of filing: 06.12.2016
(51) Int. Cl.: C12N 15/63, C12N 15/90, C12N 15/10

(54) **METHODS AND COMPOSITIONS FOR ENHANCED NUCLEASE-MEDIATED GENOME MODIFICATION AND REDUCED OFF-TARGET SITE EFFECTS**
VERFAHREN UND ZUSAMMENSETZUNGEN FÜR VERBESSERTE NUKLEASEVERMITTELTE GENOMMODIFIZIERUNG UND VERRINGERTE OFF-TARGET-NEBENWIRKUNGEN
PROCÉDÉS ET COMPOSITIONS POUR MODIFICATION DE GÉNOME MÉDIÉE PAR NUCLÉASE AUGMENTÉE ET EFFETS HORS-SITE CIBLE RÉDUITS

(30) Priority: 11.12.2015 US 201562266051 P
(43) Date of publication of application: 17.10.2018
(73) Proprietor: Danisco US Inc., Palo Alto, California 94304 (US)
(72) Inventor: FRISCH, Ryan L., Palo Alto California 94304 (US)
(74) Representative: Mewburn Ellis LLP
(86) International application number: PCT/US2016/065070
(87) International publication number: WO 2017/100158

(56) References cited:
- WO-A1-2016/110511
- WO-A2-2005/095624
- FULLER K K ET AL: "Development of the CRISPR/Cas9 System for Targeted Gene Disruption in Aspergillus fumigatus", EUKARYOTIC CELL, AMERICAN SOCIETY FOR MICROBIOLOGY, US, vol. 14, no. 11, 28 August 2015 (2015-08-28), pages 1073-1080, XP002755315, ISSN: 1535-9778, DOI: 10.1128/EC.00107-15 [retrieved on 2015-08-28]
- CHRISTINA S. NØDVIG ET AL: "A CRISPR-Cas9 System for Genetic Engineering of Filamentous Fungi", PLOS ONE, vol. 10, no. 7, 15 July 2015 (2015-07-15), page e0133085, XP055256394, DOI: 10.1371/journal.pone.0133085
- RUI LIU ET AL: "Efficient genome editing in filamentous fungus Trichoderma reesei using the CRISPR/Cas9 system", CELL DISCOVERY, vol. 1, 12 May 2015 (2015-05-12), page 15007, XP055263032, DOI: 10.1038/celldisc.2015.7
- J. E. DICARLO ET AL: "Genome engineering in Saccharomyces cerevisiae using CRISPR-Cas systems", NUCLEIC ACIDS RESEARCH, vol. 41, no. 7, 4 March 2013 (2013-03-04), pages 4336-4343, XP055086617, ISSN: 0305-1048, DOI: 10.1093/nar/gkt135
- V. K. VYAS ET AL: "A Candida albicans CRISPR system permits genetic engineering of essential genes and gene families", SCIENCE ADVANCES, vol. 1, no. 3, 3 April 2015 (2015-04-03), pages e1500248-e1500248, XP055343721, DOI: 10.1126/sciadv.1500248
- VAN TRUNG CHU ET AL: "Increasing the efficiency of homology-directed repair for CRISPR-Cas9-induced precise gene editing in mammalian cells", NATURE BIOTECHNOLOGY, vol. 33, no. 5, 24 March 2015 (2015-03-24) , pages 543-548, XP055290254, US ISSN: 1087-0156, DOI: 10.1038/nbt.3198
- TAKESHI MARUYAMA ET AL: "Increasing the efficiency of precise genome editing with CRISPR-Cas9 by inhibition of nonhomologous end joining", NATURE BIOTECHNOLOGY, vol. 33, no. 5, 20 April 2015 (2015-04-20) , pages 538-542, XP055290186, US ISSN: 1087-0156, DOI: 10.1038/nbt.3190
- SUPRIYA V. VARTAK ET AL: "Inhibition of nonhomologous end joining to increase the specificity of CRISPR/Cas9 genome editing", FEBS JOURNAL, vol. 282, no. 22, 9 September 2015 (2015-09-09), pages 4289-4294, XP055342300, GB ISSN: 1742-464X, DOI: 10.1111/febs.13416
- JORDAN PINDER ET AL: "Nuclear domain 'knock-in' screen for the evaluation and identification of small molecule enhancers of CRISPR-based genome editing", NUCLEIC ACIDS RESEARCH, vol. 43, no. 19, 1 October 2015 (2015-10-01), pages 9379-9392, XP055342317, ISSN: 0305-1048, DOI: 10.1093/nar/gkv993
- JONATHAN VERBEKE ET AL: "Efficient homologous recombination with short length flanking fragments in Ku70 deficient Yarrowia lipolytica strains", BIOTECHNOLOGY LETTERS, vol. 35, no. 4, 9 December 2012 (2012-12-09), pages 571-576, XP055342472, NL ISSN: 0141-5492, DOI: 10.1007/s10529-012-1107-0
- ANNE KRETZSCHMAR ET AL: "Increased homologous integration frequency in Yarrowia lipolytica strains defective in non-homologous end-joining", CURRENT GENETICS, vol. 59, no. 1-2, 20 February 2013 (2013-02-20), pages 63-72, XP055342477, US ISSN: 0172-8083, DOI: 10.1007/s00294-013-0389-7
- CORY M. SCHWARTZ ET AL: "Synthetic RNA Polymerase III Promoters Facilitate High-Efficiency CRISPR-Cas9-Mediated Genome Editing in Yarrowia lipolytica", ACS SYNTHETIC BIOLOGY, vol. 5, no. 4, 29 December 2015 (2015-12-29), pages 356-359, XP055343808, USA ISSN: 2161-5063, DOI: 10.1021/acssynbio.5b00162
- GAO SHULIANG ET AL: "Multiplex gene editing of theYarrowia lipolyticagenome using the CRISPR-Cas9 system", JOURNAL OF INDUSTRIAL MICROBIOLOGY AND BIOTECHNOLOGY, BASINGSTOKE, GB, vol. 43, no. 8, 27 June 2016 (2016-06-27), pages 1085-1093, XP036000917, ISSN: 1367-5435, DOI: 10.1007/S10295-016-1789-8 [retrieved on 2016-06-27]

## Description

### FIELD

The disclosure relates to the field of molecular biology, in particular, to methods for altering the genome of a cell.

### BACKGROUND

Recombinant DNA technology has made it possible to insert DNA sequences at targeted genomic locations and/or modify (edit) specific endogenous chromosomal sequences, thus altering the organism's phenotype. Site-specific integration techniques, which employ site-specific recombination systems, as well as other types of recombination technologies, have been used to generate targeted insertions of genes of interest in a variety of organism. Genome-editing techniques such as designer zinc finger nucleases (ZFNs,), transcription activator-like effector nucleases (TALENs), homing meganucleases, engineered nucleases are available for producing targeted genome perturbations, but these systems tend to have a low specificity and employ designed nucleases that need to be redesigned for each target site, which renders them costly and time-consuming to prepare. CRISPR-associated (Cas) RNA-guided endonuclease systems have been developed as a means for introducing site-specific DNA strand breaks at specific target sites. These nuclease based systems can create a single strand or double strand break (DSB) in a target nucleotide, which can increase the frequency of homologous recombination at the target locus. A potential limitation of these nuclease systems is that they can exibit off-target activity.

Inhibition of gene expression can be accomplished, for example, by interrupting or deleting the DNA sequence of the gene, resulting in "knock-out" of the gene. Gene knock-outs mostly have been carried out through homologous recombination (HR), a technique applicable across a wide array of organisms from bacteria to mammals. Another tool for studying gene function can be through genetic "knock-in", which is also usually performed by HR. HR for purposes of gene targeting (knock-out or knock-in) can use the presence of an exogenously supplied DNA having homology with the target site. Although gene targeting by HR is a powerful tool, it can be a complex, labor-intensive procedure. Most studies using HR have generally been limited to knock-out of a single gene rather than multiple genes in a pathway, since HR is generally difficult to scale-up in a cost-effective manner. This difficulty is exacerbated in organisms in which HR is not efficient. Such low efficiency typically forces practitioners to rely on selectable phenotypes or exogenous markers to help identify cells in which a desired HR event occurred.

Vyas et al. (Sci. Adv. 2015;1:e15002483 April 2015; DOI: 10.1126/sciadv.1500248) describe a CRISPR system for use in targeting genes in *C. albicans.*

Thus there remains a need for new and more efficient genome engineering technologies that are affordable, easy to set up, scalable, and amenable to targeting multiple positions within the genome of an organism while limiting or eliminating any off-target site effects.

### BRIEF SUMMARY

Compositions and methods are provided for editing nucleotides or altering target sites in the genome of a cell. The methods and compositions employ a guide RNA/Cas endonuclease system and an inhibitor of a DNA Ligase IV (LIG4), wherein the inhibitor is Scr7, to provide an effective system for editing nucleotides or altering target sites within the genome of a cell. The present disclosure also describes methods for editing a nucleotide sequence in the genome of a non-conventional yeast cell employing a guide RNA/Cas endonuclease system and an inhibitor of a DNA Ligase IV (LIG4), wherein the inhibitor is Scr7.

In one embodiment of the disclosure, the method comprises a method for altering a target site in the genome of a non-conventional yeast cell, the method comprising providing to a non-conventional yeast cell at least one guide RNA, at least one Cas endonuclease capable of introducing a double strand break at said target site, and an inhibitor of a DNA Ligase IV (LIG4), wherein the inhibitor is Scr7. The cells can be pretreated by being exposed to a medium comprising said inhibitor and/or activator at a concentration of at least 0.5 microMolar, for at least 6 hrs, at a temperature of at least 20 °C prior to providing said guide RNA and said Cas endonuclease to said cell.

In another embodiment of the disclosure, the method comprises a method for editing a nucleotide sequence in the genome of a non-conventional yeast cell, the method comprising providing to a non-conventional yeast cell at least one guide RNA, at least one polynucleotide modification template comprising at least one nucleotide modification of said nucleotide sequence, at least one Cas endonuclease capable of introducing a double strand break at a target site in the genome of said non-conventional yeast cell, and an inhibitor of a DNA Ligase IV (LIG4), wherein the inhibitor is Scr7.

In another embodiment of the disclosure, the method comprises a method for selecting a non-conventional yeast cell comprising an altered target sequence, the method comprising: a) providing to a non-conventional yeast cell at least one guide RNA, at least one Cas endonuclease capable of introducing a double strand break at a target site in the genome of said non-conventional yeast cell, and an inhibitor of a DNA Ligase IV (LIG4), wherein the inhibitor is Scr7,' b) evaluating the non-conventional yeast cell of (a) for off-target site effects as well as for the presence of said altered target sequence; and, c) selecting a non-conventional yeast cell from (b) that has said altered target site while having reduced or no off-target site effects.

In another embodiment of the disclosure, the method comprises a method for selecting an edited non-conventional yeast cell, the method comprising: a) providing to a non-conventional yeast cell comprising a nucleotide sequence to be edited, at least one guide RNA, at least one polynucleotide modification template comprising at least one nucleotide modification of said nucleotide sequence, at least one Cas endonuclease capable of introducing a double strand break at a target site in the genome of said non-conventional yeast cell, and an inhibitor of a DNA Ligase IV (LIG4), wherein the inhibitor is Scr7; b) evaluating the non-conventional yeast cell of (a) for off-target site effects as well as for the presence said at least one nucleotide modification of said nucleotide sequence; and,c) selecting a non-conventional yeast cell from (b) that has said at least one nucleotide modification of said nucleotide sequence while having reduced or no off-target site effects.

In another embodiment of the disclosure, the method comprises a method for selecting a non-conventional yeast cell comprising a polynucleotide of interest inserted into a target site in its genome, the method comprising: a) providing to a non-conventional yeast cell, at least one guide RNA, at least one polynucleotide donor DNA comprising a polynucleotide of interest, at least one Cas endonuclease capable of introducing a double strand break at a target site in the genome of said non-conventional yeast cell, and an inhibitor of a DNA Ligase IV (LIG4), wherein the inhibitor is Scr7; b) evaluating the non-conventional yeast cell of (a) for off-target site effects as well as for the presence said at least one polynucleotide of interest; and,c) selecting a non-conventional yeast cell from (b) that has said at least one polynucleotide of interest while having reduced or no off-target site effects.

The alteration at said target site can be selected from the group of (i) at least one nucleotide deletion, (ii) at least one nucleotide substitution, (iii) at least one nucleotide insertion, or (iv) any one combination of (i)-(iii).

In one embodiment of the disclosure, the method further comprises determining the frequency of Homologous Directed Repair (HDR) and/or Non-Homologous End Joining (NHEJ) in said cell. The frequency of HDR can be increased when compared to the frequency of HDR derived from a control method lacking the inhibitor. The frequency of NHEJ can be decreased when compared to the frequency of NHEJ derived from a control method lacking the least one inhibitor.

Also described are nucleic acid constructs, and non-conventional yeast cells, produced by the methods described herein. Additional embodiments of the methods and compositions of the present disclosure are shown herein.

### BRIEF DESCRIPTION OF THE DRAWINGS AND THE SEQUENCE LISTING

The disclosure can be more fully understood from the following detailed description and the accompanying drawings and Sequence Listing, which form a part of this application. The sequence descriptions and sequence listing attached hereto comply with the rules governing nucleotide and amino acid sequence disclosures in patent applications as set forth in 37 C.F.R. §§1.821-1.825. The sequence descriptions contain the three letter codes for amino acids as defined in 37 C.F.R. §§ 1.821-1.825.

### Figures

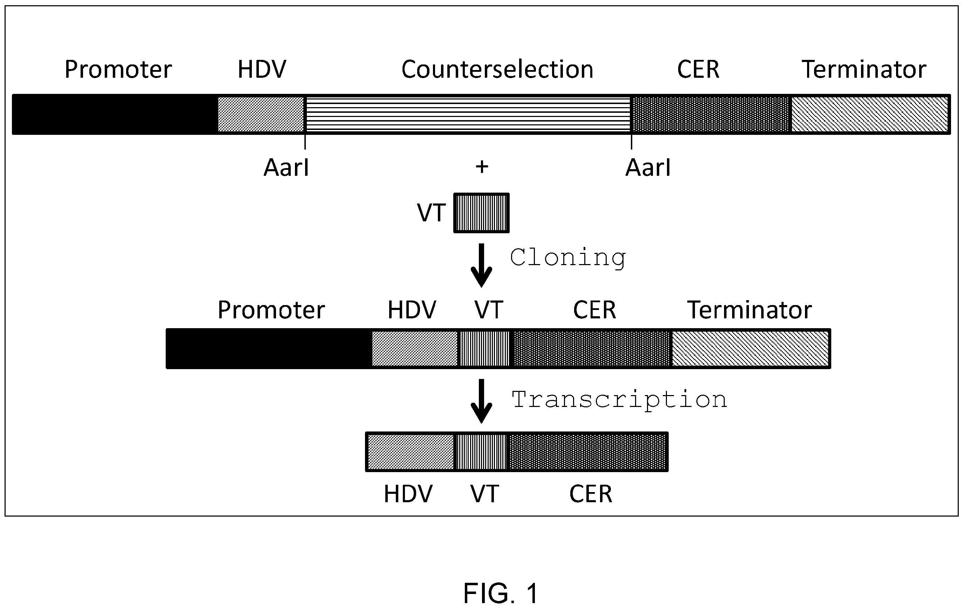

**Figure 1** depicts the structure of a high throughput gRNA cloning cassette (for example, but not limiting to, SEQ ID NO: 12 on pRF291. The cassette is composed of a promoter (shown in solid black), DNA encoding a 5' ribozyme (shown in solid gray), a counter selection cassette flanked by two restriction sites (shown in horizontal line fill), a DNA encoding the CER domain (shown as CER) and a transcriptional terminator (dot fill). When a DNA duplex containing a variable targeting domain with the correct overhanging ends (VT, shown as vertical stripe fill) is mixed with a plasmid containing a cassette in the presence of the restriction enzyme and DNA ligase, the counterselection cassette (horizontal stripe fill) can be replaced by the VT domain (Vertical stripe). These events can be selected *in vitro* by selecting for the absence of the counter selection cassette. The product is a functional gRNA expression cassette.

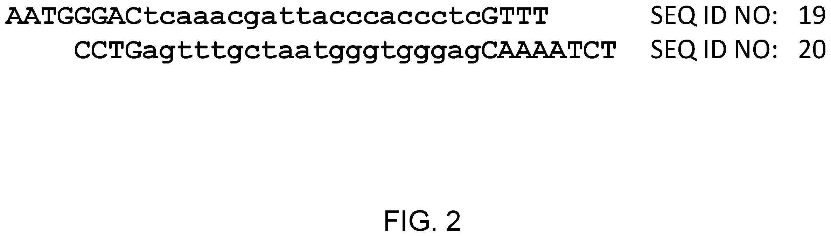

**Figure 2** depicts a variable targeting domain duplex (SEQ ID NO: 19 and SEQ ID NO: 20) for use with the high-throughput plasmid pRF291 comprising SEQ ID NO: 19 and SEQ ID NO: 20)

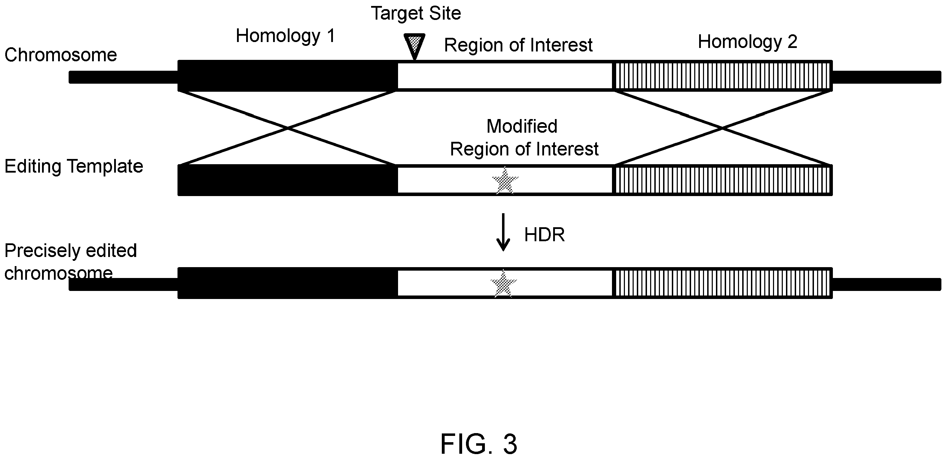

**Figure 3** depicts precise genome editing using a polynucleotide modification template (editing template) to repair a Cas9 induced DNA double strand break (DSB). The region of interest (white box) contained on the native chromosomal DNA contains a target site for Cas9 (gray triangle) and is flanked by two regions, homology arm 1 (black box), and homology arm 2 (vertical stripe fill). When a Cas9 induced DSB occurs, the damage can be repaired using homology directed repair (HDR) and an exogenously provided DNA editing template that contains both homology arm 1(black box) and homology arm 2 (vertical stripe box) and a desired edit (gray star), but lacks the Cas9 target site (gray triangle). The last step of HDR is double holiday junction resolution indicated by the two crossed lines between homology arms of the chromosome and the editing template. The resulting precisely edited chromosome now contains the modified region of interest with the desired edits and lacks the Cas9 target site.

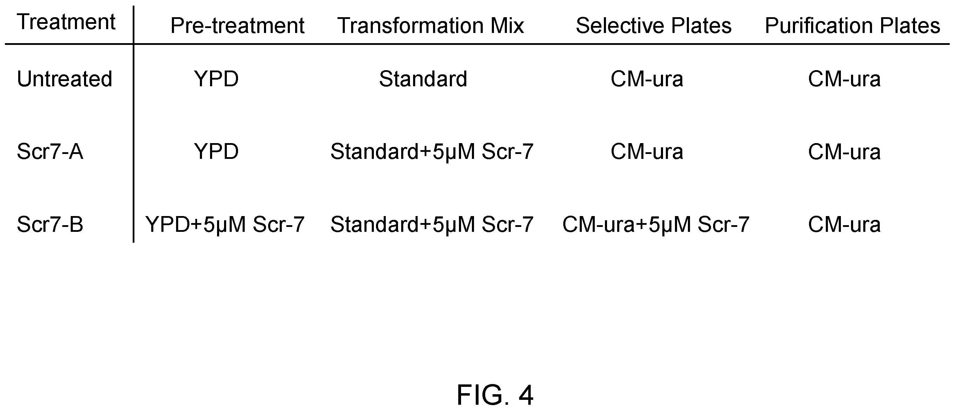

**Figure 4** depicts a schematic of the three different treatment conditions using Scr-7 in this study. Cells were grown for 24 hours under pretreatment conditions at 30°C. Mixed with DNA in transformation mix at 39°C for 1 hour. Recovered on selective plates for 48 hours at 30°C, and streak purified on purification plates for single colonies for 48 hours at 30°C. YPD contains1% Yeast extract, 2% Peptone, 2% dextrose solidified with 1.5% w/v agar. Standard transformation mix contains 35% w/v PEG₃₅₅₀, 100mM Lithium acetate, 100 mM DTT, 10mM Tris pH 6.0, 1mM EDTA. CM-ura is complete minimal medium lacking uracil solidified with 1.5% w/v agar.

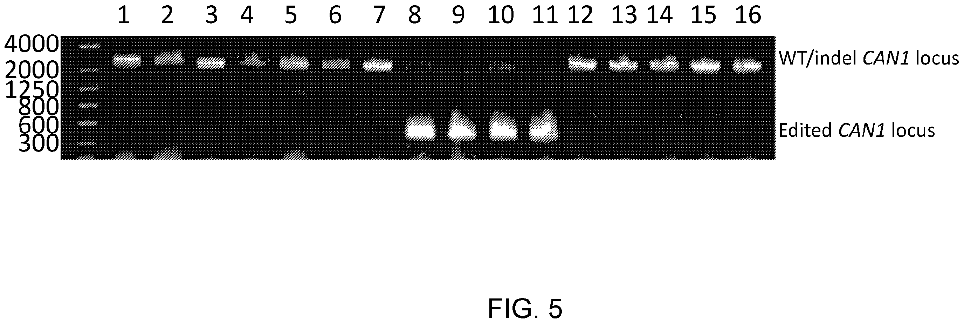

**Figure 5** depicts an example of PCR evaluation of the *CAN1* locus to determine if HDR between editing template (SEQ ID NO: 34) and chromosomal *CAN1* locus (SEQ ID NO: 35) has occurred. Lane 1 is molecular weight marker (molecular weight indicated in base pairs on side). WT and indel *CAN1* locus band (SEQ ID NO: 40) and precisely edited *CAN1* locus (SEQ ID NO: 41) bands are indicated. Lane 2 through 17 contain individual colonies from purification from cells treated with pRF303 (SEQ ID NO: 24), *CAN1* deletion editing template (SEQ ID NO: 34), and Scr-7 treatment B.

### Sequences

**Table 1**

| **Summary of Nucleic Acid and Protein SEQ ID Numbers** | | |
|---|---|---|
| Description | Nucleic acid SEQ ID NO. | Protein SEQ ID NO. |
| Cas9 endonuclease, *Streptococcus pyogenes* | 1 | |
| Yarrowia codon optimized Cas9 | 2 | |
| SV40 Nuclear localization signal | | 3 |
| FBA1 promoter | 4 | |
| Yarrowia optimized expression cassette | 5 | |
| pZufCas9 | 6 | |
| Aarl-removal 1 primer | 7 | |
| Aarl-removal 2 primer | 8 | |
| pRF109 | 9 | |
| Aar1- Cas9 ORF (Aar1-Cas9CG gene) | 10 | |
| pRF141 | 11 | |
| high-throughput cloning cassette | 12 | |
| yl52 promoter | 13 | |
| DNA encoding the HDV ribozyme | 14 | |
| rpsL counterselectable marker | 15 | |
| DNA encoding Cas9 CER domain | 16 | |
| SUP4 terminator | 17 | |
| pRF291 | 18 | |
| Can1-1F | 19 | |
| Can1-1R | 20 | |
| DNA encoding Can1-1 VT domain | 21 | |
| Can1-1target site | 22 | |
| CAN1 gene, *Yarrowia lipolytica* | 23 | |
| pRF303 | 24 | |
| can1 upstream homology arm | 25 | |
| Can1 upstream forward | 26 | |
| Can1 upstream reverse | 27 | |
| Can1 downstream homology arm | 28 | |
| Can1 downstream homology arm forward primer | 29 | |
| Can1 downstream homology arm reverse primer | 30 | |
| Can1 polynucleotide modification template (editing template) cloning fragment | 31 | |
| pUC18 | 32 | |
| pRF80 | 33 | |
| Can1 polynucleotide modification template (editing deletion template) | 34 | |
| CAN1 locus (colony PCR) | 35 | |
| editing template forward primer | 36 | |
| editing template reverse primer | 37 | |
| Can1 locus Forward | 38 | |
| Can1 locus reverse | 39 | |
| Can1 locus WT | 40 | |
| Can1 locus deletion | 41 | |
| Copy number analysis fragment | 42 | |
| Can1 copy number F | 43 | |
| Can1 copy number R | 44 | |
| Can1 copy number probe (6FAM-CTTTTCGCCCCCACTGCAGCC-TAMRA) | 45 | |
| TEF1 locus | 46 | |
| TEF1 forward | 47 | |
| TEF1 reverse | 48 | |
| TEF1 probe (6FAM-TGCTGGTGGTGTTGGTGAGTT-TAMRA) | 49 | |

### DETAILED DESCRIPTION

Compositions and methods are provided for editing nucleotides or altering target sites in the genome of a cell. The methods and compositions employ a guide RNA/Cas endonuclease system and an inhibitor of a DNA Ligase IV (LIG4), wherein the inhibitor is Scr7, to provide an effective system for editing nucleotides or altering target sites within the genome of a cell. Further provided are methods for editing a nucleotide sequence in the genome of a non-conventional yeast cell employing a guide RNA/Cas endonuclease system and an inhibitor of a DNA Ligase IV (LIG4), wherein the inhibitor is Scr7.

CRISPR (clustered regularly interspaced short palindromic repeats) loci refers to certain genetic loci encoding factors of class I, II, or III DNA cleavage systems, for example, used by bacterial and archaeal cells to destroy foreign DNA (Horvath and Barrangou, 2010, Science 327:167-170). Components of CRISPR systems are taken advantage of herein in a heterologous manner for DNA targeting in cells.

The type II CRISPR/Cas system from bacteria employs a crRNA (CRISPR RNA) and tracrRNA (trans-activating CRISPR RNA) to guide the Cas endonuclease to its DNA target. The crRNA contains a region complementary to one strand of the double strand DNA target and a region that base pairs with the tracrRNA (trans-activating CRISPR RNA) forming a RNA duplex that directs the Cas endonuclease to cleave the DNA target. CRISPR systems belong to different classes, with different repeat patterns, sets of genes, and species ranges. The number of CRISPR-associated genes at a given CRISPR locus can vary between species (Haft et al. (2005) Computational Biology, PLoS Comput Biol 1(6): e60. doi:10.1371/journal.pcbi.0010060).

The term "Cas gene" herein refers to a gene that is generally coupled, associated or close to, or in the vicinity of flanking CRISPR loci. The terms "Cas gene", "CRISPR-associated (Cas) gene" are used interchangeably herein. The term "Cas endonuclease" herein refers to a protein encoded by a Cas gene. A Cas endonuclease herein, when in complex with a suitable polynucleotide component, is capable of recognizing, binding to, and optionally nicking or cleaving all or part of a specific DNA target sequence. A Cas endonuclease described herein comprises one or more nuclease domains. Cas endonucleases of the disclosure include those having a HNH or HNH-like nuclease domain and / or a RuvC or RuvC-like nuclease domain. A Cas endonuclease of the disclosure includes a Cas9 protein, a Cpf1 protein, a C2c1 protein, a C2c2 protein, a C2c3 protein, Cas3, Cas 5, Cas7, Cas8, Cas10, or complexes of these.

As used herein, the terms "guide polynucleotide/Cas endonuclease complex", "guide polynucleotide/Cas endonuclease system", " guide polynucleotide/Cas complex", "guide polynucleotide/Cas system", "guided Cas system" are used interchangeably herein and refer to at least one guide polynucleotide and at least one Cas endonuclease that are capable of forming a complex, wherein said guide polynucleotide/Cas endonuclease complex can direct the Cas endonuclease to a DNA target site, enabling the Cas endonuclease to recognize, bind to, and optionally nick or cleave (introduce a single or double strand break) the DNA target site. A guide polynucleotide/Cas endonuclease complex herein can comprise Cas protein(s) and suitable polynucleotide component(s) of any of the four known CRISPR systems (Horvath and Barrangou, 2010, Science 327:167-170) such as a type I, II, or III CRISPR system. A Cas endonuclease unwinds the DNA duplex at the target sequence and optionally cleaves at least one DNA strand, as mediated by recognition of the target sequence by a polynucleotide (such as, but not limited to, a crRNA or guide RNA) that is in complex with the Cas protein. Such recognition and cutting of a target sequence by a Cas endonuclease typically occurs if the correct protospacer-adjacent motif (PAM) is located at or adjacent to the 3' end of the DNA target sequence. Alternatively, a Cas protein herein may lack DNA cleavage or nicking activity, but can still specifically bind to a DNA target sequence when complexed with a suitable RNA component. (See also U.S. Patent Application US 2015-0082478 A1, published on March 19, 2015 and US 2015-0059010 A1, published on February 26, 2015).

A guide polynucleotide/Cas endonuclease complex can cleave one or both strands of a DNA target sequence. A guide polynucleotide/Cas endonuclease complex that can cleave both strands of a DNA target sequence typically comprises a Cas protein that has all of its endonuclease domains in a functional state (e.g., wild type endonuclease domains or variants thereof retaining some or all activity in each endonuclease domain). Thus, a wild type Cas protein (e.g., a Cas9 protein disclosed herein), or a variant thereof retaining some or all activity in each endonuclease domain of the Cas protein, is a suitable example of a Cas endonuclease that can cleave both strands of a DNA target sequence. A Cas9 protein comprising functional RuvC and HNH nuclease domains is an example of a Cas protein that can cleave both strands of a DNA target sequence. A guide polynucleotide/Cas endonuclease complex that can cleave one strand of a DNA target sequence can be characterized herein as having nickase activity (e.g., partial cleaving capability). A Cas nickase typically comprises one functional endonuclease domain that allows the Cas to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain. Non-limiting examples of Cas9 nickases suitable for use herein are disclosed in U.S. Patent Appl. Publ. No. 2014/0189896.

A pair of Cas9 nickases can be used to increase the specificity of DNA targeting. In general, this can be done by providing two Cas9 nickases that, by virtue of being associated with RNA components with different guide sequences, target and nick nearby DNA sequences on opposite strands in the region for desired targeting. Such nearby cleavage of each DNA strand creates a double strand break (i.e., a DSB with single-stranded overhangs), which is then recognized as a substrate for non-homologous-end-joining, NHEJ (prone to imperfect repair leading to mutations) or homologous recombination, HR. Each nick in these embodiments can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair. For example, a Cas9 nickase with a mutant RuvC domain, but functioning HNH domain (i.e., Cas9 HNH+/RuvC-), could be used (e.g., *Streptococcus pyogenes* Cas9 HNH+/RuvC-). Each Cas9 nickase (e.g., Cas9 HNH+/RuvC-) would be directed to specific DNA sites nearby each other (up to 100 base pairs apart) by using suitable RNA components herein with guide RNA sequences targeting each nickase to each specific DNA site.

A Cas protein can be part of a fusion protein comprising one or more heterologous protein domains (e.g., 1, 2, 3, or more domains in addition to the Cas protein). Such a fusion protein may comprise any additional protein sequence, and optionally a linker sequence between any two domains, such as between Cas and a first heterologous domain. Examples of protein domains that may be fused to a Cas protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A Cas protein herein can be from any of the following genera: *Aeropyrum, Pyrobaculum, Sulfolobus, Archaeoglobus, Haloarcula, Methanobacteriumn, Methanococcus, Methanosarcina, Methanopyrus, Pyrococcus, Picrophilus, Thernioplasnia, Corynebacterium, Mycobacterium, Streptomyces, Aquifrx, Porphvromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myrococcus, Campylobacter, Wolinella, Acinetobacter, Erwinia, Escherichia, Legionella, Methylococcus, Pasteurella, Photobacterium, Salmonella, Xanthomonas, Yersinia, Streptococcus, Treponema, Francisella,* or *Thermotoga.* See also US patent applications 62/162,377 filed May 15, 2015 and 62/162,353 filed May 15, 2015 for more examples of Cas proteins.

A guide polynucleotide/Cas endonuclease complex in certain embodiments can bind to a DNA target site sequence, but does not cleave any strand at the target site sequence. Such a complex may comprise a Cas protein in which all of its nuclease domains are mutant, dysfunctional. For example, a Cas9 protein herein that can bind to a DNA target site sequence, but does not cleave any strand at the target site sequence, may comprise both a mutant, dysfunctional RuvC domain and a mutant, dysfunctional HNH domain. A Cas protein herein that binds, but does not cleave, a target DNA sequence can be used to modulate gene expression, for example, in which case the Cas protein could be fused with a transcription factor (or portion thereof) (e.g., a repressor or activator, such as any of those disclosed herein).

The Cas endonuclease gene herein can encode a Type II Cas9 endonuclease such as but not limited to, Cas9 genes listed in SEQ ID NOs: 462, 474, 489, 494, 499, 505, and 518 of WO2007/025097, published March 1, 2007. In another embodiment, the Cas endonuclease gene is a microbe or optimized Cas9 endonuclease gene. The Cas endonuclease gene can be operably linked to a SV40 nuclear targeting signal upstream of the Cas codon region and a bipartite VirD2 nuclear localization signal (Tinland et al. (1992) Proc. Natl. Acad. Sci. USA 89:7442-6) downstream of the Cas codon region.

The Cas endonuclease gene includes a plant or microbial codon optimized *Streptococcus pyogenes* Cas9 gene that can recognize any genomic sequence of the form N(12-30)NGG can in principle be targeted or a Cas9 endonuclease originated from an organism selected from the group consisting of *Brevibacillus laterosporus, Lactobacillus reuteri* Mlc3, *Lactobacillus rossiae* DSM 15814, *Pediococcus pentosaceus* SL4, *Lactobacillus nodensis* JCM 14932, *Sulfurospirillum sp.* SCADC, *Bifidobacterium thermophilum* DSM 20210, *Loktanella vestfoldensis, Sphingomonas sanxanigenens* NX02, *Epilithonimonas tenax* DSM 16811, *Sporocytophaga myxococcoides* and *Psychroflexus torquis* ATCC 700755, wherein said Cas9 endonuclease can form a guide RNA/Cas endonuclease complex capable of recognizing, binding to, and optionally nicking or cleaving all or part of a DNA target sequence. Other Cas endonuclease systems have been described in US patent applications 62/162,377 filed May 15, 2015 and 62/162,353 filed May 15, 2015.

"Cas9" (formerly referred to as Cas5, Csn1, or Csx12) herein refers to a Cas endonuclease of a type II CRISPR system that forms a complex with a crNucleotide and a tracrNucleotide, or with a single guide polynucleotide, for specifically recognizing and cleaving all or part of a DNA target sequence. Cas9 protein comprises a RuvC nuclease domain and an HNH (H-N-H) nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al, Cell 157:1262-1278). A type II CRISPR system includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA.

The amino acid sequence of a Cas9 protein described herein, as well as certain other Cas proteins herein, may be derived from a *Streptococcus* (e.g., *S*. *pyogenes, S. pneumoniae, S. thermophilus, S. agalactiae, S. parasanguinis, S. oralis, S. salivarius, S*. *macacae, S. dysgalactiae, S. anginosus, S. constellatus, S. pseudoporcinus, S. mutans*), *Listeria* (e.g., *L. innocua*), *Spiroplasma* (e.g., *S*. *apis, S. syrphidicola*), *Peptostreptococcaceae, Atopobium, Porphyromonas* (e.g., *P. catoniae), Prevotella* (e.g., *P. intermedia), Veillonella, Treponema* (e.g., *T. socranskii, T. denticola*), *Capnocytophaga, Finegoldia* (e.g., *F. magna*), *Coriobacteriaceae* (e.g., *C. bacterium*), *Olsenella* (e.g., *O. profusa*), *Haemophilus* (e.g., *H. sputorum, H. pittmaniae*), *Pasteurella* (e.g., *P. bettyae*), *Olivibacter* (e.g., *O. sitiensis*), *Epilithonimonas* (e.g., E. *tenax*), *Mesonia* (e.g., *M. mobilis*), *Lactobacillus* (e.g., *L. plantarum*), *Bacillus* (e.g., *B. cereus*), *Aquimarina* (e.g., *A. muelleri*), *Chryseobacterium* (e.g., *C. palustre*), *Bacteroides* (e.g., *B. graminisolvens*), *Neisseria* (e.g., *N. meningitidis*), *Francisella* (e.g., *F. novicida*), or *Flavobacterium* (e.g., *F. frigidarium, F. soli*) species, for example. As another example, a Cas9 protein can be any of the Cas9 proteins disclosed in Chylinski et al. (RNA Biology 10:726-737 and US patent application 62/162377, filed May 15, 2015).

Accordingly, the sequence of a Cas9 protein herein can comprise, for example, any of the Cas9 amino acid sequences disclosed in GenBank Accession Nos. G3ECR1 (*S. thermophilus*), WP_026709422, WP_027202655, WP_027318179, WP_027347504, WP_027376815, WP_027414302, WP_027821588, WP_027886314, WP_027963583, WP_028123848, WP_028298935, Q03JI6 (*S*. *thermophilus*), EGP66723, EGS38969, EGV05092, EHI65578 (*S. pseudoporcinus*), EIC75614 (*S. oralis*), EID22027 (*S. constellatus*), EIJ69711, EJP22331 (*S. oralis*), EJP26004 (*S. anginosus*), EJP30321, EPZ44001 (*S. pyogenes*), EPZ46028 (*S. pyogenes*), EQL78043 (*S. pyogenes*), EQL78548 (*S. pyogenes*), ERL10511, ERL12345, ERL19088 (*S. pyogenes*), ESA57807 (*S*. *pyogenes*), ESA59254 (*S. pyogenes*), ESU85303 (*S. pyogenes*), ETS96804, UC75522, EGR87316 (*S*. *dysgalactiae*), EGS33732, EGV01468 (*S. oralis*), EHJ52063 (*S*. *macacae*), EID26207 (*S*. *oralis*), EID33364, EIG27013 (*S*. *parasanguinis*), EJF37476, EJO19166 (*Streptococcus* sp. BS35b), EJU16049, EJU32481, YP_006298249, ERF61304, ERK04546, ETJ95568 (*S. agalactiae*), TS89875, ETS90967 (*Streptococcus* sp. SR4), ETS92439, EUB27844 (*Streptococcus* sp. BS21), AFJ08616, EUC82735 (*Streptococcus* sp. CM6), EWC92088, EWC94390, EJP25691, YP_008027038, YP_008868573, AGM26527, AHK22391, AHB36273, Q927P4, G3ECR1, or Q99ZW2 (*S*. *pyogenes*). A variant of any of these Cas9 protein sequences may be used, but should have specific binding activity, and optionally endonucleolytic activity, toward DNA when associated with an RNA component herein. Such a variant may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9.

Alternatively, a Cas9 protein may comprise an amino acid sequence that is at least about 80%, 81 %, 82%, 83%, 84%, 85%, 86%, 87%, 88%, 89%, 90%, 91%, 92%, 93%, 94%, 95%, 96%, 97%, 98%, or 99% identical to any of the foregoing amino acid sequences, for example. Such a variant Cas9 protein should have specific binding activity, and optionally cleavage or nicking activity, toward DNA when associated with an RNA component herein.

A Cas protein herein such as a Cas9 can comprise a heterologous nuclear localization sequence (NLS). A heterologous NLS amino acid sequence herein may be of sufficient strength to drive accumulation of a Cas protein in a detectable amount in the nucleus of a yeast cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas amino acid sequence but such that it is exposed on the protein surface. An NLS may be operably linked to the N-terminus or C-terminus of a Cas protein herein, for example. Two or more NLS sequences can be linked to a Cas protein, for example, such as on both the N- and C-termini of a Cas protein. Non-limiting examples of suitable NLS sequences herein include those disclosed in U.S. Patent No. 7,309,576.

The Cas endonuclease can comprise a modified form of the Cas9 polypeptide. The modified form of the Cas9 polypeptide can include an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide (US patent application US20140068797 A1, published on March 6, 2014). In some cases, the modified form of the Cas9 polypeptide has no substantial nuclease activity and is referred to as catalytically "inactivated Cas9" or "deactivated cas9 (dCas9)." Catalytically inactivated Cas9 variants include Cas9 variants that contain mutations in the HNH and RuvC nuclease domains. These catalytically inactivated Cas9 variants are capable of interacting with sgRNA and binding to the target site in vivo but cannot cleave either strand of the target DNA.

A catalytically inactive Cas9 can be fused to a heterologous sequence (US patent application US2014/0068797 A1, published on March 6, 2014). Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cas9 can also be fused to a Fokl nuclease to generate double strand breaks (Guilinger et al. Nature biotechnology, volume 32, number 6, June 2014).

The terms "functional fragment ", "fragment that is functionally equivalent" and "functionally equivalent fragment" of a Cas endonuclease are used interchangeably herein, and refer to a portion or subsequence of the Cas endonuclease sequence of the present disclosure in which the ability to recognize, bind to, and optionally nick or cleave (introduce a single or double strand break in) the target site is retained.

The terms "functional variant", "Variant that is functionally equivalent" and "functionally equivalent variant" of a Cas endonuclease are used interchangeably herein, and refer to a variant of the Cas endonuclease of the present disclosure in which the ability to recognize, bind to, and optionally nick or cleave (introduce a single or double strand break in) the target site is retained. Fragments and variants can be obtained via methods such as site-directed mutagenesis and synthetic construction.

Any guided endonuclease can be used in the methods disclosed herein. Such endonucleases include, but are not limited to Cas9 and Cpf1 endonucleases. Many endonucleases have been described to date that can recognize specific PAM sequences (see for example Jinek et al. (2012) Science 337 p 816-821, US Patent Application Nos. 62/162377, filed May 15, 2015 and 62/162353, filed May 15, 2015 and Zetsche B et al. 2015. Cell 163, 1013) and cleave the target DNA at a specific position. It is understood that based on the methods and embodiments described herein utilizing a guided Cas system one can now tailor these methods such that they can utilize any guided endonuclease system.

A potential limitation of nuclease systems is that they may exhibit off-target site activity (introduction of single strand or double strand breaks at an unintended site). Off-target site activity of an endonuclease includes cleavage of off-target sites that are not fully complementary to the guide. In non-conventional yeast, traditional HDR can occur using at least a donor DNA (such as a selectable marker) flanked by two homology arms to insert the selectable marker in the chromosome between the two regions of homology. However, due to the preference for NHEJ in these organisms, the donor DNA can often be inserted randomly in the genome (in unintended sites in the genome) and substantial screening can be required to find the desired HDR product that is inserted in the intended target site.

The term "off-target site effects" and "off-target effects" are used interchangeably and include any alteration in an off-target site that is due to the activity of an endonuclease cleavage, wherein the alteration includes, for example: (i) a replacement of at least one nucleotide, (ii) a deletion of at least one nucleotide, (iii) an insertion of at least one nucleotide, or (iv) any combination of (i) - (iii), as well as any integration of a template or donor DNA at an unintended site. The unintended site can be any site in the genome of the organism that is not the target site.

Several approaches have been explored to improve the specificity and decrease off-target site effects of Cas endonucleases, including reducing the amount of enzyme active in the cell, shortening the section of the guide RNA complementary to the target, deploying pairs of engineered nicking Cas9s (Nicolas et al. Human Gene Therapy. 2015, 26(7): 425-431), and structure-guided protein engineering (Slaymaker et al. Science. 2015. Science DOI: 10.1126/science.aad5227). Many of these approaches remain to have limitations, often decreasing on-target editing efficiency.

Described herein are methods for decreasing off-target site effects in a cell while remaining and/or increasing on-target editing efficiency (*see* Examples 2 and 3) using a small molecule inhibitor of a DNA Ligase IV (LIG4), wherein the inhibitor is Scr7.

The endonuclease can be provided to a cell by any method known in the art, for example, but not limited to transient introduction methods, transfection, microinjection, and/or topical application or indirectly via recombination constructs. The endonuclease can be provided as a protein or as a guided polynucleotide complex directly to a cell or indirectly via recombination constructs. The endonuclease can be introduced into a cell transiently or can be incorporated into the genome of the host cell using any method known in the art. Uptake of the endonuclease and/or the guided polynucleotide into the cell can be facilitated with a Cell Penetrating Peptide (CPP) as described in US application 62/075999, filed November 06, 2014.

Endonucleases are enzymes that cleave the phosphodiester bond within a polynucleotide chain, and include restriction endonucleases that cleave DNA at specific sites without damaging the bases. Restriction endonucleases include Type I, Type II, Type III, and Type IV endonucleases, which further include subtypes. In the Type I and Type III systems, both the methylase and restriction activities are contained in a single complex. Endonucleases also include meganucleases, also known as homing endonucleases (HEases), which like restriction endonucleases, bind and cut at a specific recognition site, however the recognition sites for meganucleases are typically longer, about 18 bp or more (patent application PCT/US12/30061, filed on March 22, 2012). Meganucleases have been classified into four families based on conserved sequence motifs, the families are the LAGLIDADG, GIY-YIG, H-N-H, and His-Cys box families. These motifs participate in the coordination of metal ions and hydrolysis of phosphodiester bonds. HEases are notable for their long recognition sites, and for tolerating some sequence polymorphisms in their DNA substrates. The naming convention for meganuclease is similar to the convention for other restriction endonuclease. Meganucleases are also characterized by prefix F-, I-, or PI- for enzymes encoded by free-standing ORFs, introns, and inteins, respectively. One step in the recombination process involves polynucleotide cleavage at or near the recognition site. This cleaving activity can be used to produce a double-strand break. For reviews of site-specific recombinases and their recognition sites, see, Sauer (1994) Curr Op Biotechnol 5:521-7; and Sadowski (1993) FASEB 7:760-7. In some examples the recombinase is from the Integrase or Resolvase families.

TAL effector nucleases (TALEN) are a class of sequence-specific nucleases that can be used to make double-strand breaks at specific target sequences in the genome of a plant or other organism. (Miller et al. (2011) Nature Biotechnology 29:143-148). Zinc finger nucleases (ZFNs) are engineered double-strand break inducing agents comprised of a zinc finger DNA binding domain and a double-strand-break-inducing agent domain. Recognition site specificity is conferred by the zinc finger domain, which typically comprising two, three, or four zinc fingers, for example having a C2H2 structure, however other zinc finger structures are known and have been engineered. Zinc finger domains are amenable for designing polypeptides which specifically bind a selected polynucleotide recognition sequence. ZFNs include an engineered DNA-binding zinc finger domain linked to a non-specific endonuclease domain, for example nuclease domain from a Type IIs endonuclease such as Fokl. Additional functionalities can be fused to the zinc-finger binding domain, including transcriptional activator domains, transcription repressor domains, and methylases. In some examples, dimerization of nuclease domain is required for cleavage activity. Each zinc finger recognizes three consecutive base pairs in the target DNA. For example, a 3 finger domain recognized a sequence of 9 contiguous nucleotides, with a dimerization requirement of the nuclease, two sets of zinc finger triplets are used to bind an 18 nucleotide recognition sequence.

As used herein, the term "guide polynucleotide", relates to a polynucleotide sequence that can form a complex with a Cas endonuclease and enables the Cas endonuclease to recognize, bind to, and optionally cleave a DNA target site. The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" (See also U.S. Patent Application US 2015-0082478 A1, published on March 19, 2015 and US 2015-0059010 A1, published on February 26, 2015).

The guide polynucleotide can be a double molecule (also referred to as duplex guide polynucleotide) comprising a crNucleotide sequence and a tracrNucleotide sequence. The crNucleotide includes a first nucleotide sequence domain (referred to as Variable Targeting domain or VT domain) that can hybridize to a nucleotide sequence in a target DNA and a second nucleotide sequence (also referred to as a tracr mate sequence) that is part of a Cas endonuclease recognition (CER) domain. The tracr mate sequence can hybridized to a tracrNucleotide along a region of complementarity and together form the Cas endonuclease recognition domain or CER domain. The CER domain is capable of interacting with a Cas endonuclease polypeptide. The crNucleotide and the tracrNucleotide of the duplex guide polynucleotide can be RNA, DNA, and/or RNA-DNA- combination sequences. In some embodiments, the crNucleotide molecule of the duplex guide polynucleotide is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the cRNA naturally occurring in Bacteria and Archaea. The size of the fragment of the cRNA naturally occurring in Bacteria and Archaea that can be present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some embodiments the tracrNucleotide is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides. In one embodiment, the RNA that guides the RNA/ Cas9 endonuclease complex is a duplexed RNA comprising a duplex crRNA-tracrRNA.
The tracrRNA (trans-activating CRISPR RNA) contains, in the 5'-to-3' direction, (i) a sequence that anneals with the repeat region of CRISPR type II crRNA and (ii) a stem loop-containing portion (Deltcheva et al., Nature 471:602-607). The duplex guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) into the target site. (See also U.S. Patent Application US 2015-0082478 A1, published on March 19, 2015 and US 2015-0059010 A1, published on February 26, 2015.)

The guide polynucleotide can also be a single molecule (also referred to as single guide polynucleotide) comprising a crNucleotide sequence linked to a tracrNucleotide sequence. The single guide polynucleotide comprises a first nucleotide sequence domain (referred to as Variable Targeting domain or VT domain) that can hybridize to a nucleotide sequence in a target DNA and a Cas endonuclease recognition domain (CER domain), that interacts with a Cas endonuclease polypeptide. By "domain" it is meant a contiguous stretch of nucleotides that can be RNA, DNA, and/or RNA-DNA-combination sequence. The VT domain and /or the CER domain of a single guide polynucleotide can comprise a RNA sequence, a DNA sequence, or a RNA-DNA-combination sequence. The single guide polynucleotide being comprised of sequences from the crNucleotide and the tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). The single guide polynucleotide can form a complex with a Cas endonuclease, wherein said guide polynucleotide/Cas endonuclease complex (also referred to as a guide polynucleotide/Cas endonuclease system) can direct the Cas endonuclease to a genomic target site, enabling the Cas endonuclease to recognize, bind to, and optionally nick or cleave (introduce a single or double strand break) the target site. (See also U.S. Patent Application US 2015-0082478 A1, published on March 19, 2015 and US 2015-0059010 A1, published on February 26, 2015.)

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides in length. In some embodiments, the variable targeting domain comprises a contiguous stretch of 12 to 30 nucleotides. The variable targeting domain can be composed of a DNA sequence, a RNA sequence, a modified DNA sequence, a modified RNA sequence, or any combination thereof.

The term "Cas endonuclease recognition domain" or "CER domain" (of a guide polynucleotide) is used interchangeably herein and includes a nucleotide sequence that interacts with a Cas endonuclease polypeptide. A CER domain comprises a tracrNucleotide mate sequence followed by a tracrNucleotide sequence. The CER domain can be composed of a DNA sequence, a RNA sequence, a modified DNA sequence, a modified RNA sequence (see for example US 2015/0059010 A1, published on February 26, 2015), or any combination thereof.

The nucleotide sequence linking the crNucleotide and the tracrNucleotide of a single guide polynucleotide can comprise a RNA sequence, a DNA sequence, or a RNA-DNA combination sequence. In one embodiment, the nucleotide sequence linking the crNucleotide and the tracrNucleotide of a single guide polynucleotide can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In another embodiment, the nucleotide sequence linking the crNucleotide and the tracrNucleotide of a single guide polynucleotide can comprise a tetraloop sequence, such as, but not limiting to a GAAA tetraloop sequence.

Nucleotide sequence modification of the guide polynucleotide, VT domain and/or CER domain can be selected from, but not limited to , the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

The terms "functional fragment ", "fragment that is functionally equivalent" and "functionally equivalent fragment" of a guide RNA, crRNA or tracrRNA are used interchangeably herein, and refer to a portion or subsequence of the guide RNA, crRNA or tracrRNA, respectively, of the present disclosure in which the ability to function as a guide RNA, crRNA or tracrRNA, respectively, is retained.

The terms "functional variant ", "Variant that is functionally equivalent" and "functionally equivalent variant" of a guide RNA, crRNA or tracrRNA (respectively) are used interchangeably herein, and refer to a variant of the guide RNA, crRNA or tracrRNA, respectively, of the present disclosure in which the ability to function as a guide RNA, crRNA or tracrRNA, respectively, is retained.

The terms "single guide RNA" and "sgRNA" are used interchangeably herein and relate to a synthetic fusion of two RNA molecules, a crRNA (CRISPR RNA) comprising a variable targeting domain (linked to a tracr mate sequence that hybridizes to a tracrRNA), fused to a tracrRNA (trans-activating CRISPR RNA). The single guide RNA can comprise a crRNA or crRNA fragment and a tracrRNA or tracrRNA fragment of the type II CRISPR/Cas system that can form a complex with a type II Cas endonuclease, wherein said guide RNA/Cas endonuclease complex can direct the Cas endonuclease to a DNA target site, enabling the Cas endonuclease to recognize, bind to, and optionally nick or cleave (introduce a single or double strand break) the DNA target site.

The terms "guide RNA/Cas endonuclease complex", "guide RNA/Cas endonuclease system", " guide RNA/Cas complex", "guide RNA/Cas system", "gRNA/Cas complex", "gRNA/Cas system", "RNA-guided endonuclease" , "RGEN" are used interchangeably herein and refer to at least one RNA component and at least one Cas endonuclease that are capable of forming a complex , wherein said guide RNA/Cas endonuclease complex can direct the Cas endonuclease to a DNA target site, enabling the Cas endonuclease to recognize, bind to, and optionally nick or cleave (introduce a single or double strand break) the DNA target site. A guide RNA/Cas endonuclease complex herein can comprise Cas protein(s) and suitable RNA component(s) of any of the four known CRISPR systems (Horvath and Barrangou, 2010, Science 327:167-170) such as a type I, II, or III CRISPR system. A guide RNA/Cas endonuclease complex can comprise a Type II Cas9 endonuclease and at least one RNA component (e.g., a crRNA and tracrRNA, or a gRNA). (See also U.S. Patent Application US 2015-0082478 A1, published on March 19, 2015 and US 2015-0059010 A1, published on February 26, 2015).

The guide polynucleotide can be introduced into a cell transiently, as single stranded polynucleotide or a double stranded polynucleotide, using any method known in the art such as, but not limited to, particle bombardment, *Agrobacterium transformation* or topical applications. The guide polynucleotide can also be introduced indirectly into a cell by introducing a recombinant DNA molecule (via methods such as, but not limited to, particle bombardment or *Agrobacterium transformation*) comprising a heterologous nucleic acid fragment encoding a guide polynucleotide, operably linked to a specific promoter that is capable of transcribing the guide RNA in said cell. The specific promoter can be, but is not limited to, a RNA polymerase III promoter, which allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161; see also US application 62/036652, filed on August 13, 2014.

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, or any other DNA molecule in the genome (including chromosomal, choloroplastic, mitochondrial DNA, plasmid DNA) of a cell, at which a guide polynucleotide/Cas endonuclease complex can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. Cells include, but are not limited to, human, non-human, animal, bacterial, fungal, insect, yeast, non-conventional yeast, and plant cells as well as plants and seeds produced by the methods described herein. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (*i.e.,* a non-endogenous or non-native position) in the genome of a cell.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, or (iv) any combination of (i) - (iii).

Methods for "modifying a target site" and for "altering a target site" are used interchangeably herein and refer to methods for producing an altered target site.

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, wherein the active variants retain biological activity and hence are capable of being recognized and cleaved by an Cas endonuclease. Assays to measure the single or double-strand break of a target site by an endonuclease are known in the art and generally measure the overall activity and specificity of the agent on DNA substrates containing recognition sites.

A "protospacer adjacent motif" (PAM) herein refers to a short nucleotide sequence adjacent to a target sequence (protospacer) that is recognized (targeted) by a guide polynucleotide/Cas endonuclease system described herein. The Cas endonuclease may not successfully recognize a target DNA sequence if the target DNA sequence is not followed by a PAM sequence. The sequence and length of a PAM herein can differ depending on the Cas protein or Cas protein complex used. The PAM sequence can be of any length but is typically 0, 1, 2, 3, 4, 5, 6, 7, 8, 9, 10, 11, 12, 13, 14, 15, 16, 17, 18, 19 or 20 nucleotides long.

The terms "targeting", "gene targeting" and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with an endonuclease associated with a suitable polynucleotide component. Such DNA cleavage, if a double-strand break (DSB), can prompt NHEJ or HDR processes which can lead to modifications at the target site.

A targeting method herein can be performed in such a way that two or more DNA target sites are targeted in the method, for example. Such a method can optionally be characterized as a multiplex method. Two, three, four, five, six, seven, eight, nine, ten, or more target sites can be targeted at the same time in certain embodiments. A multiplex method is typically performed by a targeting method herein in which multiple different RNA components are provided, each designed to guide an guide polynucleotide/Cas endonuclease complex to a unique DNA target site. (US application 62/036652, filed on August 13, 2014.)

The terms "knock-out", "gene knock-out" and "genetic knock-out" are used interchangeably herein. A knock-out represents a DNA sequence of a cell that has been rendered partially or completely inoperative by targeting with a Cas protein; such a DNA sequence prior to knock-out could have encoded an amino acid sequence, or could have had a regulatory function (e.g., promoter), for example. A knock-out may be produced by an indel (insertion or deletion of nucleotide bases in a target DNA sequence through NHEJ), or by specific removal of sequence that reduces or completely destroys the function of sequence at or near the targeting site.

The guide polynucleotide/Cas endonuclease system can be used in combination with a co-delivered polynucleotide modification template to allow for editing (modification) of a genomic nucleotide sequence of interest. (See also U.S. Patent Application US 2015-0082478 A1, published on March 19, 2015 and WO2015/026886 A1, published on February 26, 2015.)

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution, addition or deletion. Optionally, the polynucleotide modification template can further comprise homologous nucleotide sequences flanking the at least one nucleotide modification, wherein the flanking homologous nucleotide sequences provide sufficient homology to the desired nucleotide sequence to be edited.

Genome editing can be accomplished using any method of gene editing available. For example, gene editing can be accomplished through the introduction into a host cell of a polynucleotide modification template (sometimes also referred to as a gene repair oligonucleotide) containing a targeted modification to a gene within the genome of the host cell. The polynucleotide modification template for use in such methods can be either single-stranded or double-stranded. Examples of such methods are generally described, for example, in US Publication No. 2013/0019349.

In some embodiments, gene editing may be facilitated through the induction of a double-stranded break (DSB) in a defined position in the genome near the desired alteration. DSBs can be induced using any DSB-inducing agent available, including, but not limited to, TALENs, meganucleases, zinc finger nucleases, Cas9-gRNA systems (based on bacterial CRISPR-Cas systems), and the like. In some embodiments, the introduction of a DSB can be combined with the introduction of a polynucleotide modification template.

The process for editing a genomic sequence combining DSB and modification templates generally comprises: providing to a host cell, a DSB-inducing agent, or a nucleic acid encoding a DSB-inducing agent, that recognizes a target sequence in the chromosomal sequence and is able to induce a DSB in the genomic sequence, and at least one polynucleotide modification template comprising at least one nucleotide alteration when compared to the nucleotide sequence to be edited. The polynucleotide modification template can further comprise nucleotide sequences flanking the at least one nucleotide alteration, in which the flanking sequences are substantially homologous to the chromosomal region flanking the DSB. Genome editing using DSB-inducing agents, such as Cas9-gRNA complexes, has been described, for example in U.S. Patent Application US 2015-0082478 A1, published on March 19, 2015, WO2015/026886 A1, published on February 26, 2015, US application 62/023246, filed on July 07, 2014, and US application 62/036,652, filed on August 13, 2014.

The terms "knock-in", "gene knock-in, "gene insertion" and "genetic knock-in" are used interchangeably herein. A knock-in represents the replacement or insertion of a DNA sequence at a specific DNA sequence in cell by targeting with a Cas protein (by HR, wherein a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cas endonuclease. Such methods can employ homologous recombination to provide integration of the polynucleotide of Interest at the target site. In one method provided, a polynucleotide of interest is provided to the organism cell in a donor DNA construct. As used herein, "donor DNA" is a DNA construct that comprises a polynucleotide of Interest to be inserted into the target site of a Cas endonuclease. The donor DNA construct further comprises a first and a second region of homology that flank the polynucleotide of Interest. The first and second regions of homology of the donor DNA share homology to a first and a second genomic region, respectively, present in or flanking the target site of the cell or organism genome. By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also be described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular Biology--Hybridization with Nucleic Acid Probes, (Elsevier, New York).

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

Polynucleotides of interest and/or traits can be stacked together in a complex trait locus as described in US 2013/0263324-A1, published October 3, 2013 and in PCT/US13/22891, published January 24, 2013. The guide polynucleotide/Cas9 endonuclease system described herein provides for an efficient system to generate double strand breaks and allows for traits to be stacked in a complex trait locus.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some embodiments the regions of homology share significant sequence homology to the genomic sequence immediately flanking the target site, it is recognized that the regions of homology can be designed to have sufficient homology to regions that may be further 5' or 3' to the target site. In still other embodiments, the regions of homology can also have homology with a fragment of the target site along with downstream genomic regions. In one embodiment, the first region of homology further comprises a first fragment of the target site and the second region of homology comprises a second fragment of the target site, wherein the first and second fragments are dissimilar.

As used herein, "homologous recombination" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also species-variable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem. 79:181-211). The most common form of HDR is called homologous recombination (HR), which has the longest sequence homology requirements between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels. (2014) PNAS (0027-8424), 111 (10), p. E924-E932).

Alteration of the genome of a plant cell, for example, through homologous recombination (HR), is a powerful tool for genetic engineering. Homologous recombination has been demonstrated in plants (Halfter et al., (1992) Mol Gen Genet 231:186-93) and insects (Dray and Gloor, 1997, Genetics 147:689-99). Homologous recombination has also been accomplished in other organisms. For example, at least 150-200 bp of homology was required for homologous recombination in the parasitic protozoan Leishmania (Papadopoulou and Dumas, (1997) Nucleic Acids Res 25:4278-86). In the filamentous fungus *Aspergillus nidulans,* gene replacement has been accomplished with as little as 50 bp flanking homology (Chaveroche et al., (2000) Nucleic Acids Res 28:e97). Targeted gene replacement has also been demonstrated in the ciliate *Tetrahymena thermophila* (Gaertig et al., (1994) Nucleic Acids Res 22:5391-8). In mammals, homologous recombination has been most successful in the mouse using pluripotent embryonic stem cell lines (ES) that can be grown in culture, transformed, selected and introduced into a mouse embryo (Watson et al., 1992, Recombinant DNA, 2nd Ed., (Scientific American Books distributed by WH Freeman & Co.).

Error-prone DNA repair mechanisms can produce mutations at double-strand break sites. The Non-Homologous-End-Joining (NHEJ) pathways are the most common repair mechanism to bring the broken ends together (Bleuyard et al., (2006) DNA Repair 5:1-12). The structural integrity of chromosomes is typically preserved by the repair, but deletions, insertions, or other rearrangements are possible. The two ends of one double-strand break are the most prevalent substrates of NHEJ (Kirik et al., (2000) EMBO J 19:5562-6), however if two different double-strand breaks occur, the free ends from different breaks can be ligated and result in chromosomal deletions (Siebert and Puchta, (2002) Plant Cell 14:1121-31), or chromosomal translocations between different chromosomes (Pacher et al., (2007) Genetics 175:21-9). Microhomology-mediated end joining MMEH is described in US patent application US2014/0242702, published on August 28, 2014.

Inhibitors of non-homologous end joining (NHEJ) are known in the art and include molecules, such as but not limited to small molecules that inhibits (decrease) the binding or activity of a DNA-dependent- protein kinase catalytic subunit (DNA-PKcs), a Poly(ADP-ribose) polymerase 1/2 (PARPI/2), a PARPI, Ku70/80, a DNA-PKcs, a XRCC4/XLF, a Ligase IV, a Ligase III, a XRCCI, an Artemis Polynucleotide Kinase (PNK), and any one combinations thereof (Sfeir et al. 2015, TIBS Vol 40 (11), pp701-713, US patent application US2014/0242702, published on August 28, 2014). Other molecules that decrease the activity of the non-homologous end joining (NHEJ) DNA repair complex are known in the art and include RNAi-molecules, antisense nucleic acid molecules, ribozymes, compounds inhibiting the formation of a functional DNA Ligase IV (LIG4) complex and compounds enhancing proteolytic degradation of a functional DNA Ligase IV complex (US patent application 2014/0304847, published on Oct 9, 2014.

Small molecules include low molecular weight (<900 Daltons) organic compounds with a size on the order of 10⁻⁹ m that may help regulate biological processes. A molecular weight smaller than 900 Daltons, may allow these molecules to rapidly diffuse across cell membranes so that they can reach intracellular sites of action. Small molecules can activate or block certain DNA repair pathway (Srivastava et al., 2012, Cell 151 (7):1474-87). Methods and composition for increasing the effectiveness of an albumin specific ZFN mediated gene targeting in Hepa 1-6 cells, by inhibiting the NHEJ cellular repair via PARP1 and DNA-PKcs inhibitors, have been described in US patent application 2014/0242702. The efficiency of HDR mediated genome editing with CRIPSR-Cas9 in mammalian cells can be increased by targeting DNA ligase IV, KU70 or KU80, all key enzymes in the mammalian NHEJ pathway (Maruyama et al., 2015, Nature Biotechnology, Vol. 33 (5) pg. 538-542; Chu et al. Nature Biotechnology Vol. 33 (5): 543-548). DNA ligase IV is responsible for sealing of DSBs in mammalian cells during NHEJ and can be inhibited by a small molecule referred to as SCR7 (Srivastava et al. 2012. Cell 151: 1474-1487. Other small molecules such as L755507, a potent and selective beta-3-adrenergic receptor partial agonist (Parmee et al.1998, Bioorg. Med. Chem. Lett. 8, 1107-1112), and Brefeldin A, an inhibitor of intracellular protein transportation (Ktistakis et al., 1992, Nature 356, 344-346), have been reported to improve the HDR efficiency in pluripotent stem cells when combined with Cas9-gRNA (Yu et al. 2015, Cell Stem Cell 16, 142-147). A small molecule comprising 6-Amino-2,3-dihydro-5-[(phenylmethylene)]amino]-2-4(1H)-pyrimidineone) has been evaluated for its effects on NHEJ DNA repair in mammalian cells (US patent application 2014/0304847, published on Oct 9, 2014). RS-1 may stimulate HR (on Rad51), Proc. SCR7 is the inhibitor used in the methods of the invention.

Described herein are methods for enhancing HDR and / or decreasing off-target site effects in a cell using SCR7.

In one embodiment of the disclosure, the method comprises a method for altering a target site in the genome of a non-conventional yeast cell, the method comprising providing to a non-conventional yeast cell at least one guide RNA, at least one Cas endonuclease capable of introducing a double strand break at said target site, and an inhibitor of a DNA Ligase IV (LIG4), wherein the inhibitor is Scr7. The cells can be pretreated by being exposed to a medium comprising said inhibitor at a concentration of at least 0.5 microMolar, for at least 6 hrs, at a temperature of at least 20°C prior to providing said guide RNA and said Cas endonuclease to said cell.

It is understood by anyone skilled in the art that the Cas endonuclease used in the methods described herein can be substituted by any double strand break inducing agent such us but not limited to TAL nucleases (TALENs), designer zinc-finger nucleases, engineered meganucleases and homing meganucleases.

Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95). Once the sequence around the double-strand breaks is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

Once a double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1-12). The structural integrity of chromosomes is typically preserved by the repair, but deletions, insertions, or other rearrangements are possible (Siebert and Puchta, (2002) Plant Cell 14:1121-31; Pacher et al., (2007) Genetics 175:21-9).

Alternatively, the double-strand break can be repaired by homologous recombination between homologous DNA sequences. Once the sequence around the double-strand break is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

DNA double-strand breaks appear to be an effective factor to stimulate homologous recombination pathways (Puchta et al., (1995) Plant Mol Biol 28:281-92; Tzfira and White, (2005) Trends Biotechnol 23:567-9; Puchta, (2005) J Exp Bot 56:1-14). Using DNA-breaking agents, a two- to nine-fold increase of homologous recombination was observed between artificially constructed homologous DNA repeats in plants (Puchta et al., (1995) Plant Mol Biol 28:281-92). In maize protoplasts, experiments with linear DNA molecules demonstrated enhanced homologous recombination between plasmids (Lyznik et al., (1991) Mol Gen Genet 230:209-18).

The donor DNA may be introduced by any means known in the art. The donor DNA may be provided by any transformation method known in the art including, for example, Agrobacterium-mediated transformation or biolistic particle bombardment. The donor DNA may be present transiently in the cell or it could be introduced via a viral replicon. In the presence of the Cas endonuclease and the target site, the donor DNA is inserted into the transformed plant's genome. (see guide language)

Further uses for guide RNA/Cas endonuclease systems have been described (See U.S. Patent Application US 2015-0082478 A1, published on March 19, 2015, WO2015/026886 A1, published on February 26, 2015, US 2015-0059010 A1, published on February 26, 2015, US application 62/023246, filed on July 07, 2014, and US application 62/036,652, filed on August 13, 2014) and include but are not limited to modifying or replacing nucleotide sequences of interest (such as a regulatory elements), insertion of polynucleotides of interest, gene knock-out, gene-knock in, modification of splicing sites and/or introducing alternate splicing sites, modifications of nucleotide sequences encoding a protein of interest, amino acid and/or protein fusions, and gene silencing by expressing an inverted repeat into a gene of interest.

Polynucleotides of interest are further described herein and include polynucleotides reflective of the commercial markets and interests of those involved in the development of the crop. Polynucleotides/polypeptides of interest include, but are not limited to, herbicide-resistance coding sequences, insecticidal coding sequences, nematicidal coding sequences, antimicrobial coding sequences, antifungal coding sequences, antiviral coding sequences, abiotic and biotic stress tolerance coding sequences, or sequences modifying plant traits such as yield, grain quality, nutrient content, starch quality and quantity, nitrogen fixation and/or utilization, fatty acids, and oil content and/or composition.

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in plants. Methods for suppressing gene expression in plants using polynucleotides in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, generally greater than about 65% sequence identity, about 85% sequence identity, or greater than about 95% sequence identity. See, U.S. Patent Nos. 5,283,184 and 5,034,323.

The polynucleotide of interest can also be a phenotypic marker. A phenotypic marker is screenable or a selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

As used herein, "nucleic acid" means a polynucleotide and includes a single or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Open reading frame" is abbreviated ORF.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of genes to produce the desired phenotype in a transformed plant. Genes can be designed for use in suppression by linking a nucleic acid fragment or subfragment thereof, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid fragments wherein changes in one or more amino acids or nucleotide bases do not affect the function of the molecule, such as the ability to mediate gene expression or to produce a certain phenotype. These terms also refer to modification(s) of nucleic acid fragments that do not substantially alter the functional properties of the resulting nucleic acid fragment relative to the initial, unmodified fragment. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign™ program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign™ program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) Comput Appl Biosci 8:189-191) and found in the MegAlign™ v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch, (1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain embodiments of the disclosure, the mutated gene comprises an alteration that results from a guide polynucleotide/Cas endonuclease system as disclosed herein. A mutated plant is a plant comprising a mutated gene.

As used herein, a "targeted mutation" is a mutation in a native gene that was made by altering a target sequence within the native gene using a method involving a double-strand-break-inducing agent that is capable of inducing a double-strand break in the DNA of the target sequence as disclosed herein or known in the art.

The guide RNA/Cas endonuclease induced targeted mutation can occur in a nucleotide sequence that is located within or outside a genomic target site that is recognized and cleaved by a Cas endonuclease.

The term "genome" as it applies to a cell encompasses not only chromosomal DNA found within the nucleus, but organelle DNA found within subcellular components (e.g., mitochondria, or plastid) of the cell.

A "codon-modified gene" or "codon-preferred gene" or "codon-optimized gene" is a gene having its frequency of codon usage designed to mimic the frequency of preferred codon usage of the host cell.

An "allele" is one of several alternative forms of a gene occupying a given locus on a chromosome. When all the alleles present at a given locus on a chromosome are the same, that plant is homozygous at that locus. If the alleles present at a given locus on a chromosome differ, that plant is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

A promoter is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". It has been shown that certain promoters are able to direct RNA synthesis at a higher rate than others. These are called "strong promoters". Certain other promoters have been shown to direct RNA synthesis at higher levels only in particular types of cells or tissues and are often referred to as "tissue specific promoters", or "tissue-preferred promoters" if the promoter directs RNA synthesis preferably in certain tissues but also in other tissues at reduced levels. Since patterns of expression of a chimeric gene (or genes) introduced into a plant are controlled using promoters, there is an ongoing interest in the isolation of novel promoters which are capable of controlling the expression of a chimeric gene or (genes) at certain levels in specific tissue types or at specific plant developmental stages.

The term "inducible promoter" refers to promoters that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners.

Examples of strong promoters useful in certain aspects herein (e.g., fungal and/or yeast cells) herein include those disclosed in U.S. Patent Appl. Publ. Nos. 2012/0252079 (DGAT2), 2012/0252093 (EL1), 2013/0089910 (ALK2), 2013/0089911 (SPS19), 2006/0019297 (GPD and GPM), 2011/0059496 (GPD and GPM), 2005/0130280 (FBA, FBAIN, FBAINm), 2006/0057690 (GPAT) and 2010/0068789 (YAT1). Other examples of strong promoters include XPR2 (U.S. Pat. No. 4937189; EP220864), GPD, GPM (U.S. Pat. Nos. 7259255 and 7459546), TEF (U.S. Pat. No. 6265185), GPDIN (U.S. Pat. No. 7459546, GPM/FBAIN (U.S. Pat. No. 7202356), FBA, FBAIN, FBAINm (U.S. Pat. No. 7202356), GPAT (U.S. Pat. No. 7264949), YAT1 (U.S. Pat. Appl. Publ. No. 2006/0094102) and EXP1(U.S. Pat. No. 7932077). Other examples of strong promoters useful in certain embodiments herein include PGK1, ADH1, TDH3, TEF1, PHO5, LEU2, and GAL1 promoters, as well as strong yeast promoters disclosed in Velculescu et al. (Cell 88:243-251).

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Examples of translation leader sequences have been described (e.g., Turner and Foster, (1995) Mol Biotechno/ 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1 :671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre mRNAt. "Messenger RNA" or "mRNA" refers to the RNA that is without introns and that can be translated into protein by the cell. "cDNA" refers to a DNA that is complementary to, and synthesized from, a mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra.*

"PCR" or "polymerase chain reaction" is a technique for the synthesis of specific DNA segments and consists of a series of repetitive denaturation, annealing, and extension cycles. Typically, a double-stranded DNA is heat denatured, and two primers complementary to the 3' boundaries of the target segment are annealed to the DNA at low temperature, and then extended at an intermediate temperature. One set of these three consecutive steps is referred to as a "cycle".

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to an extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The terms "recombinant DNA molecule", "recombinant construct", "expression construct", "construct", "construct", and "recombinant DNA construct" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

The term "providing" includes providing a nucleic acid (e.g., expression construct) or peptide, polypeptide or protein to a cell. Providing includes reference to the incorporation of a nucleic acid or polypeptide into a eukaryotic or prokaryotic cell where the nucleic acid may be incorporated into the genome of the cell, and includes reference to the transient provision of a nucleic acid or protein to the cell. Providing includes reference to stable or transient transformation methods, transfection, transduction, microinjection, electroporation, viral methods, *Agrobacterium*-mediated transformation, ballistic particle acceleration as well as sexually crossing. Thus, "providing" in the context of inserting a nucleic acid fragment (e.g., a recombinant DNA construct/expression construct, guide RNA, guide DNA, template DNA, donor DNA) into a cell, includes "transfection" or "transformation" or "transduction" and includes reference to the incorporation of a nucleic acid fragment into a eukaryotic or prokaryotic cell where the nucleic acid fragment may be incorporated into the genome of the cell (e.g., chromosome, plasmid, plastid, or mitochondrial DNA), converted into an autonomous replicon, or transiently expressed (e.g., transfected mRNA).

A variety of methods are known for contacting, providing, and/or introducing a composition (such as a nucleotide sequence, a peptide or a polypeptide) into an organisms including stable transformation methods, transient transformation methods, virus-mediated methods, sexual crossing and sexual breeding. Stable transformation indicates that the introduced polynucleotide integrates into the genome of the organism and is capable of being inherited by progeny thereof. Transient transformation indicates that the introduced composition is only temporarily expressed or present in the organism.

Protocols for contacting, providing, introducing polynucleotides and polypeptides to cells or organisms are known. and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, *Agrobacterium-mediated* transformation (U.S. Patent Nos. 5,563,055 and 5,981,840), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (*Liliaceae*); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via *Agrobacterium tumefaciens).*

Alternatively, polynucleotides may be introduced into cells or organisms by contacting cells or organisms with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931. Transient transformation methods include, but are not limited to, the introduction of polypeptides, such as a double-strand break inducing agent, directly into the organism, the introduction of polynucleotides such as DNA and/or RNA polynucleotides, and the introduction of the RNA transcript, such as an mRNA encoding a double-strand break inducing agent, into the organism. Such methods include, for example, microinjection or particle bombardment. See, for example Crossway et al., (1986) Mol Gen Genet 202:179-85; Nomura et al., (1986) Plant Sci 44:53-8; Hepler et al., (1994) Proc. Natl. Acad. Sci. USA 91:2176-80; and, Hush et al., (1994) J Cell Sci 107:775-84.

Nucleid acids and proteins can be provided to a cell by any method including methods using molecules to facilitate the uptake of anyone or all componens of a guided Cas system (protein and/or nucleic acids), such as cell-penetrating peptdes and nanocariers. See also US20110035836 Nanocarier based plant transfection and transduction, and EP 2821486 A1 Method of introducing nucleic acid intoi plant cells.

Providing a guide RNA/Cas endonuclease complex to a cell includes providing the individual components of said complex to the cell either directly or via recombination constructs, and includes providing the whole complex to the cell as well.

"Stable transformation" refers to the transfer of a nucleic acid fragment into a genome of a host organism, including both nuclear and organellar genomes, resulting in genetically stable inheritance. In contrast, "transient transformation" refers to the transfer of a nucleic acid fragment into the nucleus, or other DNA-containing organelle, of a host organism resulting in gene expression without integration or stable inheritance. Host organisms containing the transformed nucleic acid fragments are referred to as "transgenic" organisms.

The terms "control cell" and "suitable control cell" are used interchangeably herein and may be referenced with respect to a cell in which a particular modification (e.g., over-expression of a polynucleotide, down-regulation of a polynucleotide) has been made (i.e., an "experimental cell"). A control cell may be any cell that does not have or does not express the particular modification of the experimental cell. Thus, a control cell may be an untransformed wild type cell or may be genetically transformed but does not express the genetic transformation. For example, a control cell may be a direct parent of the experimental cell, which direct parent cell does not have the particular modification that is in the experimental cell. Alternatively, a control cell may be a parent of the experimental cell that is removed by one or more generations. Alternatively, a control cell may be a sibling of the experimental cell, which sibling does not comprise the particular modification that is present in the experimental cell.

The term "yeast" herein refers to fungal species that predominantly exist in unicellular form. Yeast can alternatively be referred to as "yeast cells". A yeast in certain aspects herein can be one that reproduces asexually (anamorphic) or sexually (teleomorphic). While yeast herein typically exist in unicellular form, certain types of these yeast may optionally be able to form pseudohyphae (strings of connected budding cells). In still further aspects, a yeast may be haploid or diploid, and/or may have the ability to exist in either of these ploidy forms. A yeast herein can be characterized as either a conventional yeast or non-conventional yeast, for example.

The term "conventional yeast" ("model yeast") herein generally refers to Saccharomyces or Schizosaccharomyces yeast species. Conventional yeast include yeast that favor homologous recombination (HR) DNA repair processes over repair processes mediated by non-homologous end-joining (NHEJ). Examples of conventional yeast herein include species of the genera *Saccharomyces* (e.g., *S. cerevisiae,* which is also known as budding yeast, baker's yeast, and/or brewer's yeast; *S. bayanus; S. boulardii; S. bulderi; S. cariocanus; S. cariocus; S. chevalieri; S. dairenensis; S. ellipsoideus; S. eubayanus; S. exiguus; S. florentinus; S. kluyveri; S. martiniae; S. monacensis; S. norbensis; S. paradoxus; S. pastorianus; S. spencerorum; S. turicensis; S. unisporus; S. uvarum; S. zonatus*) and *Schizosaccharomyces* (e.g., *S. pombe,* which is also known as fission yeast; *S. cryophilus; S. japonicus; S. octosporus*).

The term "non-conventional yeast" herein refers to any yeast that is not a *Saccharomyces* (e.g., *S. cerevisiae*) or *Schizosaccharomyces* yeast species. Non-conventional yeast are described in Non-Conventional Yeasts in Genetics, Biochemistry and Biotechnology: Practical Protocols (K. Wolf, K.D. Breunig, G. Barth, Eds., Springer-Verlag, Berlin, Germany, 2003). Non-conventional yeast in certain embodiments may additionally (or alternatively) be yeast that favor non-homologous end-joining (NHEJ) DNA repair processes over repair processes mediated by homologous recombination (HR).

Conventional yeasts such as *S. cerevisiae* and *S. pombe* typically exhibit specific integration of donor DNA with short flanking homology arms (30-50 bp) with efficiencies routinely over 70%, whereas non-conventional yeasts such as *Pichia pastoris, Pichia stipitis, Hansenula polymorpha, Yarrowia lipolytica* and *Kluyveromyces lactis* usually show specific integration with similarly structured donor DNA at efficiencies of less than 1% (Chen et al., PLoS ONE 8:e57952). Thus, a preference for HR processes can be gauged, for example, by transforming yeast with a suitable donor DNA and determining the degree to which it is specifically recombined with a genomic site predicted to be targeted by the donor DNA. A preference for NHEJ (or low preference for HR), for example, would be manifest if such an assay yielded a high degree of random integration of the donor DNA in the yeast genome. Assays for determining the rate of specific (HR-mediated) and/or random (NHEJ-mediated) integration of DNA in yeast are known in the art (e.g., Ferreira and Cooper, Genes Dev. 18:2249-2254; Corrigan et al., PLoS ONE 8:e69628; Weaver et al., Proc. Natl. Acad. Sci. U.S.A. 78:6354-6358; Keeney and Boeke, Genetics 136:849-856).

Given their low level of HR activity, non-conventional yeast herein can (i) exhibit a rate of specific targeting by a suitable donor DNA having 30-50 bp flanking homology arms of less than about 1%, 2%, 3%, 4%, 5%, 6%, 7%, or 8%, for example, and/or (ii) exhibit a rate of random integration of the foregoing donor DNA of more than about 65%, 66%, 67%, 68%, 69%, 70%, 71 %, 72%, 73%, 74%, or 75%, for example. These rates of (i) specific targeting and/or (ii) random integration of a suitable donor DNA can characterize a non-conventional yeast as it exists before being provided an RGEN as disclosed herein. An aim for providing an RGEN to a non-conventional yeast in certain embodiments is to create site-specific DNA single-strand breaks (SSB) or double-strand breaks (DSB) for biasing the yeast toward HR at the specific site. Thus, providing a suitable RGEN in a non-conventional yeast typically should allow the yeast to exhibit an increased rate of HR with a particular donor DNA. Such an increased rate can be at least about 2-, 3-, 4-, 5-, 6-, 7-, 8-, 9-, or 10-fold higher than the rate of HR in a suitable control (e.g., same non-conventional yeast transformed with the same donor DNA, but lacking a suitable RGEN).

A non-conventional yeast herein can be cultivated following any means known in the art, such as described in Non-Conventional Yeasts in Genetics. Biochemistry and Biotechnology: Practical Protocols (K. Wolf, K.D. Breunig, G. Barth, Eds., Springer-Verlag, Berlin, Germany, 2003), Yeasts in Natural and Artificial Habitats (J.F.T. Spencer, D.M. Spencer, Eds., Springer-Verlag, Berlin, Germany, 1997), and/or Yeast Biotechnology: Diversity and Applications (T. Satyanarayana, G. Kunze, Eds., Springer, 2009).

Non-limiting examples of non-conventional yeast herein include yeasts of the following genera: *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces, Pachysolen,* and *Moniliella.* A suitable example of a *Yarrowia* species is *Y. lipolytica.* Suitable examples of *Pichia* species include *P. pastoris, P. methanolica, P. stipitis, P. anomala* and *P. angusta.* Suitable examples of *Schwanniomyces* species include S. *castellii, S. alluvius, S. hominis, S. occidentalis, S.* capriottii, *S. etchellsii, S. polymorphus, S. pseudopolymorphus, S. vanrijiae* and *S. yamadae.* Suitable examples of *Kluyveromyces* species include *K. lactis, K. marxianus, K. fragilis, K. drosophilarum, K. thermotolerans, K. phaseolosporus, K. vanudenii, K. waltii, K. africanus* and *K. polysporus.* Suitable examples of *Arxula* species include *A. adeninivorans* and *A. terrestre.* Suitable examples of *Trichosporon* species include *T. cutaneum, T. capitatum, T. inkin* and *T. beemeri.* Suitable examples of *Candida* species include *C. albicans, C. ascalaphidarum, C. amphixiae, C. antarctica, C. apicola, C. argentea, C. atlantica, C. atmosphaerica, C. blattae, C. bromeliacearum, C. carpophila, C. carvajalis, C. cerambycidarum, C. chauliodes, C. corydali, C. dosseyi, C. dubliniensis, C. ergatensis, C. fructus, C. glabrata, C. fermentati, C. guilliermondii, C. haemulonii, C. insectamens, C. insectorum, C. intermedia, C. jeffresii, C. kefyr, C. keroseneae, C. krusei, C. lusitaniae, C. lyxosophila, C. maltosa, C. marina, C. membranifaciens, C. milleri, C. mogii, C. oleophila, C. oregonensis, C. parapsilosis, C. quercitrusa, C. rugosa, C. sake, C. shehatea, C. temnochilae, C. tenuis, C. theae, C. tolerans, C. tropicalis, C. tsuchiyae, C. sinolaborantium, C. sojae, C. subhashii, C. viswanathii, C. utilis, C. ubatubensis* and *C. zemplinina.* Suitable examples of *Ustilago* species include *U. avenae, U. esculenta, U. hordei, U. maydis, U. nuda* and *U. tritici.* Suitable examples of *Torulopsis* species include *T. geochares, T. azyma, T. glabrata* and *T. candida.* Suitable examples of *Zygosaccharomyces* species include *Z. bailii, Z. bisporus, Z. cidri, Z. fermentati, Z. florentinus, Z. kombuchaensis, Z. lentus, Z. mellis, Z. microellipsoides, Z. mrakii, Z. pseudorouxii* and *Z. rouxii.* Suitable examples of *Trigonopsis* species include *T. variabilis.* Suitable examples of *Cryptococcus* species include *C. laurentii, C. albidus, C. neoformans, C. gattii, C. uniguttulatus, C. adeliensis, C. aerius, C. albidosimilis, C. antarcticus, C. aquaticus, C. ater, C. bhutanensis, C. consortionis, C. curvatus, C. phenolicus, C. skinneri, C. terreus* and *C. vishniacci.* Suitable examples of *Rhodotorula* species include *R. acheniorum, R. tula, R. acuta, R. americana, R. araucariae, R. arctica, R. armeniaca, R. aurantiaca, R. auriculariae, R. bacarum, R. benthica, R. biourgei, R. bogoriensis, R. bronchialis, R. buffonii, R. calyptogenae, R. chungnamensis, R. cladiensis, R. corallina, R. cresolica, R. crocea, R. cycloclastica, R. dairenensis, R. diffluens, R. evergladiensis, R. ferulica, R. foliorum, R. fragaria, R. fujisanensis, R. futronensis, R. gelatinosa, R. glacialis, R. glutinis, R. gracilis, R. graminis, R. grinbergsii, R. himalayensis, R. hinnulea, R. histolytica, R. hylophila, R. incarnata, R. ingeniosa, R. javanica, R. koishikawensis, R. lactosa, R. lamellibrachiae, R. laryngis, R. lignophila, R. lini, R. longissima, R. ludwigii, R. lysinophila, R. marina, R. martyniae-fragantis, R. matritensis, R. meli, R. minuta, R. mucilaginosa, R. nitens, R. nothofagi, R. oryzae, R. pacifica, R. pallida, R. peneaus, R. philyla, R. phylloplana, R. pilatii, R. pilimanae, R. pinicola, R. plicata, R. polymorpha, R. psychrophenolica, R. psychrophila, R. pustula, R. retinophila, R. rosacea, R. rosulata, R. rubefaciens, R. rubella, R. rubescens, R. rubra, R. rubrorugosa, R. rufula, R. rutila, R. sanguinea, R. sanniei, R. sartoryi, R. silvestris, R. simplex, R. sinensis, R. slooffiae, R. sonckii, R. straminea, R. subericola, R. suganii, R. taiwanensis, R. taiwaniana, R. terpenoidalis, R. terrea, R. texensis, R. tokyoensis, R. ulzamae, R. vanillica, R. vuilleminii, R. yarrowii, R. yunnanensis* and *R. zsoltii.* Suitable examples of *Phaffia* species include *P. rhodozyma.* Suitable examples of *Sporobolomyces* species include *S. alborubescens, S. bannaensis, S. beijingensis, S. bischofiae, S. clavatus, S. coprosmae, S. coprosmicola, S. corallinus, S. dimmenae, S. dracophylli, S. elongatus, S. gracilis, S. inositophilus, S. johnsonii, S. koalae, S. magnisporus, S. novozealandicus, S. odorus, S. patagonicus, S. productus, S. roseus, S. sasicola, S. shibatanus, S. singularis, S. subbrunneus, S. symmetricus, S. syzygii, S. taupoensis, S. tsugae, S. xanthus* and *S. yunnanensis.* Suitable examples of *Pachysolen* and *Moniliella* species include *P. tannophilus* and *M. pollinis,* respectively. Still other examples of non-conventional yeasts herein include *Pseudozyma* species (e.g., *S. antarctica*), *Thodotorula* species (e.g., *T. bogoriensis*), *Wickerhamiella* species (e.g., *W. domercqiae*), and *Starmerella* species (e.g., *S. bombicola*).

*Yarrowia lipolytica* is preferred in certain embodiments disclosed herein. Examples of suitable *Y. lipolytica* include the following isolates available from the American Type Culture Collection (ATCC, Manassas, VA): strain designations ATCC #20362, #8862, #8661, #8662, #9773, #15586, #16617, #16618, #18942, #18943, #18944, #18945, #20114, #20177, #20182, #20225, #20226, #20228, #20327, #20255, #20287, #20297, #20315, #20320, #20324, #20336, #20341, #20346, #20348, #20363, #20364, #20372, #20373, #20383, #20390, #20400, #20460, #20461, #20462, #20496, #20510, #20628, #20688, #20774, #20775, #20776, #20777, #20778, #20779, #20780, #20781, #20794, #20795, #20875, #20241, #20422, #20423, #32338, #32339, #32340, #32341, #34342, #32343, #32935, #34017, #34018, #34088, #34922, #34922, #38295, #42281, #44601, #46025, #46026, #46027, #46028, #46067, #46068, #46069, #46070, #46330, #46482, #46483, #46484, #46436, #60594, #62385, #64042, #74234, #76598, #76861, #76862, #76982, #90716, #90811, #90812, #90813, #90814, #90903, #90904, #90905, #96028, #201241, #201242, #201243, #201244, #201245, #201246, #201247, #201249, and/or #201847.

The terms "5'-cap" and "7-methylguanylate (m⁷G) cap" are used interchangeably herein. A 7-methylguanylate residue is located on the 5' terminus of RNA transcribed by RNA polymerase II (Pol II) in eukaryotes. A capped RNA herein has a 5'-cap, whereas an uncapped RNA does not have such a cap.

The terminology "uncapped", "not having a 5'-cap", and the like are used interchangeably herein to refer to RNA lacking a 5'-cap and optionally having, for example, a 5'-hydroxyl group instead of a 5'-cap. Uncapped RNA can better accumulate in the nucleus following transcription, since 5'-capped RNA is subject to nuclear export.

The terms "ribozyme", "ribonucleic acid enzyme" and "self-cleaving ribozyme" are used interchangeably herein. A ribozyme refers to one or more RNA sequences that form secondary, tertiary, and/or quaternary structure(s) that can cleave RNA at a specific site, particularly at a cis-site relative to the ribozyme sequence (i.e., auto-catalytic, or self-cleaving). The general nature of ribozyme nucleolytic activity has been described (e.g., Lilley, Biochem. Soc. Trans. 39:641-646). A "hammerhead ribozyme" (HHR) herein may comprise a small catalytic RNA motif made up of three base-paired stems and a core of highly conserved, non-complementary nucleotides that are involved in catalysis. Pley et al. (Nature 372:68-74) and Hammann et al. (RNA 18:871-885), disclose hammerhead ribozyme structure and activity. A hammerhead ribozyme herein may comprise a "minimal hammerhead" sequence as disclosed by Scott et al. (Cell81:991-1002), for example.

The term "increased" as used herein may refer to a quantity or activity that is at least about 1%, 2%, 3%, 4%, 5%, 6%, 7%, 8%, 9%, 10%, 11%, 12%, 13%, 14%, 15%, 16%, 17%, 18%, 19%, 20%, 50%, 100%, or 200% more than the quantity or activity for which the increased quantity or activity is being compared. The terms "increased", "elevated", "enhanced", "greater than", and "improved" are used interchangeably herein. The term "increased" can be used to characterize the expression of a polynucleotide encoding a protein, for example, where "increased expression" can also mean "over-expression".

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Standard DNA isolation, purification, molecular cloning, vector construction, and verification/characterization methods are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

### EXAMPLES

In the following Examples, unless otherwise stated, parts and percentages are by weight and degrees are Celsius. It should be understood that these Examples, while indicating embodiments of the disclosure, are given by way of illustration only. From the above discussion and these Examples, one skilled in the art can make various changes and modifications of the disclosure to adapt it to various usages and conditions. Such modifications are also intended to fall within the scope of the appended claims.

### EXAMPLE 1

### Cas9 HDV-gRNA expression plasmid targeting Can1

This example discusses the use of sgRNAs that are flanked on the 5' end by a HDV ribozyme. The HDV ribozyme cleaves 5' of its own sequence removing any preceding RNA sequence but leaving the HDV sequence fused to the 5' end of the gRNA.

In order to test a sgRNA/Cas endonuclease system in *Yarrowia,* the Cas9 gene from *Streptococcus pyrogenes* M1 GAS (SF370 (SEQ ID NO: 1) was *Yarrowia* codon optimized per standard techniques known in the art (SEQ ID NO: 2). In order to localize the Cas9 protein to the nucleus of the cells, *Simian virus 40* (SV40) monopartite (PKKKRKV, SEQ ID NO: 3) nuclear localization signal was incorporated at the carboxy terminus of the Cas9 protein. The Yarrowia codon optimized Cas9 gene was fused to a Yarrowia constitutive promoter, FBA1 (SEQ ID NO: 4), by standard molecular biology techniques. An example of a Yarrowia codon optimized Cas9 expression cassette (SEQ ID NO: 5) contains the FBA1 promoter, the Yarrowia optimized Cas9-NLS fusion, and the Cas9 expression cassette was cloned into the plasmid pZuf and the new construct called pZufCas9 (SEQ ID NO 6).

Plasmid pZuf-Cas9CS (SEQ ID NO: 6) was mutagenized using Agilent QuickChange and the following primers:
Aarl-removal-1:
   (AGAAGTATCCTACCATCTACcatctccGAAAGAAACTCGTCGATTCC; SEQ ID NO: 7) and
Aarl-removal-2:
   (GGAATCGACGAGTTTCTTTCggagatgGTAGATGGTAGGATACTTCT; SEQ ID NO:8)
to remove the endogenous Aarl site present in the *Yarrowia* codon optimized Cas9 gene present in pZuf-Cas9CS generating pRF109 (SEQ ID NO: 9). The modified Aar1- Cas9CS gene (SEQ ID NO: 10) was cloned as a Ncol/Notl fragment from pRF109 (SEQ ID NO: 9) into the Ncol/Notl site of pZufCas9CS (SEQU ID NO: 6) replacing the existing Cas9 gene (SEQ ID NO: 2) with the Aar1- Cas9 gene (SEQ ID NO: 10) generating pRF141 (SEQ ID NO: 11).

One example of a high throughput variable targeting domain cloning cassette (depicted in Figure 1, SEQ ID NO: 12) is composed of the yl52 promoter (SEQ ID NO: 13), the DNA sequence encoding the HDV ribozyme (SEQ ID NO: 14), the *Escherichia coli* counterselection cassette *rpsL* (SEQ ID NO: 15), the DNA encoding the Cas9 CER domain (SEQ ID NO: 16) and the *S. cerevisiae SUP4* terminator (SEQ ID NO: 17). Flanking the ends of the high-throughput cloning cassette (SEQ ID NO: 12) are Pacl and Clal restriction enzyme recognition sites. The high-throughput cloning cassette (SEQ ID NO: 12) was cloned into the Pacl/Clal sites of pRF141 (SEQ ID NO: 11) to generate pRF291 (SEQ ID NO 14). The *rpsL* counterselection cassette (SEQ ID NO: 15) contains a WT copy of the *E. coli rpsL* gene with its native promoter and terminator. *rpsL* encodes the S12 ribosomal protein subunit *(*Escherichia coli and Salmonella typhimurium: Cellular and Molecular Biology, 1987 American Society of Microbiology). Some mutations in the S12 subunit cause resistance to the antibiotic streptomycin (Ozaki, M., et al. (1969). "Identification and functional characterization of the protein controlled by the streptomycin-resistant locus in E. coli." Nature 222(5191): 333-339). in a recessive manner (Lederberg, J. (1951). "Streptomycin resistance; a genetically recessive mutation." J Bacteriol 61(5): 549-550.) such that if a wild-type copy of the *rpsL* gene is present, the strain is phenotypically sensitive to streptomycin (Lederberg, J. (1951). "Streptomycin resistance; a genetically recessive mutation." J Bacteriol 61(5): 549-550.). Common cloning strains such as Top10 (Life technologies) and D10B have a mutated copy of *rpsL* on their chromosome such that the cells are recessively resistant to streptomycin.

Cloning a variable targeting domain (VT), Figure 1) into pRF291 requires two partially complimentary oligonucleotides that when annealed contain the DNA sequence encoding the desired variable targeting domain as well as the correct overhangs for cloning into the two Aarl sites present in the high-throughput cloning cassette. Two oligonucleotides, Can1-1F ( AATGGGACtcaaacgattacccaccctcGTTT, SEQ ID NO: 19) and Can1-1R (TCTAAAACgagggtgggtaatcgtttgaGTCC, SEQ ID NO: 20) containing the DNA encoding the variable targeting domain Can1-1 (SEQ ID NO: 21) which targets the Can1-1 target site (SEQ ID NO: 22) in the *CAN1* gene of *Yarrowia lipolytica* (SEQ ID NO: 23), were resuspended in duplex buffer (30 mM HEPES pH 7.5, 100 mM Sodium Acetate) at 100 µM. Can1-1F (SEQ ID NO: 19) and Can1-1R (SEQ ID NO: 20) were mixed at a final concentration of 50 µM each in a single tube, heated to 95°C for 5 minutes and cooled to 25°C at 0.1°C/min to anneal the two oligonucleotides to form a small duplex DNA molecule (Figure 2). A single tube digestion/ligation reaction was created containing 50 ng of pRF291, 2.5 µM of the small duplex DNA composed of Can1-1 F (SEQ ID NO: 19) and Can1-1 R (SEQ ID NO: 20), 1x T4 ligase buffer (50 mM Tris-HCI, 10 mM MgCl₂, 1 mM ATP, 10 mM DTT pH 7.5), 0.5 µM Aarl oligonucleotide, 2 units Aarl, 40 units T4 DNA ligase in a 20 µl final volume. A second control reaction lacking the duplexed Can1-1F and Can1-1R duplex was also assembled. The reactions were incubated at 37°C for 30 minutes. 10µl of each reaction was transformed into Top10 *E. coli* cells as previously described (Green, M. R. & Sambrook, J. Molecular Cloning: A Laboratory Manual. Fourth Edition edn, (Cold Spring Harbor Laboratory Press, 2012)). In order to select for the presence of pRF291 where the duplex of Can1-1F (SEQ ID NO: 19) and Can1-1R (SEQ ID NO: 20) had replaced the *rpsL* counterselection marker flanked by Aarl restriction sites (Figure 1) cells were plated on lysogeny broth (1% w/v Tryptone, 0.5% w/v Yeast Extract, 1% w/v Sodium chloride) solidified with 1.5% (w/v) Bacto agar containing 100µg/ml Ampicillin and 50 µg/ml Streptomycin. The presence of pRF291 containing the high-throughput cloning cassette yielded colonies phenotypically resistant to the antibiotic ampicillin but sensitive to the antibiotic streptomycin due to the presence of the counterselection cassette on the plasmid.

However, in cases where the counterselection cassette was removed via the Aarl enzyme and the cassette containing the DNA duplex encoding the Can1-1 variable targeting domain was ligated into the site (removing the recognition sequences for Aarl) the cells transformed with the plasmid had an ampicillin resistant, streptomycin resistant phenotype (Figure 1). pRF291 containing the Can1-1 variable targeting domain replacing the counterselection cassette created a recombinant Can1-1 gRNA expression cassette (SEQ ID NO: 19) containing the yl52 promoter (SEQ ID NO: 13) fused to the DNA encoding the HDV ribozyme (SEQ ID NO: 14) fused to the DNA encoding the Can1-1 variable targeting domain (SEQ ID NO: 21) fused to the DNA encoding the CER domain (SEQ ID NO: 16) fused to the *SUP4* terminator (SEQ ID NO: 17). The plasmid containing this construct, pRF303 (SEQ ID NO: 24) was used to target the *CAN1* gene (SEQ ID NO: 23) of *Yarrowia lipolytica* with Cas9.

### EXAMPLE 2

### Generation of linear polynucleotide modification (editing)\ templates

A polynucleotide modification template (also referred to as an editing template) was generated by making two PCR products, one, the 629 bp ending 2 bp 5' of the CAN1 open reading frame (SEQ ID NO: 25) which was amplified from *Yarrowia lipolytica* ATCC20362 genomic DNA using standard techniques (primers used, GGGAAGCTTGCTACGTTAGGAGAAGACGC (forward, SEQ ID NO: 26) and GGAGAGAGCGTCGGGAGTGGTCGGATGGATGGAGACG (reverse, SEQ ID NO:27)). The reverse primer adds 17 nucleotides complementary to the sequence 37 bp 3' of the *CAN1* open reading frame and the forward primer adds a 5' HinDIII recognition site. The second PCR product, consisting of 637 bp starting 37 base pairs 3' of the CAN1 open-reading frame (SEQ ID NO: 28). This PCR product was amplified from *Yarrowia lipolytica* ATCC20362 genomic DNA using standard techniques (primers used, CGTCTCCATCCATCCGACCACTCCCGACGCTCTCTCC (forward, SEQ ID NO: 29) and CCATACATCCTTCCACCACTGC (reverse, SEQ ID NO: 30)). The forward primer adds the 20 nucleotides complementary to the region ending 2 bp 5' of the *CAN1* open reading frame. Both the upstream (SEQ ID NO: 25) and the downstream PCR product (SEQ ID NO:28) were purified using Zymo clean and concentrate columns. 10ng of each PCR product were mixed in a new PCR reaction. The 3' 37 nucleotides of the upstream product is identical to the 5' 37 nucleotides of the downstream product. The upstream and downstream fragments were used to prime each other creating a single product (SEQ ID NO: 31) by synthesis from overlapping ends containing both the upstream and downstream sequences (Horton et al (2013) Biotechniques 54(3):129-133) (Figure 2A). The complete editing template was digested with HinDIII and cloned into the HinDIII site of pUC18 (SEQ ID NO: 32) using standard techniques generating plasmid pRF80 (SEQ ID NO: 33) containing a 1210bp editing template (SEQ ID NO: 34, Figure 1 HDV-VT-CER) that when used as a linear template for homology directed repair (HDR) will lead to the deletion of the entire *CAN1* open reading frame.

A linear editing template (linear polynucleotide modification template) was amplified from pRF80 using chemically synthesized oligonucleotide primers and standard techniques (primers used, AGCTTGCTACGTTAGGAGAA, forward (SEQ ID NO: 36) and TATGAGCTTATCCTGTATCG, reverse (SEQ ID NO: 37) to yield a 1215bp linear *CAN1* deletion polynucleotide modification (editing) template (SEQ ID NO: 31). PCR reactions of linear editing templates were purified using Zymo clean and concentrate 25 columns and eluted in 25µl of 10mM Tris 1mM EDTA pH8.0.

### EXAMPLE 3

### Enhanced Cas9/sgRNA precise gene modification using Scr7 treated cells and a linear editing template

In this example *Yarrowia* lipolytica cells treated with the DNA Ligase IV inhibitor, Scr7, were transformed with targeting plasmids in the presence and absence of editing templates. Homology directed repair (HDR) occurs between an editing template and the target DNA when there are two regions of homology flanking a region of interest (Figure 3). A DNA double-stranded break at a target site within the region of interest can initiate HDR replacing the region of interest (Figure 3, white box) with a modified region of interest carried on the editing template. In this example the modified region of interest lacks the entire open-reading frame of the *CAN1* gene. Hence, when this linear template is used for homology directed repair, HDR will lead to the deletion of the entire *CAN1* open reading frame making the cells Canavanine resistant.

Cells were phenotypically scored for Canavanine resistance to determine overall targeting efficiency (NHEJ+HDR). Colony PCR of the *CAN1* locus (SEQ ID NO: 35) was performed to differentiate repair of the Cas9/gRNA generated double-strand break by HDR or NHEJ.

Three different treatment conditions were tested: Untreated, Scr7-A, and Scr7-B (A schematic of the three treatments is shown in Figure 4). For all three conditions a *uracil* auxotrophic strain of *Yarrowia lipolytica* ATCC20362 was used For the untreated condition the strain was grown for 24 hours on YPD medium plates (Teknova) at 30°C (also referred to as a pretreatment). 1 loop of cells were resuspended in transformation buffer (35% polyethylene glycol average molecular weight of 3550, 100mM lithium acetate, 100mM dithiothreitol, 10mM Tris, 1mM EDTA pH 6.0). 100µl of cell suspension was mixed with 100ng of either pRF291 (SEQ ID NO: 18) (Cas9 expression, no gRNA) or pRF303 (SEQ ID NO: 24) (Cas9 expression, Can1-1 gRNA expression) with either no editing template or 1 µg linear editing template (SEQ ID NO: 34). Transformation mixtures were incubated at 39°C for 1 hour at 800 RPM. Transformation mixtures were plated on complete minimal medium plates lacking uracil (Teknova) to select for cells transformed with plasmid DNA. Plates were incubated at 30°C for 48 hours.

For treatment Scr7-A the strain was grown for 24 hours on YPD medium plates (Teknova) at 30°C (also referred to as a pretreatment). 1 loop of cells were resuspended in modified transformation buffer (35% polyethylene glycol average molecular weight of 3550, 100mM lithium acetate, 100mM dithiothreitol, 10mM Tris, 1mM EDTA pH 6.0, 5µM Scr7). 100µl of cell suspension was mixed with 100ng of either pRF291 (SEQ ID NO:18) (Cas9 expression, no gRNA) or pRF303 (SEQ ID NO: 24) (Cas9 expression, Can1-1 gRNA expression) with either no editing template or 1µg linear editing template (SEQ ID NO: 31). Transformation mixtures were incubated at 39°C for 1 hour at 800 RPM. Transformation mixtures were plated on complete minimal medium plates lacking uracil (Teknova) to select for cells transformed with plasmid DNA. Plates were incubated at 30°C for 48 hours.

For treatment Scr7-B the strain was grown for 24 hours on YPD medium plates containing 5µM Scr7 at 30°C (also referred to as a pretreatment). 1 loop of cells were resuspended in modified transformation buffer (35% polyethylene glycol average molecular weight of 3550, 100mM lithium acetate, 100mM dithiothreitol, 10mM Tris, 1mM EDTA pH 6.0, 5µM Scr7). 100µl of cell suspension was mixed with 100ng of either pRF291 (SEQ ID NO:18) (Cas9 expression, no gRNA) or pRF303 (SEQ ID NO: 24) (Cas9 expression, Can1-1 gRNA expression) with either no editing template or 1µg linear editing template (SEQ ID NO: 31). Transformation mixtures were incubated at 39°C for 1 hour at 800 RPM. Transformation mixtures were plated on complete minimal medium containing 5µM Scr-7 plates lacking uracil. Plates were incubated at 30°C for 48 hours.

For each condition, 24 colonies from each transformation were streak purified on complete minimal plates lacking uracil (Teknova) for single colonies. 4 single colonies from each streak purified colony (96 for each transformation) were patched to complete minimal plates lacking arginine containing 60µg/ml L-canavanine. L-canavanine is toxic to cells with a functional *CAN1* gene which is an importer of arginine and L-canavanine to the cells. Cells containing a loss of function allele in the *CAN1* gene (due to NHEJ and/or HDR) will be phenotypically resistant to the presence of L-canavanine in the medium and will form colonies on plates containing L-canavanine. Cells containing a wild-type copy of the *CAN1* gene will be unable to grow on medium containing L-canavanine. The mode of action of L-canavanine is well known (Rosenthal G.A., The Biological effects and mode of action of L-Canavanine, a structural analog of L-arginine, The quarterly review of biology, volume 52, 1977, 155-178). The frequency of Canavanine resistance by transformation treatment is given in Table 2. Cells transformed with pRF291 (SEQ ID NO: 18) which carries a Cas9 expression cassette but lacks a functional gRNA targeting the *CAN1* gene did not give rise to Canavanine resistance cells (Table 2) regardless of Scr-7 treatment scheme. Cells transformed with pRF303 (SEQ ID NO: 24) in the presence or absence of editing templates regardless of the treatment scheme with Scr-7 gave similar frequencies of canavanine resistant colonies (Table 2) suggesting that neither the presence of an editing template in the transformation mix nor the treatment of cells with Scr-7 negatively affects the ability of Cas9/gRNA to induce targeted double strand breaks.

To determine the frequency of HDR at the targeted double-strand breaks generated by Cas9/gRNA at the Can1-1 target site Yarrowia colony PCR of the *CAN1* locus (SEQ ID NO: 40) (primers used, GGAAGGCACATATGGCAAGG, forward (SEQ ID NO: 38) and GTAAGAGTGGTTTGCTCCAGG, reverse (SEQ ID NO: 39)) was performed using standard techniques. If the *CAN1* locus was unmodified or contained a small indel generated by NHEJ the colony PCR result would give a band similar in size to the WT *CAN1* locus at an apparent size of 2125bp (SEQ ID NO: 40). If the Cas9/gRNA generated double-strand break had been repaired using the editing templates the PCR would generate a smaller *CAN1* locus product 392bp indicating the deletion of the entire open-reading frame (SEQ ID NO: 41). An example of the colony PCR data is shown in Figure 4. Colony PCR was performed on all canavanine resistant colonies from cells transformed with pRF303 in the presence of editing template and the fraction of cells where the Cas9/gRNA generated double-strand break was repaired via HDR with the editing template and various Scr-7 treatment schemes was determined (Table 1).

**Table 2**

| **Frequency of *CAN1* inactivation** | | | | | | |
|---|---|---|---|---|---|---|
| Scr7 treatment | plasmid | Target site | Editing template | Canavanine resistance frequency (± Std. Dev) | HDR Frequency (±SEM) | Fold HDR over untreated (±SEM) |
| None | pRF291 | None | - | 0±0 | ND¹ | ND |
| None | pRF291 | None | + | 0±0 | ND | ND |
| None | pRF303 | Can1-1 | - | 63.2±10.4 | ND | ND |
| None | pRF303 | Can1-1 | + | 67.7±4.8 | 11.1±2.8 | 1.0±0.0 |
| A | pRF291 | None | - | 0±0 | ND | ND |
| A | pRF291 | None | + | 0±0 | ND | ND |
| A | pRF303 | Can1-1 | - | 62.2±6.7 | ND | ND |
| A | pRF303 | Can1-1 | + | 64.2±6.4 | 9.7±1.4 | 0.9±0.1 |
| B | pRF291 | None | - | 0±0 | ND | ND |
| B | pRF291 | None | + | 0±0 | ND | ND |
| B | pRF303 | Can1-1 | - | 68.7±6.3 | ND | ND |
| B | pRF303 | Can1-1 | + | 64.6±20.3 | 16.7±4.8 | 1.5±0.3 |

| | | | | | | |
|---|---|---|---|---|---|---|
| ¹Not determined. | | | | | | |

Cells treated with editing template with no Scr-7 addition or Scr-7 treatment "A" had similar frequencies of HDR at the *CAN1* locus (Table 1). Cells treated with editing template and Scr-7 treatment "B" had HDR frequencies 1.5 fold higher than the untreated control or Scr-7 treatment "A". In the repair of Cas9/gRNA generated double-stranded breaks linear editing templates combined with the extended Scr-7 treatment B provided substantial increases in the fraction of breaks repaired via HDR with 150% of the frequency of untreated or the short Scr-7 treatment A.

An additional complication of repair of Cas9/gRNA generated double-stranded breaks using editing template is the possibility that the editing template can be incorporated by the NHEJ pathway at other regions of DNA damage. In order to determine the frequency at which this occurs in the cells treated with editing templates under the three different Scr-7 treatments, relative copy number analysis was performed looking for a 62bp fragment of the *CAN1* locus present in both the chromosome and the editing template (SEQ ID NO: 42). Relative copy number analysis was performed on colonies from cells treated with pRF303 (SEQ ID NO: 24) and linear editing template (SEQ ID NO: 34) under all three treatment conditions. If the editing template is only incorporated during the HDR of the Cas9/gRNA double strand break at the *CAN1* locus (SEQ ID NO: 35) the cell will only carry a single copy of the copy number analysis fragment (SEQ ID NO: 42). However, if the cell incorporates additional copies of the editing template elsewhere in the genome due to the activity of the NHEJ pathway additional copies of the fragment will be present and the cell will return a higher relative copy number. Briefly, genomic DNA was isolated using standard techniques from colonies that scored positive for HDR of the Can1-1 Cas9/gRNA targeted double strand break. Genomic DNA was normalized to 5ng/µl. 1µl of the normalized genomic DNA from each colony was added to three replicate qPCR reactions for both the *CAN1* locus (SEQ ID NO: 42), (primers used, AGCGCCAAACCCAAAGC, forward (SEQ ID NO: 43), CTTGCCATATGTGCCTTCCA, reverse (SEQ ID NO: 44), and 6FAM-CTTTTCGCCCCCACTGCAGCC-TAMRA, probe (SEQ ID NO: 45)) or as a control the *TEF1* locus (SEQ ID NO: 56) (primers used, CGACTGTGCCATCCTCATCA, forward (SEQ ID NO: 47), TGACCGTCCTTGGAGATACCA, reverse (SEQ ID NO: 48) and 6FAM-TGCTGGTGGTGTTGGTGAGTT-TAMRA, probe (SEQ ID NO: 49)). Reactions were run in TaqMAN universal PCR master mix (ABI life technologies) on a life technologies Quant Studio 7 instrument using the following cycling conditions 95°C for 10 minutes followed by 40 cycles of 95°C for 15 seconds, 60°C for 1 minute. 6FAM fluorescence from the probes was monitored through the 40 cycles of the PCR and Ct values were collected. Relative gene copy number was determined by the ΔΔCt method (User Bulletin #2 ABI PRISM 7700 Sequence Detection System (Updated 2001)). Briefly, TEF1 Ct values were used to normalize the data for differences in cell copy number between genomic DNA samples. Genomic DNA from a wildtype strain was used as a reference for the relative quantification for the *CAN1* copy number fragment (SEQ ID NO: 42). Software on the Quant studio 7 calculated relative gene copy number and corresponding error for each sample relative to the wildtype strain. The colonies were separated into two bins, those with a single relative copy of the *CAN1* analysis fragment (SEQ ID NO: 42) and those with 2 or more relative copies (SEQ ID NO: 42). The first bin indicates that the editing template was only used for HDR repair of the Cas9/gRNA generated double strand break at the Can1-1 target site (SEQ ID NO: 22) and was not integrated elsewhere in the genome by NHEJ. The second bin represents cells where the editing template was used to repair the Cas9/gRNA generated double-strand break at the Can1-1 target site (SEQ ID NO: 22) and was integrated at least once somewhere else in the genome via a NHEJ mechanism. The results of the copy number analysis are presented in Table 3.

**Table 3**

| **Frequency of off-target insertion** | | |
|---|---|---|
| Scr7 treatment | Fraction of cells with single copy of *CAN1* | Fraction of cells with ≥2 copies of *CAN1* |
| None | 0.43 | 0.57 |
| A | 0.38 | 0.63 |
| B | 1 | 0 |

Both the untreated and Scr-7 treatment A colonies treated with pRF303 (SEQ ID NO: 24) and linear editing template (SEQ ID NO: 34) yield approximately 40% of colonies with a single copy of the *CAN1* editing template indicating that the editing template was used for HDR of the Cas9/gRNA generated DSB but did not integrate elsewhere in the genome (Table 2). The colonies from Scr-7 treatment B, pRF303 (SEQ ID NO: 24) and editing template (SEQ ID NO: 34) demonstrated 100 percent of the colonies with only a single copy of the *CAN1* locus indicating that the editing template was only used for HDR of the Cas9/gRNA generated break at Can1-1 and was not integrated elsewhere in the chromosome. The lack of additional chromosomal insertions under treatment condition B was surprising given that some of the Cas9 generated DSBs at *CAN1-1* under these conditions are repaired using the NHEJ pathway. However, this represents a useful improvement to repair of Cas9 mediated DSBs using HDR.

## Claims

1. A method for altering a target site in the genome of a non-conventional yeast cell, the method comprising providing to a non-conventional yeast cell at least one guide RNA, at least one Cas endonuclease capable of introducing a double strand break at the target site, and an inhibitor of a DNA Ligase IV (LIG4), wherein the inhibitor is Scr7.

2. The method of claim 1, wherein the cell is grown in a medium comprising said inhibitor at a concentration of at least 0.5 microMolar, for at least 6 hours, at a temperature of at least 20°C prior to providing the guide RNA and the Cas endonuclease to the cell.

3. A method for editing a nucleotide sequence in the genome of a non-conventional yeast cell, the method comprising providing to a non-conventional yeast cell at least one guide RNA, at least one polynucleotide modification template comprising at least one nucleotide modification of the nucleotide sequence, at least one Cas endonuclease capable of introducing a double strand break at a target site in the genome of said cell, and an inhibitor of a DNA Ligase IV (LIG4), wherein the inhibitor is Scr7.

4. A method for selecting a non-conventional yeast cell comprising an altered target sequence, the method comprising:
a) providing to a non-conventional yeast cell at least one guide RNA, at least one Cas endonuclease capable of introducing a double strand break at a target site in the genome of said non-conventional yeast cell, and an inhibitor of a DNA Ligase IV (LIG4), wherein the inhibitor is Scr7,
b) evaluating the non-conventional yeast cell of (a) for off-target site effects as well as for the presence of said altered target sequence; and,
c) selecting a non-conventional yeast cell from (b) that has said altered target site while having reduced or no off-target site effects.

5. A method for selecting an edited non-conventional yeast cell, the method comprising:
a) providing to a non-conventional yeast cell comprising a nucleotide sequence to be edited, at least one guide RNA, at least one polynucleotide modification template comprising at least one nucleotide modification of said nucleotide sequence, at least one Cas endonuclease capable of introducing a double strand break at a target site in the genome of said cell, and an inhibitor of a DNA Ligase IV (LIG4), wherein the inhibitor is Scr7,
b) evaluating the non-conventional yeast cell of (a) for off-target site effects as well as for the presence said at least one nucleotide modification of said nucleotide sequence; and,
c) selecting a non-conventional yeast cell from (b) that has said at least one nucleotide modification of said nucleotide sequence while having reduced or no off-target site effects.

6. A method for selecting a non-conventional yeast cell comprising a polynucleotide of interest inserted into a target site in its genome, the method comprising:
a) providing to a non-conventional yeast cell, at least one guide RNA, at least one polynucleotide donor DNA comprising a polynucleotide of interest, at least one Cas endonuclease capable of introducing a double strand break at a target site in the genome of said cell, and an inhibitor of a DNA Ligase IV (LIG4), wherein the inhibitor is Scr7,
b) evaluating the non-conventional yeast cell of (a) for off-target site effects as well as for the presence said at least one polynucleotide of interest; and,
c) selecting a non-conventional yeast cell from (b) that has said at least one polynucleotide of interest while having reduced or no off-target site effects.

7. The method of claim 1 or claim 4, wherein the alteration at said target site is selected from the group of (i) at least one nucleotide deletion, (ii) at least one nucleotide substitution, (iii) at least one nucleotide insertion, or (iv) any one combination of (i)-(iii).

8. The method of any one of claims 4-6, further determining the frequency of Homologous Directed Repair (HDR) and/or Non-Homologous End Joining (NHEJ) in said cell.

9. The method of claim 8, wherein the frequency of HDR is increased when compared to the frequency of HDR derived from a control method lacking the inhibitor.

10. The method of claim 8, wherein the frequency of NHEJ is decreased when compared to the frequency of NHEJ derived from a control method lacking the inhibitor.

11. The method of any one of claims 1 to 10, wherein the non-conventional yeast is a member of a genus selected from the group consisting of *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces,* and *Pachysolen.*

## Patentansprüche

1. Verfahren zum Ändern einer Zielstelle in dem Genom einer nicht konventionellen Hefezelle, wobei das Verfahren Bereitstellen für eine nicht konventionelle Hefezelle mindestens einer Leit-RNA, mindestens einer Cas-Endonuclease, die zum Einführen eines Doppelstrangbruchs an der Zielstelle in der Lage ist, und eines Inhibitors einer DNA-Ligase IV (LIG4) umfasst, wobei der Inhibitor Scr7 ist.

2. Verfahren nach Anspruch 1, wobei die Zelle in einem Medium aufgezogen wird, das den Inhibitor bei einer Konzentration von mindestens 0,5 µmolar für mindestens 6 Stunden bei einer Temperatur von mindestens 20 °C vor Bereitstellen der Leit-RNA und der Cas-Endonuclease für die Zelle umfasst.

3. Verfahren zum Editing einer Nucleotidsequenz in dem Genom einer nicht konventionellen Hefezelle, wobei das Verfahren Bereitstellen für eine nicht konventionelle Hefezelle mindestens einer Leit-RNA, mindestens eines Polynucleotid-Modifikationstemplate, umfassend mindestens eine Nucleotid-Modifikation der Nucleotidsequenz, mindestens einer Cas-Endonuclease, die zum Einführen eines Doppelstrangbruchs an einer Zielstelle in dem Genom der Zelle in der Lage ist, und eines Inhibitors einer DNA-Ligase IV (LIG4) umfasst, wobei der Inhibitor Scr7 ist.

4. Verfahren zum Selektieren einer nicht konventionellen Hefezelle, umfassend eine veränderte Zielsequenz, wobei das Verfahren umfasst:
a) Bereitstellen für eine nicht konventionellen Hefezelle mindestens einer Leit-RNA, mindestens einer Cas-Endonuclease, die zum Einführen eines Doppelstrangbruchs an der Zielstelle in dem Genom der nicht konventionellen Hefezelle in der Lage ist, und eines Inhibitors einer DNA-Ligase IV (LIG4), wobei der Inhibitor Scr7 ist,
b) Bewerten der nicht konventionellen Hefezelle von (a) auf off-Zielstellen-Effekte sowie auf das Vorhandensein der veränderten Zielsequenz; und
c) Selektieren einer nicht konventionellen Hefezelle von (b), welche die veränderte Zielstelle aufweist, obwohl verminderte oder keine off-Zielstellen-Effekte aufweisend.

5. Verfahren zum Selektieren einer editierten nicht konventionellen Hefezelle, wobei das Verfahren umfasst:
a) Bereitstellen für eine nicht konventionelle Hefezelle, umfassend eine Nucleotidsequenz, die editiert werden soll, mindestens einer Leit-RNA, mindestens eines Polynucleotid-Modifikationstemplates, das mindestens eine Nucleotid-Modifikation der Nucleotidsequenz umfasst, mindestens einer Cas-Endonuclease, die zum Einführen eines Doppelstrangbruchs an einer Zielstelle in dem Genom der Zelle in der Lage ist, und eines Inhibitors einer DNA-Ligase IV (LIG4), wobei der Inhibitor Scr7 ist,
b) Bewerten der nicht konventionellen Hefezelle von (a) auf off-Zielstellen-Effekte sowie auf das Vorhandensein der mindestens einen Nucleotid-Modifikation der Nucleotidsequenz; und
c) Selektieren einer nicht konventionellen Hefezelle von (b), welche die mindestens eine Nucleotid-Modifikation der Nucleotidsequenz aufweist, obwohl verminderte oder keine off-Zielstellen-Effekte aufweisend.

6. Verfahren zum Selektieren einer nicht konventionellen Hefezelle, umfassend ein Polynucleotid von Interesse, das in eine Zielstelle in seinem Genom insertiert ist, wobei das Verfahren umfasst:
a) Bereitstellen für eine nicht konventionelle Hefezelle mindestens einer Leit-RNA, mindestens einer Polynucleotid Donor-DNA, umfassend ein Polynucleotid von Interesse, mindestens einer Cas-Endonuclease, die zum Einführen eines Doppelstrangbruchs an einer Zielstelle in dem Genom der Zelle in der Lage ist, und eines Inhibitors einer DNA-Ligase IV (LIG4), wobei der Inhibitor Scr7 ist.
b) Bewerten der nicht konventionellen Hefezelle von (a) auf off-Zielstellen-Effekte sowie auf das Vorhandensein des mindestens einen Polynucleotids von Interesse; und
c) Selektieren einer nicht konventionellen Hefezelle von (b), welche das mindestens eine Polynucleotid von Interesse aufweist, obwohl verminderte oder keine off-Zielstellen-Effekte aufweisend.

7. Verfahren nach Anspruch 1 oder Anspruch 4, wobei die Veränderung an der Zielstelle ausgewählt ist aus der Gruppe von (i) mindestens einer Nucleotid Deletion, (ii) mindestens einer Nucleotid Substitution, (iii) mindestens einer Nucleotid Insertion oder (iv) einer beliebigen Kombination von (i) bis (iii).

8. Verfahren nach einem der Ansprüche 4 bis 6, wobei ferner die Frequenz von homologer gesteuerter Reparatur (HDR) und/oder nichthomologer Endverbindung (NHEJ) in der Zelle bestimmt wird.

9. Verfahren nach Anspruch 8, wobei die Frequenz von HDR, verglichen mit der Frequenz von HDR, die von einem Kontrollverfahren abgeleitet wird, bei dem der Inhibitor fehlt, erhöht ist,.

10. Verfahren nach Anspruch 8, wobei die Frequenz von NHEJ verglichen mit der Frequenz von NHEJ, die von einem Kontrollverfahren abgeleitet wird, bei dem der Inhibitor fehlt, verringert ist.

11. Verfahren nach einem der Ansprüche 1 bis 10, wobei die nicht konventionelle Hefe ein Vertreter einer Gattung ist, die ausgewählt ist aus der Gruppe bestehend aus *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces* und *Pachysolen.*

## Revendications

1. Procédé pour la modification d'un site cible dans le génome d'une cellule de levure non conventionnelle, le procédé comprenant la fourniture à une cellule de levure non conventionnelle d'au moins un ARN de guidage, d'au moins une endonucléase Cas capable d'introduire une cassure double brin au niveau du site cible et d'un inhibiteur d'une ADN ligase IV (LIG4), dans lequel l'inhibiteur est Scr7.

2. Procédé selon la revendication 1, dans lequel la cellule est mise à croître dans un milieu comprenant ledit inhibiteur à une concentration d'au moins 0,5 micromolaire, pendant au moins 6 heures, à une température d'au moins 20°C avant la fourniture de l'ARN de guidage et de l'endonucléase Cas à la cellule.

3. Procédé pour l'édition d'une séquence de nucléotides dans le génome d'une cellule de levure non conventionnelle, le procédé comprenant la fourniture à une cellule de levure non conventionnelle d'au moins un ARN de guidage, d'au moins une matrice de modification de polynucléotide comprenant au moins une modification de nucléotide de la séquence de nucléotides, d'au moins une endonucléase Cas capable d'introduire une cassure double brin au niveau d'un site cible dans le génome de ladite cellule et d'un inhibiteur d'une ADN ligase IV (LIG4), dans lequel l'inhibiteur est Scr7.

4. Procédé pour la sélection d'une cellule de levure non conventionnelle comprenant une séquence cible modifiée, le procédé comprenant:
a) la fourniture à une cellule de levure non conventionnelle d'au moins un ARN de guidage, d'au moins une endonucléase Cas capable d'introduire une cassure double brin au niveau d'un site cible dans le génome de ladite cellule de levure non conventionnelle et d'un inhibiteur d'une ADN ligase IV (LIG4), dans lequel l'inhibiteur est Scr7,
b) l'évaluation de la cellule de levure non conventionnelle de (a) sur les effets hors-site cible aussi bien que sur la présence de ladite séquence cible modifiée; et
c) la sélection d'une cellule de levure non conventionnelle à partir de (b) qui comporte ledit site cible modifié tout en ayant des effets hors-site cible réduits ou pas d'effets.

5. Procédé pour la sélection d'une cellule de levure non conventionnelle éditée, le procédé comprenant:
a) la fourniture à une cellule de levure non conventionnelle comprenant une séquence de nucléotides à éditer d'au moins un ARN de guidage, d'au moins une matrice de modification de polynucléotide comprenant au moins une modification de nucléotide de ladite séquence de nucléotides, d'au moins une endonucléase Cas capable d'introduire une cassure double brin au niveau d'un site cible dans le génome de ladite cellule et d'un inhibiteur d'une ADN ligase IV (LIG4), dans lequel l'inhibiteur est Scr7,
b) l'évaluation de la cellule de levure non conventionnelle de (a) sur les effets hors-site cible ainsi que sur la présence de ladite au moins une modification de nucléotide de ladite séquence de nucléotides; et
c) la sélection d'une cellule de levure non conventionnelle à partir de (b) qui possède ladite au moins une modification de nucléotide de ladite séquence de nucléotides tout en ayant des effets hors-site cible réduits ou pas d'effets.

6. Procédé pour la sélection d'une cellule de levure non conventionnelle comprenant un polynucléotide d'intérêt inséré dans un site cible dans son génome, le procédé comprenant:
a) la fourniture à une cellule de levure non conventionnelle d'au moins un ARN de guidage, d'au moins un ADN donneur de polynucléotide comprenant un polynucléotide d'intérêt, d'au moins une endonucléase Cas capable d'introduire une cassure double brin au niveau d'un site cible dans le génome de ladite cellule et d'un inhibiteur d'une ADN ligase IV (LIG4), dans lequel l'inhibiteur est Scr7,
b) l'évaluation de la cellule de levure non conventionnelle de (a) sur les effets hors-site cible ainsi que sur la présence dudit au moins un polynucléotide d'intérêt; et
c) la sélection d'une cellule de levure non conventionnelle à partir de (b) qui possède ledit au moins un polynucléotide d'intérêt tout en ayant des effets hors-site cible réduits ou pas d'effets.

7. Procédé selon la revendication 1 ou la revendication 4, dans lequel la modification au niveau dudit site cible est sélectionnée dans le groupe de (i) au moins une délétion de nucléotide, (ii) au moins une substitution de nucléotide, (iii) au moins une insertion de nucléotide, ou (iv) n'importe quelle combinaison de (i)-(iii).

8. Procédé selon l'une quelconque des revendications 4-6, déterminant en outre la fréquence de réparation dirigée homologue (HDR) et/ou de ligature d'extrémités non homologues (NHEJ) dans ladite cellule.

9. Procédé selon la revendication 8, dans lequel la fréquence de HDR est augmentée lorsqu'elle est comparée à la fréquence de HDR dérivée d'un procédé témoin sans l'inhibiteur.

10. Procédé selon la revendication 8, dans lequel la fréquence de NHEJ est diminuée lorsqu'elle est comparée à la fréquence de NHEJ dérivée d'un procédé témoin sans l'inhibiteur.

11. Procédé selon l'une quelconque des revendications 1 à 10, dans lequel la levure non conventionnelle est un membre d'un genre choisi dans le groupe constitué de *Yarrowia, Pichia, Schwanniomyces, Kluyveromyces, Arxula, Trichosporon, Candida, Ustilago, Torulopsis, Zygosaccharomyces, Trigonopsis, Cryptococcus, Rhodotorula, Phaffia, Sporobolomyces* et *Pachysolen.*


### SEQUENCE LISTING

<110> E. I. du Pont de Nemours and Company Frisch, Ryan L.
<120> METHODS AND COMPOSITIONS FOR ENHANCED NUCLEASE-MEDIATED GENOME MODIFICATION AND REDUCED OFF-TARGET SITE EFFECTS
<130> CL6501WOPCT
<150> 62/266,051
   <151> 2015-12-11
<160> 49
<170> PatentIn version 3.5
<210> 1
   <211> 1372
   <212> PRT
   <213> Streptococcus pyogenes
<400> 1
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225                 230                 235                 240
   Leu Ile Ala Leu Ser Leu Gly Leu Thr Pro Asn Phe Lys Ser Asn Phe
                   245                 250                 255
   Asp Leu Ala Glu Asp Ala Lys Leu Gln Leu Ser Lys Asp Thr Tyr Asp
               260                 265                 270
   Asp Asp Leu Asp Asn Leu Leu Ala Gln Ile Gly Asp Gln Tyr Ala Asp
           275                 280                 285
   Leu Phe Leu Ala Ala Lys Asn Leu Ser Asp Ala Ile Leu Leu Ser Asp
       290                 295                 300
   Ile Leu Arg Val Asn Thr Glu Ile Thr Lys Ala Pro Leu Ser Ala Ser
   305                 310                 315                 320
   Met Ile Lys Arg Tyr Asp Glu His His Gln Asp Leu Thr Leu Leu Lys
                   325                 330                 335
   Ala Leu Val Arg Gln Gln Leu Pro Glu Lys Tyr Lys Glu Ile Phe Phe
               340                 345                 350
   Asp Gln Ser Lys Asn Gly Tyr Ala Gly Tyr Ile Asp Gly Gly Ala Ser
           355                 360                 365
   Gln Glu Glu Phe Tyr Lys Phe Ile Lys Pro Ile Leu Glu Lys Met Asp
       370                 375                 380
   Gly Thr Glu Glu Leu Leu Val Lys Leu Asn Arg Glu Asp Leu Leu Arg
   385                 390                 395                 400
   Lys Gln Arg Thr Phe Asp Asn Gly Ser Ile Pro His Gln Ile His Leu
                   405                 410                 415
   Gly Glu Leu His Ala Ile Leu Arg Arg Gln Glu Asp Phe Tyr Pro Phe
               420                 425                 430
   Leu Lys Asp Asn Arg Glu Lys Ile Glu Lys Ile Leu Thr Phe Arg Ile
           435                 440                 445
   Pro Tyr Tyr Val Gly Pro Leu Ala Arg Gly Asn Ser Arg Phe Ala Trp
       450                 455                 460
   Met Thr Arg Lys Ser Glu Glu Thr Ile Thr Pro Trp Asn Phe Glu Glu
   465                 470                 475                 480
   Val Val Asp Lys Gly Ala Ser Ala Gln Ser Phe Ile Glu Arg Met Thr
                   485                 490                 495
   Asn Phe Asp Lys Asn Leu Pro Asn Glu Lys Val Leu Pro Lys His Ser
               500                 505                 510
   Leu Leu Tyr Glu Tyr Phe Thr Val Tyr Asn Glu Leu Thr Lys Val Lys
           515                 520                 525
   Tyr Val Thr Glu Gly Met Arg Lys Pro Ala Phe Leu Ser Gly Glu Gln
       530                 535                 540
   Lys Lys Ala Ile Val Asp Leu Leu Phe Lys Thr Asn Arg Lys Val Thr
   545                 550                 555                 560
   Val Lys Gln Leu Lys Glu Asp Tyr Phe Lys Lys Ile Glu Cys Phe Asp
                   565                 570                 575
   Ser Val Glu Ile Ser Gly Val Glu Asp Arg Phe Asn Ala Ser Leu Gly
               580                 585                 590
   Thr Tyr His Asp Leu Leu Lys Ile Ile Lys Asp Lys Asp Phe Leu Asp
           595                 600                 605
   Asn Glu Glu Asn Glu Asp Ile Leu Glu Asp Ile Val Leu Thr Leu Thr
       610                 615                 620
   Leu Phe Glu Asp Arg Glu Met Ile Glu Glu Arg Leu Lys Thr Tyr Ala
   625                 630                 635                 640
   His Leu Phe Asp Asp Lys Val Met Lys Gln Leu Lys Arg Arg Arg Tyr
                   645                 650                 655
   Thr Gly Trp Gly Arg Leu Ser Arg Lys Leu Ile Asn Gly Ile Arg Asp
               660                 665                 670
   Lys Gln Ser Gly Lys Thr Ile Leu Asp Phe Leu Lys Ser Asp Gly Phe
           675                 680                 685
   Ala Asn Arg Asn Phe Met Gln Leu Ile His Asp Asp Ser Leu Thr Phe
       690                 695                 700
   Lys Glu Asp Ile Gln Lys Ala Gln Val Ser Gly Gln Gly Asp Ser Leu
   705                 710                 715                 720
   His Glu His Ile Ala Asn Leu Ala Gly Ser Pro Ala Ile Lys Lys Gly
                   725                 730                 735
   Ile Leu Gln Thr Val Lys Val Val Asp Glu Leu Val Lys Val Met Gly
               740                 745                 750
   Arg His Lys Pro Glu Asn Ile Val Ile Glu Met Ala Arg Glu Asn Gln
           755                 760                 765
   Thr Thr Gln Lys Gly Gln Lys Asn Ser Arg Glu Arg Met Lys Arg Ile
       770                 775                 780
   Glu Glu Gly Ile Lys Glu Leu Gly Ser Gln Ile Leu Lys Glu His Pro
   785                 790                 795                 800
   Val Glu Asn Thr Gln Leu Gln Asn Glu Lys Leu Tyr Leu Tyr Tyr Leu
                       805                 810                 815
   Gln Asn Gly Arg Asp Met Tyr Val Asp Gln Glu Leu Asp Ile Asn Arg
                   820                 825                 830
   Leu Ser Asp Tyr Asp Val Asp His Ile Val Pro Gln Ser Phe Leu Lys
               835                 840                 845
   Asp Asp Ser Ile Asp Asn Lys Val Leu Thr Arg Ser Asp Lys Asn Arg
           850                 855                 860
   Gly Lys Ser Asp Asn Val Pro Ser Glu Glu Val Val Lys Lys Met Lys
   865                 870                 875                 880
   Asn Tyr Trp Arg Gln Leu Leu Asn Ala Lys Leu Ile Thr Gln Arg Lys
                       885                 890                 895
   Phe Asp Asn Leu Thr Lys Ala Glu Arg Gly Gly Leu Ser Glu Leu Asp
                   900                 905                 910
   Lys Ala Gly Phe Ile Lys Arg Gln Leu Val Glu Thr Arg Gln Ile Thr
               915                 920                 925
   Lys His Val Ala Gln Ile Leu Asp Ser Arg Met Asn Thr Lys Tyr Asp
           930                 935                 940
   Glu Asn Asp Lys Leu Ile Arg Glu Val Lys Val Ile Thr Leu Lys Ser
   945                 950                 955                 960
   Lys Leu Val Ser Asp Phe Arg Lys Asp Phe Gln Phe Tyr Lys Val Arg
                       965                 970                 975
   Glu Ile Asn Asn Tyr His His Ala His Asp Ala Tyr Leu Asn Ala Val
                   980                 985                 990
   Val Gly Thr Ala Leu Ile Lys Lys  Tyr Pro Lys Leu Glu  Ser Glu Phe
               995                 1000                1005
   Val Tyr  Gly Asp Tyr Lys Val  Tyr Asp Val Arg Lys  Met Ile Ala
       1010                 1015                 1020
   Lys Ser  Glu Gln Glu Ile Gly  Lys Ala Thr Ala Lys  Tyr Phe Phe
       1025                 1030                 1035
   Tyr Ser  Asn Ile Met Asn Phe  Phe Lys Thr Glu Ile  Thr Leu Ala
       1040                 1045                 1050
   Asn Gly  Glu Ile Arg Lys Arg  Pro Leu Ile Glu Thr  Asn Gly Glu
       1055                 1060                 1065
   Thr Gly  Glu Ile Val Trp Asp  Lys Gly Arg Asp Phe  Ala Thr Val
       1070                 1075                 1080
   Arg Lys  Val Leu Ser Met Pro  Gln Val Asn Ile Val  Lys Lys Thr
       1085                 1090                 1095
   Glu Val  Gln Thr Gly Gly Phe  Ser Lys Glu Ser Ile  Leu Pro Lys
       1100                 1105                 1110
   Arg Asn  Ser Asp Lys Leu Ile  Ala Arg Lys Lys Asp  Trp Asp Pro
       1115                 1120                 1125
   Lys Lys  Tyr Gly Gly Phe Asp  Ser Pro Thr Val Ala  Tyr Ser Val
       1130                 1135                 1140
   Leu Val  Val Ala Lys Val Glu  Lys Gly Lys Ser Lys  Lys Leu Lys
       1145                 1150                 1155
   Ser Val  Lys Glu Leu Leu Gly  Ile Thr Ile Met Glu  Arg Ser Ser
       1160                 1165                 1170
   Phe Glu  Lys Asn Pro Ile Asp  Phe Leu Glu Ala Lys  Gly Tyr Lys
       1175                 1180                 1185
   Glu Val  Lys Lys Asp Leu Ile  Ile Lys Leu Pro Lys  Tyr Ser Leu
       1190                 1195                 1200
   Phe Glu  Leu Glu Asn Gly Arg  Lys Arg Met Leu Ala  Ser Ala Gly
       1205                 1210                 1215
   Glu Leu  Gln Lys Gly Asn Glu  Leu Ala Leu Pro Ser  Lys Tyr Val
       1220                 1225                 1230
   Asn Phe  Leu Tyr Leu Ala Ser  His Tyr Glu Lys Leu  Lys Gly Ser
       1235                 1240                 1245
   Pro Glu  Asp Asn Glu Gln Lys  Gln Leu Phe Val Glu  Gln His Lys
       1250                 1255                 1260
   His Tyr  Leu Asp Glu Ile Ile  Glu Gln Ile Ser Glu  Phe Ser Lys
       1265                 1270                 1275
   Arg Val  Ile Leu Ala Asp Ala  Asn Leu Asp Lys Val  Leu Ser Ala
       1280                 1285                 1290
   Tyr Asn  Lys His Arg Asp Lys  Pro Ile Arg Glu Gln  Ala Glu Asn
       1295                 1300                 1305
   Ile Ile  His Leu Phe Thr Leu  Thr Asn Leu Gly Ala  Pro Ala Ala
       1310                 1315                 1320
   Phe Lys  Tyr Phe Asp Thr Thr  Ile Asp Arg Lys Arg  Tyr Thr Ser
       1325                 1330                 1335
   Thr Lys  Glu Val Leu Asp Ala  Thr Leu Ile His Gln  Ser Ile Thr
       1340                 1345                 1350
   Gly Leu  Tyr Glu Thr Arg Ile  Asp Leu Ser Gln Leu  Gly Gly Asp
       1355                 1360                 1365
   Ser Arg  Ala Asp
       1370
<210> 2
   <211> 4140
   <212> DNA
   <213> Artificial sequence
<220>
   <223> Yarrowia codon optimized Cas9
<400> 2
   atggacaaga aatactccat cggcctggac attggaacca actctgtcgg ctgggctgtc      60
   atcaccgacg agtacaaggt gccctccaag aaattcaagg tcctcggaaa caccgatcga     120
   cactccatca agaaaaacct cattggtgcc ctgttgttcg attctggcga gactgccgaa     180
   gctaccagac tcaagcgaac tgctcggcga cgttacaccc gacggaagaa ccgaatctgc     240
   tacctgcagg agatcttttc caacgagatg gccaaggtgg acgattcgtt ctttcatcga     300
   ctggaggaat ccttcctcgt cgaggaagac aagaaacacg agcgtcatcc catctttggc     360
   aacattgtgg acgaggttgc ttaccacgag aagtatccta ccatctacca tctccgaaag     420
   aaactcgtcg attccaccga caaggcggat ctcagactta tctacctcgc tctggcacac     480
   atgatcaagt ttcgaggtca tttcctcatc gagggcgatc tcaatcccga caacagcgat     540
   gtggacaagc tgttcattca gctcgttcag acctacaacc agctgttcga ggaaaacccc     600
   atcaatgcct ccggagtcga tgcaaaggcc atcttgtctg ctcgactctc gaagagcaga     660
   cgactggaga acctcattgc ccaacttcct ggcgagaaaa agaacggact gtttggcaac     720
   ctcattgccc tttctcttgg tctcacaccc aacttcaagt ccaacttcga tctggcggag     780
   gacgccaagc tccagctgtc caaggacacc tacgacgatg acctcgacaa cctgcttgca     840
   cagattggcg atcagtacgc cgacctgttt ctcgctgcca agaacctttc ggatgctatt     900
   ctcttgtctg acattctgcg agtcaacacc gagatcacaa aggctcccct ttctgcctcc     960
   atgatcaagc gatacgacga gcaccatcag gatctcacac tgctcaaggc tcttgtccga    1020
   cagcaactgc ccgagaagta caaggagatc tttttcgatc agtcgaagaa cggctacgct    1080
   ggatacatcg acggcggagc ctctcaggaa gagttctaca agttcatcaa gccaattctc    1140
   gagaagatgg acggaaccga ggaactgctt gtcaagctca atcgagagga tctgcttcgg    1200
   aagcaacgaa ccttcgacaa cggcagcatt cctcatcaga tccacctcgg tgagctgcac    1260
   gccattcttc gacgtcagga agacttctac ccctttctca aggacaaccg agagaagatc    1320
   gagaagattc ttacctttcg aatcccctac tatgttggtc ctcttgccag aggaaactct    1380
   cgatttgctt ggatgactcg aaagtccgag gaaaccatca ctccctggaa cttcgaggaa    1440
   gtcgtggaca agggtgcctc tgcacagtcc ttcatcgagc gaatgaccaa cttcgacaag    1500
   aatctgccca acgagaaggt tcttcccaag cattcgctgc tctacgagta ctttacagtc    1560
   tacaacgaac tcaccaaagt caagtacgtt accgagggaa tgcgaaagcc tgccttcttg    1620
   tctggcgaac agaagaaagc cattgtcgat ctcctgttca agaccaaccg aaaggtcact    1680
   gttaagcagc tcaaggagga ctacttcaag aaaatcgagt gtttcgacag cgtcgagatt    1740
   tccggagttg aggaccgatt caacgcctct ttgggcacct atcacgatct gctcaagatt    1800
   atcaaggaca aggattttct cgacaacgag gaaaacgagg acattctgga ggacatcgtg    1860
   ctcactctta ccctgttcga agatcgggag atgatcgagg aacgactcaa gacatacgct    1920
   cacctgttcg acgacaaggt catgaaacaa ctcaagcgac gtagatacac cggctgggga    1980
   agactttcgc gaaagctcat caacggcatc agagacaagc agtccggaaa gaccattctg    2040
   gactttctca agtccgatgg ctttgccaac cgaaacttca tgcagctcat tcacgacgat    2100
   tctcttacct tcaaggagga catccagaag gcacaagtgt ccggtcaggg cgacagcttg    2160
   cacgaacata ttgccaacct ggctggttcg ccagccatca agaaaggcat tctccagact    2220
   gtcaaggttg tcgacgagct ggtgaaggtc atgggacgtc acaagcccga gaacattgtg    2280
   atcgagatgg ccagagagaa ccagacaact caaaagggtc agaaaaactc gcgagagcgg    2340
   atgaagcgaa tcgaggaagg catcaaggag ctgggatccc agattctcaa ggagcatccc    2400
   gtcgagaaca ctcaactgca gaacgagaag ctgtatctct actatctgca gaatggtcga    2460
   gacatgtacg tggatcagga actggacatc aatcgtctca gcgactacga tgtggaccac      2520
   attgtccctc aatcctttct caaggacgat tctatcgaca acaaggtcct tacacgatcc      2580
   gacaagaaca gaggcaagtc ggacaacgtt cccagcgaag aggtggtcaa aaagatgaag      2640
   aactactggc gacagctgct caacgccaag ctcattaccc agcgaaagtt cgacaatctt      2700
   accaaggccg agcgaggcgg tctgtccgag ctcgacaagg ctggcttcat caagcgtcaa      2760
   ctcgtcgaga ccagacagat cacaaagcac gtcgcacaga ttctcgattc tcggatgaac      2820
   accaagtacg acgagaacga caagctcatc cgagaggtca aggtgattac tctcaagtcc      2880
   aaactggtct ccgatttccg aaaggacttt cagttctaca aggtgcgaga gatcaacaat      2940
   taccaccatg cccacgatgc ttacctcaac gccgtcgttg gcactgcgct catcaagaaa      3000
   taccccaagc tcgaaagcga gttcgtttac ggcgattaca aggtctacga cgttcgaaag      3060
   atgattgcca agtccgaaca ggagattggc aaggctactg ccaagtactt cttttactcc      3120
   aacatcatga actttttcaa gaccgagatc accttggcca acggagagat tcgaaagaga      3180
   ccacttatcg agaccaacgg cgaaactgga gagatcgtgt gggacaaggg tcgagacttt      3240
   gcaaccgtgc gaaaggttct gtcgatgcct caggtcaaca tcgtcaagaa aaccgaggtt      3300
   cagactggcg gattctccaa ggagtcgatt ctgcccaagc gaaactccga caagctcatc      3360
   gctcgaaaga aagactggga tcccaagaaa tacggtggct tcgattctcc taccgtcgcc      3420
   tattccgtgc ttgtcgttgc gaaggtcgag aagggcaagt ccaaaaagct caagtccgtc      3480
   aaggagctgc tcggaattac catcatggag cgatcgagct tcgagaagaa tcccatcgac      3540
   ttcttggaag ccaagggtta caaggaggtc aagaaagacc tcattatcaa gctgcccaag      3600
   tactctctgt tcgaactgga gaacggtcga aagcgtatgc tcgcctccgc tggcgagctg      3660
   cagaagggaa acgagcttgc cttgccttcg aagtacgtca actttctcta tctggcttct      3720
   cactacgaga agctcaaggg ttctcccgag gacaacgaac agaagcaact cttcgttgag      3780
   cagcacaaac attacctcga cgagattatc gagcagattt ccgagttttc gaagcgagtc      3840
   atcctggctg atgccaactt ggacaaggtg ctctctgcct acaacaagca tcgggacaaa      3900
   cccattcgag aacaggcgga gaacatcatt cacctgttta ctcttaccaa cctgggtgct      3960
   cctgcagctt tcaagtactt cgataccact atcgaccgaa agcggtacac atccaccaag      4020
   gaggttctcg atgccaccct gattcaccag tccatcactg gcctgtacga gacccgaatc      4080
   gacctgtctc agcttggtgg cgactccaga gccgatccca agaaaaagcg aaaggtctaa      4140
<210> 3
   <211> 7
   <212> PRT
   <213> SV40
<400> 3
   Pro Lys Lys Lys Arg Lys Val
   1               5
<210> 4
   <211> 543
   <212> DNA
   <213> YArrowia lipolytica
<400> 4
   tcgacgttta aaccatcatc taagggcctc aaaactacct cggaactgct gcgctgatct        60
   ggacaccaca gaggttccga gcactttagg ttgcaccaaa tgtcccacca ggtgcaggca       120
   gaaaacgctg gaacagcgtg tacagtttgt cttaacaaaa agtgagggcg ctgaggtcga       180
   gcagggtggt gtgacttgtt atagccttta gagctgcgaa agcgcgtatg gatttggctc     240
   atcaggccag attgagggtc tgtggacaca tgtcatgtta gtgtacttca atcgccccct     300
   ggatatagcc ccgacaatag gccgtggcct catttttttg ccttccgcac atttccattg       360
   ctcggtaccc acaccttgct tctcctgcac ttgccaacct taatactggt ttacattgac       420
   caacatctta caagcggggg gcttgtctag ggtatatata aacagtggct ctcccaatcg       480
   gttgccagtc tcttttttcc tttctttccc cacagattcg aaatctaaac tacacatcac       540
   acc                                                                     543
<210> 5
   <211> 4683
   <212> DNA
   <213> Artificial sequence
<220>
   <223> Yarrowia optimized expression cassette
<400> 5
   tcgacgttta aaccatcatc taagggcctc aaaactacct cggaactgct gcgctgatct      60
   ggacaccaca gaggttccga gcactttagg ttgcaccaaa tgtcccacca ggtgcaggca     120
   gaaaacgctg gaacagcgtg tacagtttgt cttaacaaaa agtgagggcg ctgaggtcga     180
   gcagggtggt gtgacttgtt atagccttta gagctgcgaa agcgcgtatg gatttggctc     240
   atcaggccag attgagggtc tgtggacaca tgtcatgtta gtgtacttca atcgccccct    300
   ggatatagcc ccgacaatag gccgtggcct catttttttg ccttccgcac atttccattg     360
   ctcggtaccc acaccttgct tctcctgcac ttgccaacct taatactggt ttacattgac     420
   caacatctta caagcggggg gcttgtctag ggtatatata aacagtggct ctcccaatcg     480
   gttgccagtc tcttttttcc tttctttccc cacagattcg aaatctaaac tacacatcac    540
   accatggaca agaaatactc catcggcctg gacattggaa ccaactctgt cggctgggct     600
   gtcatcaccg acgagtacaa ggtgccctcc aagaaattca aggtcctcgg aaacaccgat     660
   cgacactcca tcaagaaaaa cctcattggt gccctgttgt tcgattctgg cgagactgcc     720
   gaagctacca gactcaagcg aactgctcgg cgacgttaca cccgacggaa gaaccgaatc     780
   tgctacctgc aggagatctt ttccaacgag atggccaagg tggacgattc gttctttcat     840
   cgactggagg aatccttcct cgtcgaggaa gacaagaaac acgagcgtca tcccatcttt     900
   ggcaacattg tggacgaggt tgcttaccac gagaagtatc ctaccatcta ccacctgcga     960
   aagaaactcg tcgattccac cgacaaggcg gatctcagac ttatctacct cgctctggca    1020
   cacatgatca agtttcgagg tcatttcctc atcgagggcg atctcaatcc cgacaacagc    1080
   gatgtggaca agctgttcat tcagctcgtt cagacctaca accagctgtt cgaggaaaac    1140
   cccatcaatg cctccggagt cgatgcaaag gccatcttgt ctgctcgact ctcgaagagc    1200
   agacgactgg agaacctcat tgcccaactt cctggcgaga aaaagaacgg actgtttggc    1260
   aacctcattg ccctttctct tggtctcaca cccaacttca agtccaactt cgatctggcg    1320
   gaggacgcca agctccagct gtccaaggac acctacgacg atgacctcga caacctgctt    1380
   gcacagattg gcgatcagta cgccgacctg tttctcgctg ccaagaacct ttcggatgct    1440
   attctcttgt ctgacattct gcgagtcaac accgagatca caaaggctcc cctttctgcc    1500
   tccatgatca agcgatacga cgagcaccat caggatctca cactgctcaa ggctcttgtc    1560
   cgacagcaac tgcccgagaa gtacaaggag atctttttcg atcagtcgaa gaacggctac    1620
   gctggataca tcgacggcgg agcctctcag gaagagttct acaagttcat caagccaatt    1680
   ctcgagaaga tggacggaac cgaggaactg cttgtcaagc tcaatcgaga ggatctgctt    1740
   cggaagcaac gaaccttcga caacggcagc attcctcatc agatccacct cggtgagctg    1800
   cacgccattc ttcgacgtca ggaagacttc tacccctttc tcaaggacaa ccgagagaag     1860
   atcgagaaga ttcttacctt tcgaatcccc tactatgttg gtcctcttgc cagaggaaac    1920
   tctcgatttg cttggatgac tcgaaagtcc gaggaaacca tcactccctg gaacttcgag    1980
   gaagtcgtgg acaagggtgc ctctgcacag tccttcatcg agcgaatgac caacttcgac    2040
   aagaatctgc ccaacgagaa ggttcttccc aagcattcgc tgctctacga gtactttaca    2100
   gtctacaacg aactcaccaa agtcaagtac gttaccgagg gaatgcgaaa gcctgccttc    2160
   ttgtctggcg aacagaagaa agccattgtc gatctcctgt tcaagaccaa ccgaaaggtc    2220
   actgttaagc agctcaagga ggactacttc aagaaaatcg agtgtttcga cagcgtcgag    2280
   atttccggag ttgaggaccg attcaacgcc tctttgggca cctatcacga tctgctcaag    2340
   attatcaagg acaaggattt tctcgacaac gaggaaaacg aggacattct ggaggacatc    2400
   gtgctcactc ttaccctgtt cgaagatcgg gagatgatcg aggaacgact caagacatac    2460
   gctcacctgt tcgacgacaa ggtcatgaaa caactcaagc gacgtagata caccggctgg    2520
   ggaagacttt cgcgaaagct catcaacggc atcagagaca agcagtccgg aaagaccatt    2580
   ctggactttc tcaagtccga tggctttgcc aaccgaaact tcatgcagct cattcacgac    2640
   gattctctta ccttcaagga ggacatccag aaggcacaag tgtccggtca gggcgacagc    2700
   ttgcacgaac atattgccaa cctggctggt tcgccagcca tcaagaaagg cattctccag    2760
   actgtcaagg ttgtcgacga gctggtgaag gtcatgggac gtcacaagcc cgagaacatt    2820
   gtgatcgaga tggccagaga gaaccagaca actcaaaagg gtcagaaaaa ctcgcgagag    2880
   cggatgaagc gaatcgagga aggcatcaag gagctgggat cccagattct caaggagcat    2940
   cccgtcgaga acactcaact gcagaacgag aagctgtatc tctactatct gcagaatggt    3000
   cgagacatgt acgtggatca ggaactggac atcaatcgtc tcagcgacta cgatgtggac    3060
   cacattgtcc ctcaatcctt tctcaaggac gattctatcg acaacaaggt ccttacacga    3120
   tccgacaaga acagaggcaa gtcggacaac gttcccagcg aagaggtggt caaaaagatg    3180
   aagaactact ggcgacagct gctcaacgcc aagctcatta cccagcgaaa gttcgacaat    3240
   cttaccaagg ccgagcgagg cggtctgtcc gagctcgaca aggctggctt catcaagcgt    3300
   caactcgtcg agaccagaca gatcacaaag cacgtcgcac agattctcga ttctcggatg    3360
   aacaccaagt acgacgagaa cgacaagctc atccgagagg tcaaggtgat tactctcaag    3420
   tccaaactgg tctccgattt ccgaaaggac tttcagttct acaaggtgcg agagatcaac    3480
   aattaccacc atgcccacga tgcttacctc aacgccgtcg ttggcactgc gctcatcaag    3540
   aaatacccca agctcgaaag cgagttcgtt tacggcgatt acaaggtcta cgacgttcga     3600
   aagatgattg ccaagtccga acaggagatt ggcaaggcta ctgccaagta cttcttttac    3660
   tccaacatca tgaacttttt caagaccgag atcaccttgg ccaacggaga gattcgaaag    3720
   agaccactta tcgagaccaa cggcgaaact ggagagatcg tgtgggacaa gggtcgagac      3780
   tttgcaaccg tgcgaaaggt tctgtcgatg cctcaggtca acatcgtcaa gaaaaccgag      3840
   gttcagactg gcggattctc caaggagtcg attctgccca agcgaaactc cgacaagctc      3900
   atcgctcgaa agaaagactg ggatcccaag aaatacggtg gcttcgattc tcctaccgtc      3960
   gcctattccg tgcttgtcgt tgcgaaggtc gagaagggca agtccaaaaa gctcaagtcc      4020
   gtcaaggagc tgctcggaat taccatcatg gagcgatcga gcttcgagaa gaatcccatc      4080
   gacttcttgg aagccaaggg ttacaaggag gtcaagaaag acctcattat caagctgccc      4140
   aagtactctc tgttcgaact ggagaacggt cgaaagcgta tgctcgcctc cgctggcgag      4200
   ctgcagaagg gaaacgagct tgccttgcct tcgaagtacg tcaactttct ctatctggct      4260
   tctcactacg agaagctcaa gggttctccc gaggacaacg aacagaagca actcttcgtt      4320
   gagcagcaca aacattacct cgacgagatt atcgagcaga tttccgagtt ttcgaagcga      4380
   gtcatcctgg ctgatgccaa cttggacaag gtgctctctg cctacaacaa gcatcgggac      4440
   aaacccattc gagaacaggc ggagaacatc attcacctgt ttactcttac caacctgggt      4500
   gctcctgcag ctttcaagta cttcgatacc actatcgacc gaaagcggta cacatccacc      4560
   aaggaggttc tcgatgccac cctgattcac cagtccatca ctggcctgta cgagacccga      4620
   atcgacctgt ctcagcttgg tggcgactcc agagccgatc ccaagaaaaa gcgaaaggtc      4680
   taa                                                                    4683
<210> 6
   <211> 10706
   <212> DNA
   <213> Artificial sequence
<220>
   <223> pZufCas9
<400> 6
   catggacaag aaatactcca tcggcctgga cattggaacc aactctgtcg gctgggctgt      60
   catcaccgac gagtacaagg tgccctccaa gaaattcaag gtcctcggaa acaccgatcg     120
   acactccatc aagaaaaacc tcattggtgc cctgttgttc gattctggcg agactgccga     180
   agctaccaga ctcaagcgaa ctgctcggcg acgttacacc cgacggaaga accgaatctg     240
   ctacctgcag gagatctttt ccaacgagat ggccaaggtg gacgattcgt tctttcatcg     300
   actggaggaa tccttcctcg tcgaggaaga caagaaacac gagcgtcatc ccatctttgg     360
   caacattgtg gacgaggttg cttaccacga gaagtatcct accatctacc acctgcgaaa     420
   gaaactcgtc gattccaccg acaaggcgga tctcagactt atctacctcg ctctggcaca     480
   catgatcaag tttcgaggtc atttcctcat cgagggcgat ctcaatcccg acaacagcga     540
   tgtggacaag ctgttcattc agctcgttca gacctacaac cagctgttcg aggaaaaccc     600
   catcaatgcc tccggagtcg atgcaaaggc catcttgtct gctcgactct cgaagagcag     660
   acgactggag aacctcattg cccaacttcc tggcgagaaa aagaacggac tgtttggcaa     720
   cctcattgcc ctttctcttg gtctcacacc caacttcaag tccaacttcg atctggcgga     780
   ggacgccaag ctccagctgt ccaaggacac ctacgacgat gacctcgaca acctgcttgc     840
   acagattggc gatcagtacg ccgacctgtt tctcgctgcc aagaaccttt cggatgctat     900
   tctcttgtct gacattctgc gagtcaacac cgagatcaca aaggctcccc tttctgcctc     960
   catgatcaag cgatacgacg agcaccatca ggatctcaca ctgctcaagg ctcttgtccg    1020
   acagcaactg cccgagaagt acaaggagat ctttttcgat cagtcgaaga acggctacgc    1080
   tggatacatc gacggcggag cctctcagga agagttctac aagttcatca agccaattct    1140
   cgagaagatg gacggaaccg aggaactgct tgtcaagctc aatcgagagg atctgcttcg    1200
   gaagcaacga accttcgaca acggcagcat tcctcatcag atccacctcg gtgagctgca    1260
   cgccattctt cgacgtcagg aagacttcta cccctttctc aaggacaacc gagagaagat    1320
   cgagaagatt cttacctttc gaatccccta ctatgttggt cctcttgcca gaggaaactc    1380
   tcgatttgct tggatgactc gaaagtccga ggaaaccatc actccctgga acttcgagga    1440
   agtcgtggac aagggtgcct ctgcacagtc cttcatcgag cgaatgacca acttcgacaa    1500
   gaatctgccc aacgagaagg ttcttcccaa gcattcgctg ctctacgagt actttacagt    1560
   ctacaacgaa ctcaccaaag tcaagtacgt taccgaggga atgcgaaagc ctgccttctt    1620
   gtctggcgaa cagaagaaag ccattgtcga tctcctgttc aagaccaacc gaaaggtcac    1680
   tgttaagcag ctcaaggagg actacttcaa gaaaatcgag tgtttcgaca gcgtcgagat    1740
   ttccggagtt gaggaccgat tcaacgcctc tttgggcacc tatcacgatc tgctcaagat    1800
   tatcaaggac aaggattttc tcgacaacga ggaaaacgag gacattctgg aggacatcgt    1860
   gctcactctt accctgttcg aagatcggga gatgatcgag gaacgactca agacatacgc    1920
   tcacctgttc gacgacaagg tcatgaaaca actcaagcga cgtagataca ccggctgggg    1980
   aagactttcg cgaaagctca tcaacggcat cagagacaag cagtccggaa agaccattct    2040
   ggactttctc aagtccgatg gctttgccaa ccgaaacttc atgcagctca ttcacgacga    2100
   ttctcttacc ttcaaggagg acatccagaa ggcacaagtg tccggtcagg gcgacagctt    2160
   gcacgaacat attgccaacc tggctggttc gccagccatc aagaaaggca ttctccagac     2220
   tgtcaaggtt gtcgacgagc tggtgaaggt catgggacgt cacaagcccg agaacattgt     2280
   gatcgagatg gccagagaga accagacaac tcaaaagggt cagaaaaact cgcgagagcg     2340
   gatgaagcga atcgaggaag gcatcaagga gctgggatcc cagattctca aggagcatcc     2400
   cgtcgagaac actcaactgc agaacgagaa gctgtatctc tactatctgc agaatggtcg     2460
   agacatgtac gtggatcagg aactggacat caatcgtctc agcgactacg atgtggacca     2520
   cattgtccct caatcctttc tcaaggacga ttctatcgac aacaaggtcc ttacacgatc     2580
   cgacaagaac agaggcaagt cggacaacgt tcccagcgaa gaggtggtca aaaagatgaa     2640
   gaactactgg cgacagctgc tcaacgccaa gctcattacc cagcgaaagt tcgacaatct     2700
   taccaaggcc gagcgaggcg gtctgtccga gctcgacaag gctggcttca tcaagcgtca     2760
   actcgtcgag accagacaga tcacaaagca cgtcgcacag attctcgatt ctcggatgaa     2820
   caccaagtac gacgagaacg acaagctcat ccgagaggtc aaggtgatta ctctcaagtc     2880
   caaactggtc tccgatttcc gaaaggactt tcagttctac aaggtgcgag agatcaacaa     2940
   ttaccaccat gcccacgatg cttacctcaa cgccgtcgtt ggcactgcgc tcatcaagaa     3000
   ataccccaag ctcgaaagcg agttcgttta cggcgattac aaggtctacg acgttcgaaa     3060
   gatgattgcc aagtccgaac aggagattgg caaggctact gccaagtact tcttttactc     3120
   caacatcatg aactttttca agaccgagat caccttggcc aacggagaga ttcgaaagag     3180
   accacttatc gagaccaacg gcgaaactgg agagatcgtg tgggacaagg gtcgagactt     3240
   tgcaaccgtg cgaaaggttc tgtcgatgcc tcaggtcaac atcgtcaaga aaaccgaggt     3300
   tcagactggc ggattctcca aggagtcgat tctgcccaag cgaaactccg acaagctcat     3360
   cgctcgaaag aaagactggg atcccaagaa atacggtggc ttcgattctc ctaccgtcgc     3420
   ctattccgtg cttgtcgttg cgaaggtcga gaagggcaag tccaaaaagc tcaagtccgt     3480
   caaggagctg ctcggaatta ccatcatgga gcgatcgagc ttcgagaaga atcccatcga     3540
   cttcttggaa gccaagggtt acaaggaggt caagaaagac ctcattatca agctgcccaa     3600
   gtactctctg ttcgaactgg agaacggtcg aaagcgtatg ctcgcctccg ctggcgagct     3660
   gcagaaggga aacgagcttg ccttgccttc gaagtacgtc aactttctct atctggcttc     3720
   tcactacgag aagctcaagg gttctcccga ggacaacgaa cagaagcaac tcttcgttga     3780
   gcagcacaaa cattacctcg acgagattat cgagcagatt tccgagtttt cgaagcgagt     3840
   catcctggct gatgccaact tggacaaggt gctctctgcc tacaacaagc atcgggacaa     3900
   acccattcga gaacaggcgg agaacatcat tcacctgttt actcttacca acctgggtgc     3960
   tcctgcagct ttcaagtact tcgataccac tatcgaccga aagcggtaca catccaccaa     4020
   ggaggttctc gatgccaccc tgattcacca gtccatcact ggcctgtacg agacccgaat     4080
   cgacctgtct cagcttggtg gcgactccag agccgatccc aagaaaaagc gaaaggtcta     4140
   agcggccgca agtgtggatg gggaagtgag tgcccggttc tgtgtgcaca attggcaatc     4200
   caagatggat ggattcaaca cagggatata gcgagctacg tggtggtgcg aggatatagc     4260
   aacggatatt tatgtttgac acttgagaat gtacgataca agcactgtcc aagtacaata     4320
   ctaaacatac tgtacatact catactcgta cccgggcaac ggtttcactt gagtgcagtg     4380
   gctagtgctc ttactcgtac agtgtgcaat actgcgtatc atagtctttg atgtatatcg     4440
   tattcattca tgttagttgc gtacgagccg gaagcataaa gtgtaaagcc tggggtgcct     4500
   aatgagtgag ctaactcaca ttaattgcgt tgcgctcact gcccgctttc cagtcgggaa     4560
   acctgtcgtg ccagctgcat taatgaatcg gccaacgcgc ggggagaggc ggtttgcgta     4620
   ttgggcgctc ttccgcttcc tcgctcactg actcgctgcg ctcggtcgtt cggctgcggc     4680
   gagcggtatc agctcactca aaggcggtaa tacggttatc cacagaatca ggggataacg     4740
   caggaaagaa catgtgagca aaaggccagc aaaaggccag gaaccgtaaa aaggccgcgt     4800
   tgctggcgtt tttccatagg ctccgccccc ctgacgagca tcacaaaaat cgacgctcaa     4860
   gtcagaggtg gcgaaacccg acaggactat aaagatacca ggcgtttccc cctggaagct     4920
   ccctcgtgcg ctctcctgtt ccgaccctgc cgcttaccgg atacctgtcc gcctttctcc     4980
   cttcgggaag cgtggcgctt tctcatagct cacgctgtag gtatctcagt tcggtgtagg     5040
   tcgttcgctc caagctgggc tgtgtgcacg aaccccccgt tcagcccgac cgctgcgcct     5100
   tatccggtaa ctatcgtctt gagtccaacc cggtaagaca cgacttatcg ccactggcag     5160
   cagccactgg taacaggatt agcagagcga ggtatgtagg cggtgctaca gagttcttga     5220
   agtggtggcc taactacggc tacactagaa ggacagtatt tggtatctgc gctctgctga     5280
   agccagttac cttcggaaaa agagttggta gctcttgatc cggcaaacaa accaccgctg     5340
   gtagcggtgg tttttttgtt tgcaagcagc agattacgcg cagaaaaaaa ggatctcaag     5400
   aagatccttt gatcttttct acggggtctg acgctcagtg gaacgaaaac tcacgttaag     5460
   ggattttggt catgagatta tcaaaaagga tcttcaccta gatcctttta aattaaaaat     5520
   gaagttttaa atcaatctaa agtatatatg agtaaacttg gtctgacagt taccaatgct     5580
   taatcagtga ggcacctatc tcagcgatct gtctatttcg ttcatccata gttgcctgac     5640
   tccccgtcgt gtagataact acgatacggg agggcttacc atctggcccc agtgctgcaa     5700
   tgataccgcg agacccacgc tcaccggctc cagatttatc agcaataaac cagccagccg     5760
   gaagggccga gcgcagaagt ggtcctgcaa ctttatccgc ctccatccag tctattaatt     5820
   gttgccggga agctagagta agtagttcgc cagttaatag tttgcgcaac gttgttgcca     5880
   ttgctacagg catcgtggtg tcacgctcgt cgtttggtat ggcttcattc agctccggtt     5940
   cccaacgatc aaggcgagtt acatgatccc ccatgttgtg caaaaaagcg gttagctcct    6000
   tcggtcctcc gatcgttgtc agaagtaagt tggccgcagt gttatcactc atggttatgg    6060
   cagcactgca taattctctt actgtcatgc catccgtaag atgcttttct gtgactggtg    6120
   agtactcaac caagtcattc tgagaatagt gtatgcggcg accgagttgc tcttgcccgg    6180
   cgtcaatacg ggataatacc gcgccacata gcagaacttt aaaagtgctc atcattggaa    6240
   aacgttcttc ggggcgaaaa ctctcaagga tcttaccgct gttgagatcc agttcgatgt    6300
   aacccactcg tgcacccaac tgatcttcag catcttttac tttcaccagc gtttctgggt    6360
   gagcaaaaac aggaaggcaa aatgccgcaa aaaagggaat aagggcgaca cggaaatgtt    6420
   gaatactcat actcttcctt tttcaatatt attgaagcat ttatcagggt tattgtctca    6480
   tgagcggata catatttgaa tgtatttaga aaaataaaca aataggggtt ccgcgcacat    6540
   ttccccgaaa agtgccacct gacgcgccct gtagcggcgc attaagcgcg gcgggtgtgg    6600
   tggttacgcg cagcgtgacc gctacacttg ccagcgccct agcgcccgct cctttcgctt    6660
   tcttcccttc ctttctcgcc acgttcgccg gctttccccg tcaagctcta aatcgggggc    6720
   tccctttagg gttccgattt agtgctttac ggcacctcga ccccaaaaaa cttgattagg    6780
   gtgatggttc acgtagtggg ccatcgccct gatagacggt ttttcgccct ttgacgttgg    6840
   agtccacgtt ctttaatagt ggactcttgt tccaaactgg aacaacactc aaccctatct    6900
   cggtctattc ttttgattta taagggattt tgccgatttc ggcctattgg ttaaaaaatg    6960
   agctgattta acaaaaattt aacgcgaatt ttaacaaaat attaacgctt acaatttcca    7020
   ttcgccattc aggctgcgca actgttggga agggcgatcg gtgcgggcct cttcgctatt    7080
   acgccagctg gcgaaagggg gatgtgctgc aaggcgatta agttgggtaa cgccagggtt    7140
   ttcccagtca cgacgttgta aaacgacggc cagtgaattg taatacgact cactataggg    7200
   cgaattgggt accgggcccc ccctcgaggt cgatggtgtc gataagcttg atatcgaatt    7260
   catgtcacac aaaccgatct tcgcctcaag gaaacctaat tctacatccg agagactgcc    7320
   gagatccagt ctacactgat taattttcgg gccaataatt taaaaaaatc gtgttatata    7380
   atattatatg tattatatat atacatcatg atgatactga cagtcatgtc ccattgctaa    7440
   atagacagac tccatctgcc gcctccaact gatgttctca atatttaagg ggtcatctcg    7500
   cattgtttaa taataaacag actccatcta ccgcctccaa atgatgttct caaaatatat    7560
   tgtatgaact tatttttatt acttagtatt attagacaac ttacttgctt tatgaaaaac    7620
   acttcctatt taggaaacaa tttataatgg cagttcgttc atttaacaat ttatgtagaa    7680
   taaatgttat aaatgcgtat gggaaatctt aaatatggat agcataaatg atatctgcat    7740
   tgcctaattc gaaatcaaca gcaacgaaaa aaatcccttg tacaacataa atagtcatcg    7800
   agaaatatca actatcaaag aacagctatt cacacgttac tattgagatt attattggac    7860
   gagaatcaca cactcaactg tctttctctc ttctagaaat acaggtacaa gtatgtacta    7920
   ttctcattgt tcatacttct agtcatttca tcccacatat tccttggatt tctctccaat    7980
   gaatgacatt ctatcttgca aattcaacaa ttataataag atataccaaa gtagcggtat    8040
   agtggcaatc aaaaagcttc tctggtgtgc ttctcgtatt tatttttatt ctaatgatcc    8100
   attaaaggta tatatttatt tcttgttata taatcctttt gtttattaca tgggctggat    8160
   acataaaggt attttgattt aattttttgc ttaaattcaa tcccccctcg ttcagtgtca    8220
   actgtaatgg taggaaatta ccatactttt gaagaagcaa aaaaaatgaa agaaaaaaaa    8280
   aatcgtattt ccaggttaga cgttccgcag aatctagaat gcggtatgcg gtacattgtt    8340
   cttcgaacgt aaaagttgcg ctccctgaga tattgtacat ttttgctttt acaagtacaa    8400
   gtacatcgta caactatgta ctactgttga tgcatccaca acagtttgtt ttgttttttt    8460
   ttgttttttt tttttctaat gattcattac cgctatgtat acctacttgt acttgtagta    8520
   agccgggtta ttggcgttca attaatcata gacttatgaa tctgcacggt gtgcgctgcg    8580
   agttactttt agcttatgca tgctacttgg gtgtaatatt gggatctgtt cggaaatcaa    8640
   cggatgctca atcgatttcg acagtaatta attaagtcat acacaagtca gctttcttcg    8700
   agcctcatat aagtataagt agttcaacgt attagcactg tacccagcat ctccgtatcg    8760
   agaaacacaa caacatgccc cattggacag atcatgcgga tacacaggtt gtgcagtatc    8820
   atacatactc gatcagacag gtcgtctgac catcatacaa gctgaacaag cgctccatac    8880
   ttgcacgctc tctatataca cagttaaatt acatatccat agtctaacct ctaacagtta    8940
   atcttctggt aagcctccca gccagccttc tggtatcgct tggcctcctc aataggatct    9000
   cggttctggc cgtacagacc tcggccgaca attatgatat ccgttccggt agacatgaca    9060
   tcctcaacag ttcggtactg ctgtccgaga gcgtctccct tgtcgtcaag acccaccccg    9120
   ggggtcagaa taagccagtc ctcagagtcg cccttaggtc ggttctgggc aatgaagcca    9180
   accacaaact cggggtcgga tcgggcaagc tcaatggtct gcttggagta ctcgccagtg    9240
   gccagagagc ccttgcaaga cagctcggcc agcatgagca gacctctggc cagcttctcg    9300
   ttgggagagg ggactaggaa ctccttgtac tgggagttct cgtagtcaga gacgtcctcc    9360
   ttcttctgtt cagagacagt ttcctcggca ccagctcgca ggccagcaat gattccggtt    9420
   ccgggtacac cgtgggcgtt ggtgatatcg gaccactcgg cgattcggtg acaccggtac    9480
   tggtgcttga cagtgttgcc aatatctgcg aactttctgt cctcgaacag gaagaaaccg    9540
   tgcttaagag caagttcctt gagggggagc acagtgccgg cgtaggtgaa gtcgtcaatg    9600
   atgtcgatat gggttttgat catgcacaca taaggtccga ccttatcggc aagctcaatg    9660
   agctccttgg tggtggtaac atccagagaa gcacacaggt tggttttctt ggctgccacg    9720
   agcttgagca ctcgagcggc aaaggcggac ttgtggacgt tagctcgagc ttcgtaggag      9780
   ggcattttgg tggtgaagag gagactgaaa taaatttagt ctgcagaact ttttatcgga      9840
   accttatctg gggcagtgaa gtatatgtta tggtaatagt tacgagttag ttgaacttat      9900
   agatagactg gactatacgg ctatcggtcc aaattagaaa gaacgtcaat ggctctctgg      9960
   gcgtcgcctt tgccgacaaa aatgtgatca tgatgaaagc cagcaatgac gttgcagctg     10020
   atattgttgt cggccaaccg cgccgaaaac gcagctgtca gacccacagc ctccaacgaa     10080
   gaatgtatcg tcaaagtgat ccaagcacac tcatagttgg agtcgtactc caaaggcggc     10140
   aatgacgagt cagacagata ctcgtcgacg tttaaaccat catctaaggg cctcaaaact     10200
   acctcggaac tgctgcgctg atctggacac cacagaggtt ccgagcactt taggttgcac     10260
   caaatgtccc accaggtgca ggcagaaaac gctggaacag cgtgtacagt ttgtcttaac     10320
   aaaaagtgag ggcgctgagg tcgagcaggg tggtgtgact tgttatagcc tttagagctg     10380
   cgaaagcgcg tatggatttg gctcatcagg ccagattgag ggtctgtgga cacatgtcat     10440
   gttagtgtac ttcaatcgcc ccctggatat agccccgaca ataggccgtg gcctcatttt     10500
   tttgccttcc gcacatttcc attgctcggt acccacacct tgcttctcct gcacttgcca     10560
   accttaatac tggtttacat tgaccaacat cttacaagcg gggggcttgt ctagggtata     10620
   tataaacagt ggctctccca atcggttgcc agtctctttt ttcctttctt tccccacaga     10680
   ttcgaaatct aaactacaca tcacac                                          10706
<210> 7
   <211> 47
   <212> DNA
   <213> Artificial sequence
<220>
   <223> AarI-removal 1
<400> 7
   agaagtatcc taccatctac catctccgaa agaaactcgt cgattcc 47
<210> 8
   <211> 47
   <212> DNA
   <213> Artificial sequence
<220>
   <223> AarI-removal 2
<400> 8
   ggaatcgacg agtttctttc ggagatggta gatggtagga tacttct 47
<210> 9
   <211> 10706
   <212> DNA
   <213> Artificial sequence
<220>
   <223> pRF109
<400> 9
   catggacaag aaatactcca tcggcctgga cattggaacc aactctgtcg gctgggctgt        60
   catcaccgac gagtacaagg tgccctccaa gaaattcaag gtcctcggaa acaccgatcg       120
   acactccatc aagaaaaacc tcattggtgc cctgttgttc gattctggcg agactgccga       180
   agctaccaga ctcaagcgaa ctgctcggcg acgttacacc cgacggaaga accgaatctg       240
   ctacctgcag gagatctttt ccaacgagat ggccaaggtg gacgattcgt tctttcatcg       300
   actggaggaa tccttcctcg tcgaggaaga caagaaacac gagcgtcatc ccatctttgg       360
   caacattgtg gacgaggttg cttaccacga gaagtatcct accatctacc atctccgaaa       420
   gaaactcgtc gattccaccg acaaggcgga tctcagactt atctacctcg ctctggcaca       480
   catgatcaag tttcgaggtc atttcctcat cgagggcgat ctcaatcccg acaacagcga       540
   tgtggacaag ctgttcattc agctcgttca gacctacaac cagctgttcg aggaaaaccc       600
   catcaatgcc tccggagtcg atgcaaaggc catcttgtct gctcgactct cgaagagcag       660
   acgactggag aacctcattg cccaacttcc tggcgagaaa aagaacggac tgtttggcaa       720
   cctcattgcc ctttctcttg gtctcacacc caacttcaag tccaacttcg atctggcgga       780
   ggacgccaag ctccagctgt ccaaggacac ctacgacgat gacctcgaca acctgcttgc       840
   acagattggc gatcagtacg ccgacctgtt tctcgctgcc aagaaccttt cggatgctat      900
   tctcttgtct gacattctgc gagtcaacac cgagatcaca aaggctcccc tttctgcctc      960
   catgatcaag cgatacgacg agcaccatca ggatctcaca ctgctcaagg ctcttgtccg     1020
   acagcaactg cccgagaagt acaaggagat ctttttcgat cagtcgaaga acggctacgc     1080
   tggatacatc gacggcggag cctctcagga agagttctac aagttcatca agccaattct     1140
   cgagaagatg gacggaaccg aggaactgct tgtcaagctc aatcgagagg atctgcttcg     1200
   gaagcaacga accttcgaca acggcagcat tcctcatcag atccacctcg gtgagctgca     1260
   cgccattctt cgacgtcagg aagacttcta cccctttctc aaggacaacc gagagaagat     1320
   cgagaagatt cttacctttc gaatccccta ctatgttggt cctcttgcca gaggaaactc     1380
   tcgatttgct tggatgactc gaaagtccga ggaaaccatc actccctgga acttcgagga     1440
   agtcgtggac aagggtgcct ctgcacagtc cttcatcgag cgaatgacca acttcgacaa     1500
   gaatctgccc aacgagaagg ttcttcccaa gcattcgctg ctctacgagt actttacagt     1560
   ctacaacgaa ctcaccaaag tcaagtacgt taccgaggga atgcgaaagc ctgccttctt     1620
   gtctggcgaa cagaagaaag ccattgtcga tctcctgttc aagaccaacc gaaaggtcac     1680
   tgttaagcag ctcaaggagg actacttcaa gaaaatcgag tgtttcgaca gcgtcgagat     1740
   ttccggagtt gaggaccgat tcaacgcctc tttgggcacc tatcacgatc tgctcaagat     1800
   tatcaaggac aaggattttc tcgacaacga ggaaaacgag gacattctgg aggacatcgt     1860
   gctcactctt accctgttcg aagatcggga gatgatcgag gaacgactca agacatacgc     1920
   tcacctgttc gacgacaagg tcatgaaaca actcaagcga cgtagataca ccggctgggg     1980
   aagactttcg cgaaagctca tcaacggcat cagagacaag cagtccggaa agaccattct     2040
   ggactttctc aagtccgatg gctttgccaa ccgaaacttc atgcagctca ttcacgacga     2100
   ttctcttacc ttcaaggagg acatccagaa ggcacaagtg tccggtcagg gcgacagctt     2160
   gcacgaacat attgccaacc tggctggttc gccagccatc aagaaaggca ttctccagac     2220
   tgtcaaggtt gtcgacgagc tggtgaaggt catgggacgt cacaagcccg agaacattgt     2280
   gatcgagatg gccagagaga accagacaac tcaaaagggt cagaaaaact cgcgagagcg     2340
   gatgaagcga atcgaggaag gcatcaagga gctgggatcc cagattctca aggagcatcc     2400
   cgtcgagaac actcaactgc agaacgagaa gctgtatctc tactatctgc agaatggtcg     2460
   agacatgtac gtggatcagg aactggacat caatcgtctc agcgactacg atgtggacca     2520
   cattgtccct caatcctttc tcaaggacga ttctatcgac aacaaggtcc ttacacgatc     2580
   cgacaagaac agaggcaagt cggacaacgt tcccagcgaa gaggtggtca aaaagatgaa     2640
   gaactactgg cgacagctgc tcaacgccaa gctcattacc cagcgaaagt tcgacaatct     2700
   taccaaggcc gagcgaggcg gtctgtccga gctcgacaag gctggcttca tcaagcgtca     2760
   actcgtcgag accagacaga tcacaaagca cgtcgcacag attctcgatt ctcggatgaa     2820
   caccaagtac gacgagaacg acaagctcat ccgagaggtc aaggtgatta ctctcaagtc     2880
   caaactggtc tccgatttcc gaaaggactt tcagttctac aaggtgcgag agatcaacaa     2940
   ttaccaccat gcccacgatg cttacctcaa cgccgtcgtt ggcactgcgc tcatcaagaa     3000
   ataccccaag ctcgaaagcg agttcgttta cggcgattac aaggtctacg acgttcgaaa     3060
   gatgattgcc aagtccgaac aggagattgg caaggctact gccaagtact tcttttactc     3120
   caacatcatg aactttttca agaccgagat caccttggcc aacggagaga ttcgaaagag     3180
   accacttatc gagaccaacg gcgaaactgg agagatcgtg tgggacaagg gtcgagactt     3240
   tgcaaccgtg cgaaaggttc tgtcgatgcc tcaggtcaac atcgtcaaga aaaccgaggt     3300
   tcagactggc ggattctcca aggagtcgat tctgcccaag cgaaactccg acaagctcat     3360
   cgctcgaaag aaagactggg atcccaagaa atacggtggc ttcgattctc ctaccgtcgc     3420
   ctattccgtg cttgtcgttg cgaaggtcga gaagggcaag tccaaaaagc tcaagtccgt     3480
   caaggagctg ctcggaatta ccatcatgga gcgatcgagc ttcgagaaga atcccatcga     3540
   cttcttggaa gccaagggtt acaaggaggt caagaaagac ctcattatca agctgcccaa     3600
   gtactctctg ttcgaactgg agaacggtcg aaagcgtatg ctcgcctccg ctggcgagct     3660
   gcagaaggga aacgagcttg ccttgccttc gaagtacgtc aactttctct atctggcttc     3720
   tcactacgag aagctcaagg gttctcccga ggacaacgaa cagaagcaac tcttcgttga     3780
   gcagcacaaa cattacctcg acgagattat cgagcagatt tccgagtttt cgaagcgagt     3840
   catcctggct gatgccaact tggacaaggt gctctctgcc tacaacaagc atcgggacaa     3900
   acccattcga gaacaggcgg agaacatcat tcacctgttt actcttacca acctgggtgc     3960
   tcctgcagct ttcaagtact tcgataccac tatcgaccga aagcggtaca catccaccaa     4020
   ggaggttctc gatgccaccc tgattcacca gtccatcact ggcctgtacg agacccgaat     4080
   cgacctgtct cagcttggtg gcgactccag agccgatccc aagaaaaagc gaaaggtcta     4140
   agcggccgca agtgtggatg gggaagtgag tgcccggttc tgtgtgcaca attggcaatc     4200
   caagatggat ggattcaaca cagggatata gcgagctacg tggtggtgcg aggatatagc     4260
   aacggatatt tatgtttgac acttgagaat gtacgataca agcactgtcc aagtacaata     4320
   ctaaacatac tgtacatact catactcgta cccgggcaac ggtttcactt gagtgcagtg     4380
   gctagtgctc ttactcgtac agtgtgcaat actgcgtatc atagtctttg atgtatatcg     4440
   tattcattca tgttagttgc gtacgagccg gaagcataaa gtgtaaagcc tggggtgcct     4500
   aatgagtgag ctaactcaca ttaattgcgt tgcgctcact gcccgctttc cagtcgggaa     4560
   acctgtcgtg ccagctgcat taatgaatcg gccaacgcgc ggggagaggc ggtttgcgta     4620
   ttgggcgctc ttccgcttcc tcgctcactg actcgctgcg ctcggtcgtt cggctgcggc     4680
   gagcggtatc agctcactca aaggcggtaa tacggttatc cacagaatca ggggataacg     4740
   caggaaagaa catgtgagca aaaggccagc aaaaggccag gaaccgtaaa aaggccgcgt     4800
   tgctggcgtt tttccatagg ctccgccccc ctgacgagca tcacaaaaat cgacgctcaa     4860
   gtcagaggtg gcgaaacccg acaggactat aaagatacca ggcgtttccc cctggaagct     4920
   ccctcgtgcg ctctcctgtt ccgaccctgc cgcttaccgg atacctgtcc gcctttctcc     4980
   cttcgggaag cgtggcgctt tctcatagct cacgctgtag gtatctcagt tcggtgtagg     5040
   tcgttcgctc caagctgggc tgtgtgcacg aaccccccgt tcagcccgac cgctgcgcct     5100
   tatccggtaa ctatcgtctt gagtccaacc cggtaagaca cgacttatcg ccactggcag     5160
   cagccactgg taacaggatt agcagagcga ggtatgtagg cggtgctaca gagttcttga     5220
   agtggtggcc taactacggc tacactagaa ggacagtatt tggtatctgc gctctgctga     5280
   agccagttac cttcggaaaa agagttggta gctcttgatc cggcaaacaa accaccgctg     5340
   gtagcggtgg tttttttgtt tgcaagcagc agattacgcg cagaaaaaaa ggatctcaag     5400
   aagatccttt gatcttttct acggggtctg acgctcagtg gaacgaaaac tcacgttaag     5460
   ggattttggt catgagatta tcaaaaagga tcttcaccta gatcctttta aattaaaaat     5520
   gaagttttaa atcaatctaa agtatatatg agtaaacttg gtctgacagt taccaatgct     5580
   taatcagtga ggcacctatc tcagcgatct gtctatttcg ttcatccata gttgcctgac     5640
   tccccgtcgt gtagataact acgatacggg agggcttacc atctggcccc agtgctgcaa     5700
   tgataccgcg agacccacgc tcaccggctc cagatttatc agcaataaac cagccagccg     5760
   gaagggccga gcgcagaagt ggtcctgcaa ctttatccgc ctccatccag tctattaatt     5820
   gttgccggga agctagagta agtagttcgc cagttaatag tttgcgcaac gttgttgcca     5880
   ttgctacagg catcgtggtg tcacgctcgt cgtttggtat ggcttcattc agctccggtt     5940
   cccaacgatc aaggcgagtt acatgatccc ccatgttgtg caaaaaagcg gttagctcct     6000
   tcggtcctcc gatcgttgtc agaagtaagt tggccgcagt gttatcactc atggttatgg     6060
   cagcactgca taattctctt actgtcatgc catccgtaag atgcttttct gtgactggtg     6120
   agtactcaac caagtcattc tgagaatagt gtatgcggcg accgagttgc tcttgcccgg     6180
   cgtcaatacg ggataatacc gcgccacata gcagaacttt aaaagtgctc atcattggaa     6240
   aacgttcttc ggggcgaaaa ctctcaagga tcttaccgct gttgagatcc agttcgatgt     6300
   aacccactcg tgcacccaac tgatcttcag catcttttac tttcaccagc gtttctgggt     6360
   gagcaaaaac aggaaggcaa aatgccgcaa aaaagggaat aagggcgaca cggaaatgtt     6420
   gaatactcat actcttcctt tttcaatatt attgaagcat ttatcagggt tattgtctca     6480
   tgagcggata catatttgaa tgtatttaga aaaataaaca aataggggtt ccgcgcacat     6540
   ttccccgaaa agtgccacct gacgcgccct gtagcggcgc attaagcgcg gcgggtgtgg     6600
   tggttacgcg cagcgtgacc gctacacttg ccagcgccct agcgcccgct cctttcgctt     6660
   tcttcccttc ctttctcgcc acgttcgccg gctttccccg tcaagctcta aatcgggggc     6720
   tccctttagg gttccgattt agtgctttac ggcacctcga ccccaaaaaa cttgattagg     6780
   gtgatggttc acgtagtggg ccatcgccct gatagacggt ttttcgccct ttgacgttgg     6840
   agtccacgtt ctttaatagt ggactcttgt tccaaactgg aacaacactc aaccctatct     6900
   cggtctattc ttttgattta taagggattt tgccgatttc ggcctattgg ttaaaaaatg     6960
   agctgattta acaaaaattt aacgcgaatt ttaacaaaat attaacgctt acaatttcca     7020
   ttcgccattc aggctgcgca actgttggga agggcgatcg gtgcgggcct cttcgctatt     7080
   acgccagctg gcgaaagggg gatgtgctgc aaggcgatta agttgggtaa cgccagggtt     7140
   ttcccagtca cgacgttgta aaacgacggc cagtgaattg taatacgact cactataggg     7200
   cgaattgggt accgggcccc ccctcgaggt cgatggtgtc gataagcttg atatcgaatt     7260
   catgtcacac aaaccgatct tcgcctcaag gaaacctaat tctacatccg agagactgcc     7320
   gagatccagt ctacactgat taattttcgg gccaataatt taaaaaaatc gtgttatata     7380
   atattatatg tattatatat atacatcatg atgatactga cagtcatgtc ccattgctaa     7440
   atagacagac tccatctgcc gcctccaact gatgttctca atatttaagg ggtcatctcg     7500
   cattgtttaa taataaacag actccatcta ccgcctccaa atgatgttct caaaatatat     7560
   tgtatgaact tatttttatt acttagtatt attagacaac ttacttgctt tatgaaaaac     7620
   acttcctatt taggaaacaa tttataatgg cagttcgttc atttaacaat ttatgtagaa     7680
   taaatgttat aaatgcgtat gggaaatctt aaatatggat agcataaatg atatctgcat     7740
   tgcctaattc gaaatcaaca gcaacgaaaa aaatcccttg tacaacataa atagtcatcg     7800
   agaaatatca actatcaaag aacagctatt cacacgttac tattgagatt attattggac     7860
   gagaatcaca cactcaactg tctttctctc ttctagaaat acaggtacaa gtatgtacta     7920
   ttctcattgt tcatacttct agtcatttca tcccacatat tccttggatt tctctccaat     7980
   gaatgacatt ctatcttgca aattcaacaa ttataataag atataccaaa gtagcggtat     8040
   agtggcaatc aaaaagcttc tctggtgtgc ttctcgtatt tatttttatt ctaatgatcc     8100
   attaaaggta tatatttatt tcttgttata taatcctttt gtttattaca tgggctggat     8160
   acataaaggt attttgattt aattttttgc ttaaattcaa tcccccctcg ttcagtgtca     8220
   actgtaatgg taggaaatta ccatactttt gaagaagcaa aaaaaatgaa agaaaaaaaa     8280
   aatcgtattt ccaggttaga cgttccgcag aatctagaat gcggtatgcg gtacattgtt     8340
   cttcgaacgt aaaagttgcg ctccctgaga tattgtacat ttttgctttt acaagtacaa     8400
   gtacatcgta caactatgta ctactgttga tgcatccaca acagtttgtt ttgttttttt 8460
   ttgttttttt tttttctaat gattcattac cgctatgtat acctacttgt acttgtagta 8520
   agccgggtta ttggcgttca attaatcata gacttatgaa tctgcacggt gtgcgctgcg 8580
   agttactttt agcttatgca tgctacttgg gtgtaatatt gggatctgtt cggaaatcaa 8640
   cggatgctca atcgatttcg acagtaatta attaagtcat acacaagtca gctttcttcg 8700
   agcctcatat aagtataagt agttcaacgt attagcactg tacccagcat ctccgtatcg 8760
   agaaacacaa caacatgccc cattggacag atcatgcgga tacacaggtt gtgcagtatc 8820
   atacatactc gatcagacag gtcgtctgac catcatacaa gctgaacaag cgctccatac 8880
   ttgcacgctc tctatataca cagttaaatt acatatccat agtctaacct ctaacagtta 8940
   atcttctggt aagcctccca gccagccttc tggtatcgct tggcctcctc aataggatct 9000
   cggttctggc cgtacagacc tcggccgaca attatgatat ccgttccggt agacatgaca 9060
   tcctcaacag ttcggtactg ctgtccgaga gcgtctccct tgtcgtcaag acccaccccg 9120
   ggggtcagaa taagccagtc ctcagagtcg cccttaggtc ggttctgggc aatgaagcca 9180
   accacaaact cggggtcgga tcgggcaagc tcaatggtct gcttggagta ctcgccagtg 9240
   gccagagagc ccttgcaaga cagctcggcc agcatgagca gacctctggc cagcttctcg 9300
   ttgggagagg ggactaggaa ctccttgtac tgggagttct cgtagtcaga gacgtcctcc 9360
   ttcttctgtt cagagacagt ttcctcggca ccagctcgca ggccagcaat gattccggtt 9420
   ccgggtacac cgtgggcgtt ggtgatatcg gaccactcgg cgattcggtg acaccggtac 9480
   tggtgcttga cagtgttgcc aatatctgcg aactttctgt cctcgaacag gaagaaaccg 9540
   tgcttaagag caagttcctt gagggggagc acagtgccgg cgtaggtgaa gtcgtcaatg 9600
   atgtcgatat gggttttgat catgcacaca taaggtccga ccttatcggc aagctcaatg 9660
   agctccttgg tggtggtaac atccagagaa gcacacaggt tggttttctt ggctgccacg 9720
   agcttgagca ctcgagcggc aaaggcggac ttgtggacgt tagctcgagc ttcgtaggag 9780
   ggcattttgg tggtgaagag gagactgaaa taaatttagt ctgcagaact ttttatcgga 9840
   accttatctg gggcagtgaa gtatatgtta tggtaatagt tacgagttag ttgaacttat 9900
   agatagactg gactatacgg ctatcggtcc aaattagaaa gaacgtcaat ggctctctgg 9960
   gcgtcgcctt tgccgacaaa aatgtgatca tgatgaaagc cagcaatgac gttgcagctg 10020
   atattgttgt cggccaaccg cgccgaaaac gcagctgtca gacccacagc ctccaacgaa 10080
   gaatgtatcg tcaaagtgat ccaagcacac tcatagttgg agtcgtactc caaaggcggc 10140
   aatgacgagt cagacagata ctcgtcgacg tttaaaccat catctaaggg cctcaaaact 10200
   acctcggaac tgctgcgctg atctggacac cacagaggtt ccgagcactt taggttgcac 10260
   caaatgtccc accaggtgca ggcagaaaac gctggaacag cgtgtacagt ttgtcttaac 10320
   aaaaagtgag ggcgctgagg tcgagcaggg tggtgtgact tgttatagcc tttagagctg 10380
   cgaaagcgcg tatggatttg gctcatcagg ccagattgag ggtctgtgga cacatgtcat 10440
   gttagtgtac ttcaatcgcc ccctggatat agccccgaca ataggccgtg gcctcatttt 10500
   tttgccttcc gcacatttcc attgctcggt acccacacct tgcttctcct gcacttgcca 10560
   accttaatac tggtttacat tgaccaacat cttacaagcg gggggcttgt ctagggtata 10620
   tataaacagt ggctctccca atcggttgcc agtctctttt ttcctttctt tccccacaga 10680
   ttcgaaatct aaactacaca tcacac 10706
<210> 10
   <211> 4140
   <212> DNA
   <213> Artificial sequence
<220>
   <223> Aar1- Cas9 ORF
<400> 10
   atggacaaga aatactccat cggcctggac attggaacca actctgtcgg ctgggctgtc      60
   atcaccgacg agtacaaggt gccctccaag aaattcaagg tcctcggaaa caccgatcga     120
   cactccatca agaaaaacct cattggtgcc ctgttgttcg attctggcga gactgccgaa     180
   gctaccagac tcaagcgaac tgctcggcga cgttacaccc gacggaagaa ccgaatctgc     240
   tacctgcagg agatcttttc caacgagatg gccaaggtgg acgattcgtt ctttcatcga     300
   ctggaggaat ccttcctcgt cgaggaagac aagaaacacg agcgtcatcc catctttggc     360
   aacattgtgg acgaggttgc ttaccacgag aagtatccta ccatctacca tctccgaaag     420
   aaactcgtcg attccaccga caaggcggat ctcagactta tctacctcgc tctggcacac     480
   atgatcaagt ttcgaggtca tttcctcatc gagggcgatc tcaatcccga caacagcgat     540
   gtggacaagc tgttcattca gctcgttcag acctacaacc agctgttcga ggaaaacccc     600
   atcaatgcct ccggagtcga tgcaaaggcc atcttgtctg ctcgactctc gaagagcaga     660
   cgactggaga acctcattgc ccaacttcct ggcgagaaaa agaacggact gtttggcaac     720
   ctcattgccc tttctcttgg tctcacaccc aacttcaagt ccaacttcga tctggcggag     780
   gacgccaagc tccagctgtc caaggacacc tacgacgatg acctcgacaa cctgcttgca     840
   cagattggcg atcagtacgc cgacctgttt ctcgctgcca agaacctttc ggatgctatt 900
   ctcttgtctg acattctgcg agtcaacacc gagatcacaa aggctcccct ttctgcctcc 960
   atgatcaagc gatacgacga gcaccatcag gatctcacac tgctcaaggc tcttgtccga 1020
   cagcaactgc ccgagaagta caaggagatc tttttcgatc agtcgaagaa cggctacgct 1080
   ggatacatcg acggcggagc ctctcaggaa gagttctaca agttcatcaa gccaattctc 1140
   gagaagatgg acggaaccga ggaactgctt gtcaagctca atcgagagga tctgcttcgg 1200
   aagcaacgaa ccttcgacaa cggcagcatt cctcatcaga tccacctcgg tgagctgcac 1260
   gccattcttc gacgtcagga agacttctac ccctttctca aggacaaccg agagaagatc 1320
   gagaagattc ttacctttcg aatcccctac tatgttggtc ctcttgccag aggaaactct 1380
   cgatttgctt ggatgactcg aaagtccgag gaaaccatca ctccctggaa cttcgaggaa 1440
   gtcgtggaca agggtgcctc tgcacagtcc ttcatcgagc gaatgaccaa cttcgacaag 1500
   aatctgccca acgagaaggt tcttcccaag cattcgctgc tctacgagta ctttacagtc 1560
   tacaacgaac tcaccaaagt caagtacgtt accgagggaa tgcgaaagcc tgccttcttg 1620
   tctggcgaac agaagaaagc cattgtcgat ctcctgttca agaccaaccg aaaggtcact 1680
   gttaagcagc tcaaggagga ctacttcaag aaaatcgagt gtttcgacag cgtcgagatt 1740
   tccggagttg aggaccgatt caacgcctct ttgggcacct atcacgatct gctcaagatt 1800
   atcaaggaca aggatttctt cgacaacgag gaaaacgagg acattctgga ggacatcgtg 1860
   ctcactctta ccctgttcga agatcgggag atgatcgagg aacgactcaa gacatacgct 1920
   cacctgttcg acgacaaggt catgaaacaa ctcaagcgac gtagatacac cggctgggga 1980
   agactttcgc gaaagctcat caacggcatc agagacaagc agtccggaaa gaccattctg 2040
   gactttctca agtccgatgg ctttgccaac cgaaacttca tgcagctcat tcacgacgat 2100
   tctcttacct tcaaggagga catccagaag gcacaagtgt ccggtcaggg cgacagcttg 2160
   cacgaacata ttgccaacct ggctggttcg ccagccatca agaaaggcat tctccagact 2220
   gtcaaggttg tcgacgagct ggtgaaggtc atgggacgtc acaagcccga gaacattgtg 2280
   atcgagatgg ccagagagaa ccagacaact caaaagggtc agaaaaactc gcgagagcgg 2340
   atgaagcgaa tcgaggaagg catcaaggag ctgggatccc agattctcaa ggagcatccc 2400
   gtcgagaaca ctcaactgca gaacgagaag ctgtatctct actatctgca gaatggtcga 2460
   gacatgtacg tggatcagga actggacatc aatcgtctca gcgactacga tgtggaccac 2520
   attgtccctc aatcctttct caaggacgat tctatcgaca acaaggtcct tacacgatcc 2580
   gacaagaaca gaggcaagtc ggacaacgtt cccagcgaag aggtggtcaa aaagatgaag 2640
   aactactggc gacagctgct caacgccaag ctcattaccc agcgaaagtt cgacaatctt 2700
   accaaggccg agcgaggcgg tctgtccgag ctcgacaagg ctggcttcat caagcgtcaa 2760
   ctcgtcgaga ccagacagat cacaaagcac gtcgcacaga ttctcgattc tcggatgaac 2820
   accaagtacg acgagaacga caagctcatc cgagaggtca aggtgattac tctcaagtcc 2880
   aaactggtct ccgatttccg aaaggacttt cagttctaca aggtgcgaga gatcaacaat 2940
   taccaccatg cccacgatgc ttacctcaac gccgtcgttg gcactgcgct catcaagaaa 3000
   taccccaagc tcgaaagcga gttcgtttac ggcgattaca aggtctacga cgttcgaaag 3060
   atgattgcca agtccgaaca ggagattggc aaggctactg ccaagtactt cttttactcc 3120
   aacatcatga actttttcaa gaccgagatc accttggcca acggagagat tcgaaagaga 3180
   ccacttatcg agaccaacgg cgaaactgga gagatcgtgt gggacaaggg tcgagacttt 3240
   gcaaccgtgc gaaaggttct gtcgatgcct caggtcaaca tcgtcaagaa aaccgaggtt 3300
   cagactggcg gattctccaa ggagtcgatt ctgcccaagc gaaactccga caagctcatc 3360
   gctcgaaaga aagactggga tcccaagaaa tacggtggct tcgattctcc taccgtcgcc 3420
   tattccgtgc ttgtcgttgc gaaggtcgag aagggcaagt ccaaaaagct caagtccgtc 3480
   aaggagctgc tcggaattac catcatggag cgatcgagct tcgagaagaa tcccatcgac 3540
   ttcttggaag ccaagggtta caaggaggtc aagaaagacc tcattatcaa gctgcccaag 3600
   tactctctgt tcgaactgga gaacggtcga aagcgtatgc tcgcctccgc tggcgagctg 3660
   cagaagggaa acgagcttgc cttgccttcg aagtacgtca actttctcta tctggcttct 3720
   cactacgaga agctcaaggg ttctcccgag gacaacgaac agaagcaact cttcgttgag 3780
   cagcacaaac attacctcga cgagattatc gagcagattt ccgagttttc gaagcgagtc 3840
   atcctggctg atgccaactt ggacaaggtg ctctctgcct acaacaagca tcgggacaaa 3900
   cccattcgag aacaggcgga gaacatcatt cacctgttta ctcttaccaa cctgggtgct 3960
   cctgcagctt tcaagtactt cgataccact atcgaccgaa agcggtacac atccaccaag 4020
   gaggttctcg atgccaccct gattcaccag tccatcactg gcctgtacga gacccgaatc 4080
   gacctgtctc agcttggtgg cgactccaga gccgatccca agaaaaagcg aaaggtctaa 4140
<210> 11
   <211> 10706
   <212> DNA
   <213> Artificial sequence
<220>
   <223> pRF141
<400> 11
   catggacaag aaatactcca tcggcctgga cattggaacc aactctgtcg gctgggctgt     60
   catcaccgac gagtacaagg tgccctccaa gaaattcaag gtcctcggaa acaccgatcg    120
   acactccatc aagaaaaacc tcattggtgc cctgttgttc gattctggcg agactgccga    180
   agctaccaga ctcaagcgaa ctgctcggcg acgttacacc cgacggaaga accgaatctg    240
   ctacctgcag gagatctttt ccaacgagat ggccaaggtg gacgattcgt tctttcatcg    300
   actggaggaa tccttcctcg tcgaggaaga caagaaacac gagcgtcatc ccatctttgg    360
   caacattgtg gacgaggttg cttaccacga gaagtatcct accatctacc atctccgaaa    420
   gaaactcgtc gattccaccg acaaggcgga tctcagactt atctacctcg ctctggcaca    480
   catgatcaag tttcgaggtc atttcctcat cgagggcgat ctcaatcccg acaacagcga    540
   tgtggacaag ctgttcattc agctcgttca gacctacaac cagctgttcg aggaaaaccc    600
   catcaatgcc tccggagtcg atgcaaaggc catcttgtct gctcgactct cgaagagcag    660
   acgactggag aacctcattg cccaacttcc tggcgagaaa aagaacggac tgtttggcaa    720
   cctcattgcc ctttctcttg gtctcacacc caacttcaag tccaacttcg atctggcgga    780
   ggacgccaag ctccagctgt ccaaggacac ctacgacgat gacctcgaca acctgcttgc    840
   acagattggc gatcagtacg ccgacctgtt tctcgctgcc aagaaccttt cggatgctat    900
   tctcttgtct gacattctgc gagtcaacac cgagatcaca aaggctcccc tttctgcctc    960
   catgatcaag cgatacgacg agcaccatca ggatctcaca ctgctcaagg ctcttgtccg   1020
   acagcaactg cccgagaagt acaaggagat ctttttcgat cagtcgaaga acggctacgc   1080
   tggatacatc gacggcggag cctctcagga agagttctac aagttcatca agccaattct   1140
   cgagaagatg gacggaaccg aggaactgct tgtcaagctc aatcgagagg atctgcttcg   1200
   gaagcaacga accttcgaca acggcagcat tcctcatcag atccacctcg gtgagctgca   1260
   cgccattctt cgacgtcagg aagacttcta cccctttctc aaggacaacc gagagaagat   1320
   cgagaagatt cttacctttc gaatccccta ctatgttggt cctcttgcca gaggaaactc   1380
   tcgatttgct tggatgactc gaaagtccga ggaaaccatc actccctgga acttcgagga   1440
   agtcgtggac aagggtgcct ctgcacagtc cttcatcgag cgaatgacca acttcgacaa   1500
   gaatctgccc aacgagaagg ttcttcccaa gcattcgctg ctctacgagt actttacagt   1560
   ctacaacgaa ctcaccaaag tcaagtacgt taccgaggga atgcgaaagc ctgccttctt   1620
   gtctggcgaa cagaagaaag ccattgtcga tctcctgttc aagaccaacc gaaaggtcac   1680
   tgttaagcag ctcaaggagg actacttcaa gaaaatcgag tgtttcgaca gcgtcgagat   1740
   ttccggagtt gaggaccgat tcaacgcctc tttgggcacc tatcacgatc tgctcaagat   1800
   tatcaaggac aaggattttc tcgacaacga ggaaaacgag gacattctgg aggacatcgt   1860
   gctcactctt accctgttcg aagatcggga gatgatcgag gaacgactca agacatacgc   1920
   tcacctgttc gacgacaagg tcatgaaaca actcaagcga cgtagataca ccggctgggg   1980
   aagactttcg cgaaagctca tcaacggcat cagagacaag cagtccggaa agaccattct   2040
   ggactttctc aagtccgatg gctttgccaa ccgaaacttc atgcagctca ttcacgacga   2100
   ttctcttacc ttcaaggagg acatccagaa ggcacaagtg tccggtcagg gcgacagctt   2160
   gcacgaacat attgccaacc tggctggttc gccagccatc aagaaaggca ttctccagac   2220
   tgtcaaggtt gtcgacgagc tggtgaaggt catgggacgt cacaagcccg agaacattgt   2280
   gatcgagatg gccagagaga accagacaac tcaaaagggt cagaaaaact cgcgagagcg   2340
   gatgaagcga atcgaggaag gcatcaagga gctgggatcc cagattctca aggagcatcc   2400
   cgtcgagaac actcaactgc agaacgagaa gctgtatctc tactatctgc agaatggtcg   2460
   agacatgtac gtggatcagg aactggacat caatcgtctc agcgactacg atgtggacca   2520
   cattgtccct caatcctttc tcaaggacga ttctatcgac aacaaggtcc ttacacgatc   2580
   cgacaagaac agaggcaagt cggacaacgt tcccagcgaa gaggtggtca aaaagatgaa   2640
   gaactactgg cgacagctgc tcaacgccaa gctcattacc cagcgaaagt tcgacaatct   2700
   taccaaggcc gagcgaggcg gtctgtccga gctcgacaag gctggcttca tcaagcgtca   2760
   actcgtcgag accagacaga tcacaaagca cgtcgcacag attctcgatt ctcggatgaa   2820
   caccaagtac gacgagaacg acaagctcat ccgagaggtc aaggtgatta ctctcaagtc   2880
   caaactggtc tccgatttcc gaaaggactt tcagttctac aaggtgcgag agatcaacaa   2940
   ttaccaccat gcccacgatg cttacctcaa cgccgtcgtt ggcactgcgc tcatcaagaa   3000
   ataccccaag ctcgaaagcg agttcgttta cggcgattac aaggtctacg acgttcgaaa   3060
   gatgattgcc aagtccgaac aggagattgg caaggctact gccaagtact tcttttactc   3120
   caacatcatg aactttttca agaccgagat caccttggcc aacggagaga ttcgaaagag   3180
   accacttatc gagaccaacg gcgaaactgg agagatcgtg tgggacaagg gtcgagactt   3240
   tgcaaccgtg cgaaaggttc tgtcgatgcc tcaggtcaac atcgtcaaga aaaccgaggt   3300
   tcagactggc ggattctcca aggagtcgat tctgcccaag cgaaactccg acaagctcat   3360
   cgctcgaaag aaagactggg atcccaagaa atacggtggc ttcgattctc ctaccgtcgc   3420
   ctattccgtg cttgtcgttg cgaaggtcga gaagggcaag tccaaaaagc tcaagtccgt   3480
   caaggagctg ctcggaatta ccatcatgga gcgatcgagc ttcgagaaga atcccatcga   3540
   cttcttggaa gccaagggtt acaaggaggt caagaaagac ctcattatca agctgcccaa   3600
   gtactctctg ttcgaactgg agaacggtcg aaagcgtatg ctcgcctccg ctggcgagct   3660
   gcagaaggga aacgagcttg ccttgccttc gaagtacgtc aactttctct atctggcttc 3720
   tcactacgag aagctcaagg gttctcccga ggacaacgaa cagaagcaac tcttcgttga 3780
   gcagcacaaa cattacctcg acgagattat cgagcagatt tccgagtttt cgaagcgagt 3840
   catcctggct gatgccaact tggacaaggt gctctctgcc tacaacaagc atcgggacaa 3900
   acccattcga gaacaggcgg agaacatcat tcacctgttt actcttacca acctgggtgc 3960
   tcctgcagct ttcaagtact tcgataccac tatcgaccga aagcggtaca catccaccaa 4020
   ggaggttctc gatgccaccc tgattcacca gtccatcact ggcctgtacg agacccgaat 4080
   cgacctgtct cagcttggtg gcgactccag agccgatccc aagaaaaagc gaaaggtcta 4140
   agcggccgca agtgtggatg gggaagtgag tgcccggttc tgtgtgcaca attggcaatc 4200
   caagatggat ggattcaaca cagggatata gcgagctacg tggtggtgcg aggatatagc 4260
   aacggatatt tatgtttgac acttgagaat gtacgataca agcactgtcc aagtacaata 4320
   ctaaacatac tgtacatact catactcgta cccgggcaac ggtttcactt gagtgcagtg 4380
   gctagtgctc ttactcgtac agtgtgcaat actgcgtatc atagtctttg atgtatatcg 4440
   tattcattca tgttagttgc gtacgagccg gaagcataaa gtgtaaagcc tggggtgcct 4500
   aatgagtgag ctaactcaca ttaattgcgt tgcgctcact gcccgctttc cagtcgggaa 4560
   acctgtcgtg ccagctgcat taatgaatcg gccaacgcgc ggggagaggc ggtttgcgta 4620
   ttgggcgctc ttccgcttcc tcgctcactg actcgctgcg ctcggtcgtt cggctgcggc 4680
   gagcggtatc agctcactca aaggcggtaa tacggttatc cacagaatca ggggataacg 4740
   caggaaagaa catgtgagca aaaggccagc aaaaggccag gaaccgtaaa aaggccgcgt 4800
   tgctggcgtt tttccatagg ctccgccccc ctgacgagca tcacaaaaat cgacgctcaa 4860
   gtcagaggtg gcgaaacccg acaggactat aaagatacca ggcgtttccc cctggaagct 4920
   ccctcgtgcg ctctcctgtt ccgaccctgc cgcttaccgg atacctgtcc gcctttctcc 4980
   cttcgggaag cgtggcgctt tctcatagct cacgctgtag gtatctcagt tcggtgtagg 5040
   tcgttcgctc caagctgggc tgtgtgcacg aaccccccgt tcagcccgac cgctgcgcct 5100
   tatccggtaa ctatcgtctt gagtccaacc cggtaagaca cgacttatcg ccactggcag 5160
   cagccactgg taacaggatt agcagagcga ggtatgtagg cggtgctaca gagttcttga 5220
   agtggtggcc taactacggc tacactagaa ggacagtatt tggtatctgc gctctgctga 5280
   agccagttac cttcggaaaa agagttggta gctcttgatc cggcaaacaa accaccgctg 5340
   gtagcggtgg tttttttgtt tgcaagcagc agattacgcg cagaaaaaaa ggatctcaag 5400
   aagatccttt gatcttttct acggggtctg acgctcagtg gaacgaaaac tcacgttaag 5460
   ggattttggt catgagatta tcaaaaagga tcttcaccta gatcctttta aattaaaaat 5520
   gaagttttaa atcaatctaa agtatatatg agtaaacttg gtctgacagt taccaatgct 5580
   taatcagtga ggcacctatc tcagcgatct gtctatttcg ttcatccata gttgcctgac 5640
   tccccgtcgt gtagataact acgatacggg agggcttacc atctggcccc agtgctgcaa 5700
   tgataccgcg agacccacgc tcaccggctc cagatttatc agcaataaac cagccagccg 5760
   gaagggccga gcgcagaagt ggtcctgcaa ctttatccgc ctccatccag tctattaatt 5820
   gttgccggga agctagagta agtagttcgc cagttaatag tttgcgcaac gttgttgcca 5880
   ttgctacagg catcgtggtg tcacgctcgt cgtttggtat ggcttcattc agctccggtt 5940
   cccaacgatc aaggcgagtt acatgatccc ccatgttgtg caaaaaagcg gttagctcct 6000
   tcggtcctcc gatcgttgtc agaagtaagt tggccgcagt gttatcactc atggttatgg 6060
   cagcactgca taattctctt actgtcatgc catccgtaag atgcttttct gtgactggtg 6120
   agtactcaac caagtcattc tgagaatagt gtatgcggcg accgagttgc tcttgcccgg 6180
   cgtcaatacg ggataatacc gcgccacata gcagaacttt aaaagtgctc atcattggaa 6240
   aacgttcttc ggggcgaaaa ctctcaagga tcttaccgct gttgagatcc agttcgatgt 6300
   aacccactcg tgcacccaac tgatcttcag catcttttac tttcaccagc gtttctgggt 6360
   gagcaaaaac aggaaggcaa aatgccgcaa aaaagggaat aagggcgaca cggaaatgtt 6420
   gaatactcat actcttcctt tttcaatatt attgaagcat ttatcagggt tattgtctca 6480
   tgagcggata catatttgaa tgtatttaga aaaataaaca aataggggtt ccgcgcacat 6540
   ttccccgaaa agtgccacct gacgcgccct gtagcggcgc attaagcgcg gcgggtgtgg 6600
   tggttacgcg cagcgtgacc gctacacttg ccagcgccct agcgcccgct cctttcgctt 6660
   tcttcccttc ctttctcgcc acgttcgccg gctttccccg tcaagctcta aatcgggggc 6720
   tccctttagg gttccgattt agtgctttac ggcacctcga ccccaaaaaa cttgattagg 6780
   gtgatggttc acgtagtggg ccatcgccct gatagacggt ttttcgccct ttgacgttgg 6840
   agtccacgtt ctttaatagt ggactcttgt tccaaactgg aacaacactc aaccctatct 6900
   cggtctattc ttttgattta taagggattt tgccgatttc ggcctattgg ttaaaaaatg 6960
   agctgattta acaaaaattt aacgcgaatt ttaacaaaat attaacgctt acaatttcca 7020
   ttcgccattc aggctgcgca actgttggga agggcgatcg gtgcgggcct cttcgctatt 7080
   acgccagctg gcgaaagggg gatgtgctgc aaggcgatta agttgggtaa cgccagggtt 7140
   ttcccagtca cgacgttgta aaacgacggc cagtgaattg taatacgact cactataggg 7200
   cgaattgggt accgggcccc ccctcgaggt cgatggtgtc gataagcttg atatcgaatt 7260
   catgtcacac aaaccgatct tcgcctcaag gaaacctaat tctacatccg agagactgcc 7320
   gagatccagt ctacactgat taattttcgg gccaataatt taaaaaaatc gtgttatata 7380
   atattatatg tattatatat atacatcatg atgatactga cagtcatgtc ccattgctaa 7440
   atagacagac tccatctgcc gcctccaact gatgttctca atatttaagg ggtcatctcg     7500
   cattgtttaa taataaacag actccatcta ccgcctccaa atgatgttct caaaatatat     7560
   tgtatgaact tatttttatt acttagtatt attagacaac ttacttgctt tatgaaaaac     7620
   acttcctatt taggaaacaa tttataatgg cagttcgttc atttaacaat ttatgtagaa     7680
   taaatgttat aaatgcgtat gggaaatctt aaatatggat agcataaatg atatctgcat     7740
   tgcctaattc gaaatcaaca gcaacgaaaa aaatcccttg tacaacataa atagtcatcg     7800
   agaaatatca actatcaaag aacagctatt cacacgttac tattgagatt attattggac     7860
   gagaatcaca cactcaactg tctttctctc ttctagaaat acaggtacaa gtatgtacta     7920
   ttctcattgt tcatacttct agtcatttca tcccacatat tccttggatt tctctccaat     7980
   gaatgacatt ctatcttgca aattcaacaa ttataataag atataccaaa gtagcggtat     8040
   agtggcaatc aaaaagcttc tctggtgtgc ttctcgtatt tatttttatt ctaatgatcc     8100
   attaaaggta tatatttatt tcttgttata taatcctttt gtttattaca tgggctggat     8160
   acataaaggt attttgattt aattttttgc ttaaattcaa tcccccctcg ttcagtgtca     8220
   actgtaatgg taggaaatta ccatactttt gaagaagcaa aaaaaatgaa agaaaaaaaa     8280
   aatcgtattt ccaggttaga cgttccgcag aatctagaat gcggtatgcg gtacattgtt     8340
   cttcgaacgt aaaagttgcg ctccctgaga tattgtacat ttttgctttt acaagtacaa     8400
   gtacatcgta caactatgta ctactgttga tgcatccaca acagtttgtt ttgttttttt     8460
   ttgttttttt tttttctaat gattcattac cgctatgtat acctacttgt acttgtagta     8520
   agccgggtta ttggcgttca attaatcata gacttatgaa tctgcacggt gtgcgctgcg     8580
   agttactttt agcttatgca tgctacttgg gtgtaatatt gggatctgtt cggaaatcaa     8640
   cggatgctca atcgatttcg acagtaatta attaagtcat acacaagtca gctttcttcg     8700
   agcctcatat aagtataagt agttcaacgt attagcactg tacccagcat ctccgtatcg     8760
   agaaacacaa caacatgccc cattggacag atcatgcgga tacacaggtt gtgcagtatc     8820
   atacatactc gatcagacag gtcgtctgac catcatacaa gctgaacaag cgctccatac     8880
   ttgcacgctc tctatataca cagttaaatt acatatccat agtctaacct ctaacagtta     8940
   atcttctggt aagcctccca gccagccttc tggtatcgct tggcctcctc aataggatct     9000
   cggttctggc cgtacagacc tcggccgaca attatgatat ccgttccggt agacatgaca     9060
   tcctcaacag ttcggtactg ctgtccgaga gcgtctccct tgtcgtcaag acccaccccg     9120
   ggggtcagaa taagccagtc ctcagagtcg cccttaggtc ggttctgggc aatgaagcca     9180
   accacaaact cggggtcgga tcgggcaagc tcaatggtct gcttggagta ctcgccagtg     9240
   gccagagagc ccttgcaaga cagctcggcc agcatgagca gacctctggc cagcttctcg     9300
   ttgggagagg ggactaggaa ctccttgtac tgggagttct cgtagtcaga gacgtcctcc     9360
   ttcttctgtt cagagacagt ttcctcggca ccagctcgca ggccagcaat gattccggtt     9420
   ccgggtacac cgtgggcgtt ggtgatatcg gaccactcgg cgattcggtg acaccggtac     9480
   tggtgcttga cagtgttgcc aatatctgcg aactttctgt cctcgaacag gaagaaaccg     9540
   tgcttaagag caagttcctt gagggggagc acagtgccgg cgtaggtgaa gtcgtcaatg     9600
   atgtcgatat gggttttgat catgcacaca taaggtccga ccttatcggc aagctcaatg     9660
   agctccttgg tggtggtaac atccagagaa gcacacaggt tggttttctt ggctgccacg     9720
   agcttgagca ctcgagcggc aaaggcggac ttgtggacgt tagctcgagc ttcgtaggag     9780
   ggcattttgg tggtgaagag gagactgaaa taaatttagt ctgcagaact ttttatcgga     9840
   accttatctg gggcagtgaa gtatatgtta tggtaatagt tacgagttag ttgaacttat     9900
   agatagactg gactatacgg ctatcggtcc aaattagaaa gaacgtcaat ggctctctgg     9960
   gcgtcgcctt tgccgacaaa aatgtgatca tgatgaaagc cagcaatgac gttgcagctg    10020
   atattgttgt cggccaaccg cgccgaaaac gcagctgtca gacccacagc ctccaacgaa    10080
   gaatgtatcg tcaaagtgat ccaagcacac tcatagttgg agtcgtactc caaaggcggc    10140
   aatgacgagt cagacagata ctcgtcgacg tttaaaccat catctaaggg cctcaaaact    10200
   acctcggaac tgctgcgctg atctggacac cacagaggtt ccgagcactt taggttgcac    10260
   caaatgtccc accaggtgca ggcagaaaac gctggaacag cgtgtacagt ttgtcttaac    10320
   aaaaagtgag ggcgctgagg tcgagcaggg tggtgtgact tgttatagcc tttagagctg    10380
   cgaaagcgcg tatggatttg gctcatcagg ccagattgag ggtctgtgga cacatgtcat    10440
   gttagtgtac ttcaatcgcc ccctggatat agccccgaca ataggccgtg gcctcatttt    10500
   tttgccttcc gcacatttcc attgctcggt acccacacct tgcttctcct gcacttgcca    10560
   accttaatac tggtttacat tgaccaacat cttacaagcg gggggcttgt ctagggtata    10620
   tataaacagt ggctctccca atcggttgcc agtctctttt ttcctttctt tccccacaga    10680
   ttcgaaatct aaactacaca tcacac                                         10706
<210> 12
   <211> 1048
   <212> DNA
   <213> Artificial sequence
<220>
   <223> high-throughput cloning cassette
<400> 12
   gcgcacgtta attaaatttt ttttgatttt cttttttgac cccgtcttca attacacttc      60
   ccaactggga acacccctct ttatcgaccc attttaggta atttacccta gcccattgtc     120
   tccataagga atattaccct aacccacagt ccagggtgcc caggtccttc tttggccaaa     180
   ttttaacttc ggtcctatgg cacagcggta gcgcgtgaga ttgcaaatct taaggtcccg     240
   agttcgaatc tcggtgggac ctagttattt ttgatagata atttcgtgat gattagaaac     300
   ttaacgcaaa ataatggccg gcatggtccc agcctcctcg ctggcgccgg ctgggcaaca     360
   tgcttcggca tggcgaatgg gacgcaggtg atggcgggat cgttgtatat ttcttgacac     420
   cttttcggca tcgccctaaa ttcggcgtcc tcatattgtg tgaggacgtt ttattacgtg     480
   tttacgaagc aaaagctaaa accaggagct atttaatggc aacagttaac cagctggtac     540
   gcaaaccacg tgctcgcaaa gttgcgaaaa gcaacgtgcc tgcgctggaa gcatgcccgc     600
   aaaaacgtgg cgtatgtact cgtgtatata ctaccactcc taaaaaaccg aactccgcgc     660
   tgcgtaaagt atgccgtgtt cgtctgacta acggtttcga agtgacttcc tacatcggtg     720
   gtgaaggtca caacctgcag gagcactccg tgatcctgat ccgtggcggt cgtgttaaag     780
   acctcccggg tgttcgttac cacaccgtac gtggtgcgct tgactgctcc ggcgttaaag     840
   accgtaagca ggctcgttcc aagtatggcg tgaagcgtcc taaggcttag gttaataaca     900
   ggcctgctgg taatcgcagg cctttttatt tttacacctg cgttttagag ctagaaatag     960
   caagttaaaa taaggctagt ccgttatcaa cttgaaaaag tggcaccgag tcggtgcttt    1020
   tttttttgtt ttttatcgat gcgcgcac                                        1048
<210> 13
   <211> 300
   <212> DNA
   <213> Yarrowia lipolytica
<400> 13
   attttttttg attttctttt ttgaccccgt cttcaattac acttcccaac tgggaacacc      60
   cctctttatc gacccatttt aggtaattta ccctagccca ttgtctccat aaggaatatt     120
   accctaaccc acagtccagg gtgcccaggt ccttctttgg ccaaatttta acttcggtcc     180
   tatggcacag cggtagcgcg tgagattgca aatcttaagg tcccgagttc gaatctcggt     240
   gggacctagt tatttttgat agataatttc gtgatgatta gaaacttaac gcaaaataat     300
<210> 14
   <211> 68
   <212> DNA
   <213> Herpes Delta virus
<400> 14
   ggccggcatg gtcccagcct cctcgctggc gccggctggg caacatgctt cggcatggcg      60
   aatgggac                                                                68
<210> 15
   <211> 544
   <212> DNA
   <213> Escherischia coli
<400> 15
   atggcgggat cgttgtatat ttcttgacac cttttcggca tcgccctaaa ttcggcgtcc 60
   tcatattgtg tgaggacgtt ttattacgtg tttacgaagc aaaagctaaa accaggagct 120
   atttaatggc aacagttaac cagctggtac gcaaaccacg tgctcgcaaa gttgcgaaaa 180
   gcaacgtgcc tgcgctggaa gcatgcccgc aaaaacgtgg cgtatgtact cgtgtatata 240
   ctaccactcc taaaaaaccg aactccgcgc tgcgtaaagt atgccgtgtt cgtctgacta 300
   acggtttcga agtgacttcc tacatcggtg gtgaaggtca caacctgcag gagcactccg 360
   tgatcctgat ccgtggcggt cgtgttaaag acctcccggg tgttcgttac cacaccgtac 420
   gtggtgcgct tgactgctcc ggcgttaaag accgtaagca ggctcgttcc aagtatggcg 480
   tgaagcgtcc taaggcttag gttaataaca ggcctgctgg taatcgcagg cctttttatt 540
   ttta 544
<210> 16
   <211> 80
   <212> DNA
   <213> Artificial sequence
<220>
   <223> DNA encoding Cas9 CER domain
<400> 16
   gttttagagc tagaaatagc aagttaaaat aaggctagtc cgttatcaac ttgaaaaagt 60
   ggcaccgagt cggtgctttt 80
<210> 17
   <211> 14
   <212> DNA
   <213> Saccharomyces cerevisiae
<400> 17
   tttttttgtt tttt 14
<210> 18
   <211> 11714
   <212> DNA
   <213> Artificial sequence
<220>
   <223> pRF291
<400> 18
   cgataaaaaa caaaaaaaaa agcaccgact cggtgccact ttttcaagtt gataacggac      60
   tagccttatt ttaacttgct atttctagct ctaaaacgca ggtgtaaaaa taaaaaggcc     120
   tgcgattacc agcaggcctg ttattaacct aagccttagg acgcttcacg ccatacttgg     180
   aacgagcctg cttacggtct ttaacgccgg agcagtcaag cgcaccacgt acggtgtggt     240
   aacgaacacc cgggaggtct ttaacacgac cgccacggat caggatcacg gagtgctcct     300
   gcaggttgtg accttcacca ccgatgtagg aagtcacttc gaaaccgtta gtcagacgaa     360
   cacggcatac tttacgcagc gcggagttcg gttttttagg agtggtagta tatacacgag     420
   tacatacgcc acgtttttgc gggcatgctt ccagcgcagg cacgttgctt ttcgcaactt     480
   tgcgagcacg tggtttgcgt accagctggt taactgttgc cattaaatag ctcctggttt     540
   tagcttttgc ttcgtaaaca cgtaataaaa cgtcctcaca caatatgagg acgccgaatt     600
   tagggcgatg ccgaaaaggt gtcaagaaat atacaacgat cccgccatca cctgcgtccc     660
   attcgccatg ccgaagcatg ttgcccagcc ggcgccagcg aggaggctgg gaccatgccg     720
   gccattattt tgcgttaagt ttctaatcat cacgaaatta tctatcaaaa ataactaggt     780
   cccaccgaga ttcgaactcg ggaccttaag atttgcaatc tcacgcgcta ccgctgtgcc     840
   ataggaccga agttaaaatt tggccaaaga aggacctggg caccctggac tgtgggttag     900
   ggtaatattc cttatggaga caatgggcta gggtaaatta cctaaaatgg gtcgataaag     960
   aggggtgttc ccagttggga agtgtaattg aagacggggt caaaaaagaa aatcaaaaaa    1020
   aatttaatta agtcatacac aagtcagctt tcttcgagcc tcatataagt ataagtagtt    1080
   caacgtatta gcactgtacc cagcatctcc gtatcgagaa acacaacaac atgccccatt    1140
   ggacagatca tgcggataca caggttgtgc agtatcatac atactcgatc agacaggtcg    1200
   tctgaccatc atacaagctg aacaagcgct ccatacttgc acgctctcta tatacacagt    1260
   taaattacat atccatagtc taacctctaa cagttaatct tctggtaagc ctcccagcca    1320
   gccttctggt atcgcttggc ctcctcaata ggatctcggt tctggccgta cagacctcgg    1380
   ccgacaatta tgatatccgt tccggtagac atgacatcct caacagttcg gtactgctgt    1440
   ccgagagcgt ctcccttgtc gtcaagaccc accccggggg tcagaataag ccagtcctca    1500
   gagtcgccct taggtcggtt ctgggcaatg aagccaacca caaactcggg gtcggatcgg    1560
   gcaagctcaa tggtctgctt ggagtactcg ccagtggcca gagagccctt gcaagacagc    1620
   tcggccagca tgagcagacc tctggccagc ttctcgttgg gagaggggac taggaactcc    1680
   ttgtactggg agttctcgta gtcagagacg tcctccttct tctgttcaga gacagtttcc    1740
   tcggcaccag ctcgcaggcc agcaatgatt ccggttccgg gtacaccgtg ggcgttggtg    1800
   atatcggacc actcggcgat tcggtgacac cggtactggt gcttgacagt gttgccaata    1860
   tctgcgaact ttctgtcctc gaacaggaag aaaccgtgct taagagcaag ttccttgagg    1920
   gggagcacag tgccggcgta ggtgaagtcg tcaatgatgt cgatatgggt tttgatcatg    1980
   cacacataag gtccgacctt atcggcaagc tcaatgagct ccttggtggt ggtaacatcc    2040
   agagaagcac acaggttggt tttcttggct gccacgagct tgagcactcg agcggcaaag    2100
   gcggacttgt ggacgttagc tcgagcttcg taggagggca ttttggtggt gaagaggaga    2160
   ctgaaataaa tttagtctgc agaacttttt atcggaacct tatctggggc agtgaagtat    2220
   atgttatggt aatagttacg agttagttga acttatagat agactggact atacggctat    2280
   cggtccaaat tagaaagaac gtcaatggct ctctgggcgt cgcctttgcc gacaaaaatg     2340
   tgatcatgat gaaagccagc aatgacgttg cagctgatat tgttgtcggc caaccgcgcc     2400
   gaaaacgcag ctgtcagacc cacagcctcc aacgaagaat gtatcgtcaa agtgatccaa     2460
   gcacactcat agttggagtc gtactccaaa ggcggcaatg acgagtcaga cagatactcg     2520
   tcgacgttta aaccatcatc taagggcctc aaaactacct cggaactgct gcgctgatct     2580
   ggacaccaca gaggttccga gcactttagg ttgcaccaaa tgtcccacca ggtgcaggca     2640
   gaaaacgctg gaacagcgtg tacagtttgt cttaacaaaa agtgagggcg ctgaggtcga     2700
   gcagggtggt gtgacttgtt atagccttta gagctgcgaa agcgcgtatg gatttggctc 2760
   atcaggccag attgagggtc tgtggacaca tgtcatgtta gtgtacttca atcgccccct 2820
   ggatatagcc ccgacaatag gccgtggcct catttttttg ccttccgcac atttccattg     2880
   ctcggtaccc acaccttgct tctcctgcac ttgccaacct taatactggt ttacattgac     2940
   caacatctta caagcggggg gcttgtctag ggtatatata aacagtggct ctcccaatcg     3000
   gttgccagtc tcttttttcc tttctttccc cacagattcg aaatctaaac tacacatcac     3060
   accatggaca agaaatactc catcggcctg gacattggaa ccaactctgt cggctgggct     3120
   gtcatcaccg acgagtacaa ggtgccctcc aagaaattca aggtcctcgg aaacaccgat     3180
   cgacactcca tcaagaaaaa cctcattggt gccctgttgt tcgattctgg cgagactgcc     3240
   gaagctacca gactcaagcg aactgctcgg cgacgttaca cccgacggaa gaaccgaatc     3300
   tgctacctgc aggagatctt ttccaacgag atggccaagg tggacgattc gttctttcat     3360
   cgactggagg aatccttcct cgtcgaggaa gacaagaaac acgagcgtca tcccatcttt     3420
   ggcaacattg tggacgaggt tgcttaccac gagaagtatc ctaccatcta ccatctccga     3480
   aagaaactcg tcgattccac cgacaaggcg gatctcagac ttatctacct cgctctggca     3540
   cacatgatca agtttcgagg tcatttcctc atcgagggcg atctcaatcc cgacaacagc     3600
   gatgtggaca agctgttcat tcagctcgtt cagacctaca accagctgtt cgaggaaaac     3660
   cccatcaatg cctccggagt cgatgcaaag gccatcttgt ctgctcgact ctcgaagagc     3720
   agacgactgg agaacctcat tgcccaactt cctggcgaga aaaagaacgg actgtttggc     3780
   aacctcattg ccctttctct tggtctcaca cccaacttca agtccaactt cgatctggcg     3840
   gaggacgcca agctccagct gtccaaggac acctacgacg atgacctcga caacctgctt     3900
   gcacagattg gcgatcagta cgccgacctg tttctcgctg ccaagaacct ttcggatgct     3960
   attctcttgt ctgacattct gcgagtcaac accgagatca caaaggctcc cctttctgcc     4020
   tccatgatca agcgatacga cgagcaccat caggatctca cactgctcaa ggctcttgtc     4080
   cgacagcaac tgcccgagaa gtacaaggag atctttttcg atcagtcgaa gaacggctac     4140
   gctggataca tcgacggcgg agcctctcag gaagagttct acaagttcat caagccaatt     4200
   ctcgagaaga tggacggaac cgaggaactg cttgtcaagc tcaatcgaga ggatctgctt     4260
   cggaagcaac gaaccttcga caacggcagc attcctcatc agatccacct cggtgagctg     4320
   cacgccattc ttcgacgtca ggaagacttc tacccctttc tcaaggacaa ccgagagaag     4380
   atcgagaaga ttcttacctt tcgaatcccc tactatgttg gtcctcttgc cagaggaaac     4440
   tctcgatttg cttggatgac tcgaaagtcc gaggaaacca tcactccctg gaacttcgag     4500
   gaagtcgtgg acaagggtgc ctctgcacag tccttcatcg agcgaatgac caacttcgac     4560
   aagaatctgc ccaacgagaa ggttcttccc aagcattcgc tgctctacga gtactttaca     4620
   gtctacaacg aactcaccaa agtcaagtac gttaccgagg gaatgcgaaa gcctgccttc     4680
   ttgtctggcg aacagaagaa agccattgtc gatctcctgt tcaagaccaa ccgaaaggtc     4740
   actgttaagc agctcaagga ggactacttc aagaaaatcg agtgtttcga cagcgtcgag     4800
   atttccggag ttgaggaccg attcaacgcc tctttgggca cctatcacga tctgctcaag     4860
   attatcaagg acaaggattt tctcgacaac gaggaaaacg aggacattct ggaggacatc     4920
   gtgctcactc ttaccctgtt cgaagatcgg gagatgatcg aggaacgact caagacatac     4980
   gctcacctgt tcgacgacaa ggtcatgaaa caactcaagc gacgtagata caccggctgg     5040
   ggaagacttt cgcgaaagct catcaacggc atcagagaca agcagtccgg aaagaccatt     5100
   ctggactttc tcaagtccga tggctttgcc aaccgaaact tcatgcagct cattcacgac     5160
   gattctctta ccttcaagga ggacatccag aaggcacaag tgtccggtca gggcgacagc     5220
   ttgcacgaac atattgccaa cctggctggt tcgccagcca tcaagaaagg cattctccag     5280
   actgtcaagg ttgtcgacga gctggtgaag gtcatgggac gtcacaagcc cgagaacatt     5340
   gtgatcgaga tggccagaga gaaccagaca actcaaaagg gtcagaaaaa ctcgcgagag     5400
   cggatgaagc gaatcgagga aggcatcaag gagctgggat cccagattct caaggagcat     5460
   cccgtcgaga acactcaact gcagaacgag aagctgtatc tctactatct gcagaatggt     5520
   cgagacatgt acgtggatca ggaactggac atcaatcgtc tcagcgacta cgatgtggac     5580
   cacattgtcc ctcaatcctt tctcaaggac gattctatcg acaacaaggt ccttacacga     5640
   tccgacaaga acagaggcaa gtcggacaac gttcccagcg aagaggtggt caaaaagatg     5700
   aagaactact ggcgacagct gctcaacgcc aagctcatta cccagcgaaa gttcgacaat     5760
   cttaccaagg ccgagcgagg cggtctgtcc gagctcgaca aggctggctt catcaagcgt     5820
   caactcgtcg agaccagaca gatcacaaag cacgtcgcac agattctcga ttctcggatg     5880
   aacaccaagt acgacgagaa cgacaagctc atccgagagg tcaaggtgat tactctcaag     5940
   tccaaactgg tctccgattt ccgaaaggac tttcagttct acaaggtgcg agagatcaac     6000
   aattaccacc atgcccacga tgcttacctc aacgccgtcg ttggcactgc gctcatcaag     6060
   aaatacccca agctcgaaag cgagttcgtt tacggcgatt acaaggtcta cgacgttcga 6120
   aagatgattg ccaagtccga acaggagatt ggcaaggcta ctgccaagta cttcttttac 6180
   tccaacatca tgaacttttt caagaccgag atcaccttgg ccaacggaga gattcgaaag 6240
   agaccactta tcgagaccaa cggcgaaact ggagagatcg tgtgggacaa gggtcgagac 6300
   tttgcaaccg tgcgaaaggt tctgtcgatg cctcaggtca acatcgtcaa gaaaaccgag 6360
   gttcagactg gcggattctc caaggagtcg attctgccca agcgaaactc cgacaagctc 6420
   atcgctcgaa agaaagactg ggatcccaag aaatacggtg gcttcgattc tcctaccgtc 6480
   gcctattccg tgcttgtcgt tgcgaaggtc gagaagggca agtccaaaaa gctcaagtcc 6540
   gtcaaggagc tgctcggaat taccatcatg gagcgatcga gcttcgagaa gaatcccatc 6600
   gacttcttgg aagccaaggg ttacaaggag gtcaagaaag acctcattat caagctgccc 6660
   aagtactctc tgttcgaact ggagaacggt cgaaagcgta tgctcgcctc cgctggcgag 6720
   ctgcagaagg gaaacgagct tgccttgcct tcgaagtacg tcaactttct ctatctggct 6780
   tctcactacg agaagctcaa gggttctccc gaggacaacg aacagaagca actcttcgtt 6840
   gagcagcaca aacattacct cgacgagatt atcgagcaga tttccgagtt ttcgaagcga 6900
   gtcatcctgg ctgatgccaa cttggacaag gtgctctctg cctacaacaa gcatcgggac 6960
   aaacccattc gagaacaggc ggagaacatc attcacctgt ttactcttac caacctgggt 7020
   gctcctgcag ctttcaagta cttcgatacc actatcgacc gaaagcggta cacatccacc 7080
   aaggaggttc tcgatgccac cctgattcac cagtccatca ctggcctgta cgagacccga 7140
   atcgacctgt ctcagcttgg tggcgactcc agagccgatc ccaagaaaaa gcgaaaggtc 7200
   taagcggccg caagtgtgga tggggaagtg agtgcccggt tctgtgtgca caattggcaa 7260
   tccaagatgg atggattcaa cacagggata tagcgagcta cgtggtggtg cgaggatata 7320
   gcaacggata tttatgtttg acacttgaga atgtacgata caagcactgt ccaagtacaa 7380
   tactaaacat actgtacata ctcatactcg tacccgggca acggtttcac ttgagtgcag 7440
   tggctagtgc tcttactcgt acagtgtgca atactgcgta tcatagtctt tgatgtatat 7500
   cgtattcatt catgttagtt gcgtacgagc cggaagcata aagtgtaaag cctggggtgc 7560
   ctaatgagtg agctaactca cattaattgc gttgcgctca ctgcccgctt tccagtcggg 7620
   aaacctgtcg tgccagctgc attaatgaat cggccaacgc gcggggagag gcggtttgcg 7680
   tattgggcgc tcttccgctt cctcgctcac tgactcgctg cgctcggtcg ttcggctgcg 7740
   gcgagcggta tcagctcact caaaggcggt aatacggtta tccacagaat caggggataa 7800
   cgcaggaaag aacatgtgag caaaaggcca gcaaaaggcc aggaaccgta aaaaggccgc 7860
   gttgctggcg tttttccata ggctccgccc ccctgacgag catcacaaaa atcgacgctc 7920
   aagtcagagg tggcgaaacc cgacaggact ataaagatac caggcgtttc cccctggaag 7980
   ctccctcgtg cgctctcctg ttccgaccct gccgcttacc ggatacctgt ccgcctttct 8040
   cccttcggga agcgtggcgc tttctcatag ctcacgctgt aggtatctca gttcggtgta 8100
   ggtcgttcgc tccaagctgg gctgtgtgca cgaacccccc gttcagcccg accgctgcgc 8160
   cttatccggt aactatcgtc ttgagtccaa cccggtaaga cacgacttat cgccactggc 8220
   agcagccact ggtaacagga ttagcagagc gaggtatgta ggcggtgcta cagagttctt 8280
   gaagtggtgg cctaactacg gctacactag aaggacagta tttggtatct gcgctctgct 8340
   gaagccagtt accttcggaa aaagagttgg tagctcttga tccggcaaac aaaccaccgc 8400
   tggtagcggt ggtttttttg tttgcaagca gcagattacg cgcagaaaaa aaggatctca 8460
   agaagatcct ttgatctttt ctacggggtc tgacgctcag tggaacgaaa actcacgtta 8520
   agggattttg gtcatgagat tatcaaaaag gatcttcacc tagatccttt taaattaaaa 8580
   atgaagtttt aaatcaatct aaagtatata tgagtaaact tggtctgaca gttaccaatg 8640
   cttaatcagt gaggcaccta tctcagcgat ctgtctattt cgttcatcca tagttgcctg 8700
   actccccgtc gtgtagataa ctacgatacg ggagggctta ccatctggcc ccagtgctgc 8760
   aatgataccg cgagacccac gctcaccggc tccagattta tcagcaataa accagccagc 8820
   cggaagggcc gagcgcagaa gtggtcctgc aactttatcc gcctccatcc agtctattaa 8880
   ttgttgccgg gaagctagag taagtagttc gccagttaat agtttgcgca acgttgttgc 8940
   cattgctaca ggcatcgtgg tgtcacgctc gtcgtttggt atggcttcat tcagctccgg 9000
   ttcccaacga tcaaggcgag ttacatgatc ccccatgttg tgcaaaaaag cggttagctc 9060
   cttcggtcct ccgatcgttg tcagaagtaa gttggccgca gtgttatcac tcatggttat 9120
   ggcagcactg cataattctc ttactgtcat gccatccgta agatgctttt ctgtgactgg 9180
   tgagtactca accaagtcat tctgagaata gtgtatgcgg cgaccgagtt gctcttgccc 9240
   ggcgtcaata cgggataata ccgcgccaca tagcagaact ttaaaagtgc tcatcattgg 9300
   aaaacgttct tcggggcgaa aactctcaag gatcttaccg ctgttgagat ccagttcgat 9360
   gtaacccact cgtgcaccca actgatcttc agcatctttt actttcacca gcgtttctgg 9420
   gtgagcaaaa acaggaaggc aaaatgccgc aaaaaaggga ataagggcga cacggaaatg 9480
   ttgaatactc atactcttcc tttttcaata ttattgaagc atttatcagg gttattgtct 9540
   catgagcgga tacatatttg aatgtattta gaaaaataaa caaatagggg ttccgcgcac 9600
   atttccccga aaagtgccac ctgacgcgcc ctgtagcggc gcattaagcg cggcgggtgt 9660
   ggtggttacg cgcagcgtga ccgctacact tgccagcgcc ctagcgcccg ctcctttcgc 9720
   tttcttccct tcctttctcg ccacgttcgc cggctttccc cgtcaagctc taaatcgggg 9780
   gctcccttta gggttccgat ttagtgcttt acggcacctc gaccccaaaa aacttgatta 9840
   gggtgatggt tcacgtagtg ggccatcgcc ctgatagacg gtttttcgcc ctttgacgtt     9900
   ggagtccacg ttctttaata gtggactctt gttccaaact ggaacaacac tcaaccctat     9960
   ctcggtctat tcttttgatt tataagggat tttgccgatt tcggcctatt ggttaaaaaa    10020
   tgagctgatt taacaaaaat ttaacgcgaa ttttaacaaa atattaacgc ttacaatttc    10080
   cattcgccat tcaggctgcg caactgttgg gaagggcgat cggtgcgggc ctcttcgcta    10140
   ttacgccagc tggcgaaagg gggatgtgct gcaaggcgat taagttgggt aacgccaggg    10200
   ttttcccagt cacgacgttg taaaacgacg gccagtgaat tgtaatacga ctcactatag    10260
   ggcgaattgg gtaccgggcc ccccctcgag gtcgatggtg tcgataagct tgatatcgaa    10320
   ttcatgtcac acaaaccgat cttcgcctca aggaaaccta attctacatc cgagagactg    10380
   ccgagatcca gtctacactg attaattttc gggccaataa tttaaaaaaa tcgtgttata    10440
   taatattata tgtattatat atatacatca tgatgatact gacagtcatg tcccattgct    10500
   aaatagacag actccatctg ccgcctccaa ctgatgttct caatatttaa ggggtcatct    10560
   cgcattgttt aataataaac agactccatc taccgcctcc aaatgatgtt ctcaaaatat    10620
   attgtatgaa cttattttta ttacttagta ttattagaca acttacttgc tttatgaaaa    10680
   acacttccta tttaggaaac aatttataat ggcagttcgt tcatttaaca atttatgtag    10740
   aataaatgtt ataaatgcgt atgggaaatc ttaaatatgg atagcataaa tgatatctgc    10800
   attgcctaat tcgaaatcaa cagcaacgaa aaaaatccct tgtacaacat aaatagtcat    10860
   cgagaaatat caactatcaa agaacagcta ttcacacgtt actattgaga ttattattgg    10920
   acgagaatca cacactcaac tgtctttctc tcttctagaa atacaggtac aagtatgtac    10980
   tattctcatt gttcatactt ctagtcattt catcccacat attccttgga tttctctcca    11040
   atgaatgaca ttctatcttg caaattcaac aattataata agatatacca aagtagcggt    11100
   atagtggcaa tcaaaaagct tctctggtgt gcttctcgta tttattttta ttctaatgat    11160
   ccattaaagg tatatattta tttcttgtta tataatcctt ttgtttatta catgggctgg    11220
   atacataaag gtattttgat ttaatttttt gcttaaattc aatcccccct cgttcagtgt    11280
   caactgtaat ggtaggaaat taccatactt ttgaagaagc aaaaaaaatg aaagaaaaaa    11340
   aaaatcgtat ttccaggtta gacgttccgc agaatctaga atgcggtatg cggtacattg    11400
   ttcttcgaac gtaaaagttg cgctccctga gatattgtac atttttgctt ttacaagtac    11460
   aagtacatcg tacaactatg tactactgtt gatgcatcca caacagtttg ttttgttttt    11520
   ttttgttttt tttttttcta atgattcatt accgctatgt atacctactt gtacttgtag    11580
   taagccgggt tattggcgtt caattaatca tagacttatg aatctgcacg gtgtgcgctg    11640
   cgagttactt ttagcttatg catgctactt gggtgtaata ttgggatctg ttcggaaatc    11700
   aacggatgct caat                                                      11714
<210> 19
   <211> 32
   <212> DNA
   <213> Artificial sequence
<220>
   <223> Can1-1F
<400> 19
   aatgggactc aaacgattac ccaccctcgt tt 32
<210> 20
   <211> 32
   <212> DNA
   <213> Artificial sequence
<220>
   <223> Can1-1R
<400> 20
   tctaaaacga gggtgggtaa tcgtttgagt cc 32
<210> 21
   <211> 20
   <212> DNA
   <213> Artificial sequence
<220>
   <223> DNA encoding Can1-1 VT domain
<400> 21
   tcaaacgatt acccaccctc 20
<210> 22
   <211> 23
   <212> DNA
   <213> Yarrowia lipolytica
<400> 22
   tcaaacgatt acccaccctc cgg 23
<210> 23
   <211> 1719
   <212> DNA
   <213> Yarrowia lipolytica
<400> 23
   atggaaaaga cattttcaaa cgattaccca ccctccggga ctgaggccca catccacatc      60
   aaccacacgg cccactcgga tgactcagag gaggtgccct cgcacaagga aaattacaac     120
   accagtggcc acgacctgga ggagtccgac ccggataacc atgtcggtga gaccctcgag     180
   gtcaagcgag gtctcaagat gcgacacatc tccatgatct cgcttggagg aaccattggt     240
   accggtctct tcattggtac cggaggagct ctccagcagg ccggtccctg tggcgccctc     300
   gtcgcctacg tgttcatggc caccattgtc tactctgttg ccgagtctct tggagaactg     360
   gctacgtaca ttcccatcac cggctccttt gccgtcttta ctacccgata tctgtcacag     420
   tcgtttggtg cctccatggg ctggctatac tggttctcgt gggcgatcac cttcgccatc     480
   gagctcaaca ccattggtcc cgtgattgag tactggactg acgccgttcc tactgctgcc     540
   tggattgcca tcttcttcgt catcctcact accatcaact tcttccccgt gggcttctat     600
   ggcgaagtcg agttctgggt ggcctccgtg aaggtcattg ccatcattgg atggctcatc     660
   tacgcgctct gcatgacgtg tggagcaggt gtaacaggtc ctgtgggatt cagatactgg     720
   aaccaccccg gacccatggg agacggaatc tggaccgacg gcgtgcccat tgtgcgaaac     780
   gcgcccggtc gacgattcat gggatggctc aattcgctcg ttaacgccgc cttcacctac     840
   cagggctgtg agctggtcgg agtcactgcc ggtgaggccc agaaccccag aaagtccgtc     900
   cctcgagcca tcaaccgagt ctttgctcga atttgcatct tctacattgg ctctatcttc     960
   ttcatgggca tgctcgtgcc ctttaacgac cccaagctga ccgatgactc ctccgtcatc    1020
   gcctcctctc cttttgttat tgccattatc aactctggca ccaaggtgct ccctcacatt    1080
   ttcaacgccg tcattctcat caccctgatt tcggcaggaa actccaacgt ctacattggc    1140
   tcgcgagtgg tctacgccct ggctgactcc ggaaccgcac caaagttctt caagcgaacc    1200
   accaagaagg gagtgccgta cgtggcagtc tgcttcacct cggcgtttgg tctgctggcc    1260
   ttcatgtctg tgtccgagtc gtcgtccact gtcttcgact ggttcatcaa catctccgct    1320
   gtggccggcc tcatctgttg ggccttcatc tctgcctccc acatccgatt catgcaagtg    1380
   cttaagcaca gagggatctc cagagatacg ctgcccttca aggcacgatg gcagccattc    1440
   tactcatggt acgcgctcgt ctccatcatc ttcatcactc tcatccaggg cttcacgtcc    1500
   ttctggcact ttaccgccgc caagttcatg actgcataca tctccgtcat tgtctgggtc    1560
   ggtttgtaca ttatcttcca gtgtctgttc cgatgcaagt tccttatccc tattgaggat    1620
   gtggacattg acaccggccg acgagagatt gacgacgatg tgtgggagga gaagatcccc    1680
   acaaagtggt acgagaagtt ttggaatatt attgcataa                           1719
<210> 24
   <211> 11176
   <212> DNA
   <213> Artificial Sequence
<220>
   <223> pRF303
<400> 24
   tctaaaacga gggtgggtaa tcgtttgagt cccattcgcc atgccgaagc atgttgccca       60
   gccggcgcca gcgaggaggc tgggaccatg ccggccatta ttttgcgtta agtttctaat      120
   catcacgaaa ttatctatca aaaataacta ggtcccaccg agattcgaac tcgggacctt      180
   aagatttgca atctcacgcg ctaccgctgt gccataggac cgaagttaaa atttggccaa      240
   agaaggacct gggcaccctg gactgtgggt tagggtaata ttccttatgg agacaatggg      300
   ctagggtaaa ttacctaaaa tgggtcgata aagaggggtg ttcccagttg ggaagtgtaa      360
   ttgaagacgg ggtcaaaaaa gaaaatcaaa aaaaatttaa ttaagtcata cacaagtcag      420
   ctttcttcga gcctcatata agtataagta gttcaacgta ttagcactgt acccagcatc     480
   tccgtatcga gaaacacaac aacatgcccc attggacaga tcatgcggat acacaggttg     540
   tgcagtatca tacatactcg atcagacagg tcgtctgacc atcatacaag ctgaacaagc     600
   gctccatact tgcacgctct ctatatacac agttaaatta catatccata gtctaacctc     660
   taacagttaa tcttctggta agcctcccag ccagccttct ggtatcgctt ggcctcctca     720
   ataggatctc ggttctggcc gtacagacct cggccgacaa ttatgatatc cgttccggta     780
   gacatgacat cctcaacagt tcggtactgc tgtccgagag cgtctccctt gtcgtcaaga     840
   cccaccccgg gggtcagaat aagccagtcc tcagagtcgc ccttaggtcg gttctgggca     900
   atgaagccaa ccacaaactc ggggtcggat cgggcaagct caatggtctg cttggagtac     960
   tcgccagtgg ccagagagcc cttgcaagac agctcggcca gcatgagcag acctctggcc    1020
   agcttctcgt tgggagaggg gactaggaac tccttgtact gggagttctc gtagtcagag    1080
   acgtcctcct tcttctgttc agagacagtt tcctcggcac cagctcgcag gccagcaatg    1140
   attccggttc cgggtacacc gtgggcgttg gtgatatcgg accactcggc gattcggtga    1200
   caccggtact ggtgcttgac agtgttgcca atatctgcga actttctgtc ctcgaacagg    1260
   aagaaaccgt gcttaagagc aagttccttg agggggagca cagtgccggc gtaggtgaag    1320
   tcgtcaatga tgtcgatatg ggttttgatc atgcacacat aaggtccgac cttatcggca    1380
   agctcaatga gctccttggt ggtggtaaca tccagagaag cacacaggtt ggttttcttg     1440
   gctgccacga gcttgagcac tcgagcggca aaggcggact tgtggacgtt agctcgagct    1500
   tcgtaggagg gcattttggt ggtgaagagg agactgaaat aaatttagtc tgcagaactt    1560
   tttatcggaa ccttatctgg ggcagtgaag tatatgttat ggtaatagtt acgagttagt    1620
   tgaacttata gatagactgg actatacggc tatcggtcca aattagaaag aacgtcaatg    1680
   gctctctggg cgtcgccttt gccgacaaaa atgtgatcat gatgaaagcc agcaatgacg    1740
   ttgcagctga tattgttgtc ggccaaccgc gccgaaaacg cagctgtcag acccacagcc    1800
   tccaacgaag aatgtatcgt caaagtgatc caagcacact catagttgga gtcgtactcc    1860
   aaaggcggca atgacgagtc agacagatac tcgtcgacgt ttaaaccatc atctaagggc    1920
   ctcaaaacta cctcggaact gctgcgctga tctggacacc acagaggttc cgagcacttt    1980
   aggttgcacc aaatgtccca ccaggtgcag gcagaaaacg ctggaacagc gtgtacagtt    2040
   tgtcttaaca aaaagtgagg gcgctgaggt cgagcagggt ggtgtgactt gttatagcct    2100
   ttagagctgc gaaagcgcgt atggatttgg ctcatcaggc cagattgagg gtctgtggac    2160
   acatgtcatg ttagtgtact tcaatcgccc cctggatata gccccgacaa taggccgtgg    2220
   cctcattttt ttgccttccg cacatttcca ttgctcggta cccacacctt gcttctcctg    2280
   cacttgccaa ccttaatact ggtttacatt gaccaacatc ttacaagcgg ggggcttgtc    2340
   tagggtatat ataaacagtg gctctcccaa tcggttgcca gtctcttttt tcctttcttt    2400
   ccccacagat tcgaaatcta aactacacat cacaccatgg acaagaaata ctccatcggc    2460
   ctggacattg gaaccaactc tgtcggctgg gctgtcatca ccgacgagta caaggtgccc    2520
   tccaagaaat tcaaggtcct cggaaacacc gatcgacact ccatcaagaa aaacctcatt    2580
   ggtgccctgt tgttcgattc tggcgagact gccgaagcta ccagactcaa gcgaactgct    2640
   cggcgacgtt acacccgacg gaagaaccga atctgctacc tgcaggagat cttttccaac    2700
   gagatggcca aggtggacga ttcgttcttt catcgactgg aggaatcctt cctcgtcgag    2760
   gaagacaaga aacacgagcg tcatcccatc tttggcaaca ttgtggacga ggttgcttac    2820
   cacgagaagt atcctaccat ctaccatctc cgaaagaaac tcgtcgattc caccgacaag    2880
   gcggatctca gacttatcta cctcgctctg gcacacatga tcaagtttcg aggtcatttc    2940
   ctcatcgagg gcgatctcaa tcccgacaac agcgatgtgg acaagctgtt cattcagctc    3000
   gttcagacct acaaccagct gttcgaggaa aaccccatca atgcctccgg agtcgatgca    3060
   aaggccatct tgtctgctcg actctcgaag agcagacgac tggagaacct cattgcccaa    3120
   cttcctggcg agaaaaagaa cggactgttt ggcaacctca ttgccctttc tcttggtctc    3180
   acacccaact tcaagtccaa cttcgatctg gcggaggacg ccaagctcca gctgtccaag    3240
   gacacctacg acgatgacct cgacaacctg cttgcacaga ttggcgatca gtacgccgac    3300
   ctgtttctcg ctgccaagaa cctttcggat gctattctct tgtctgacat tctgcgagtc    3360
   aacaccgaga tcacaaaggc tcccctttct gcctccatga tcaagcgata cgacgagcac    3420
   catcaggatc tcacactgct caaggctctt gtccgacagc aactgcccga gaagtacaag    3480
   gagatctttt tcgatcagtc gaagaacggc tacgctggat acatcgacgg cggagcctct    3540
   caggaagagt tctacaagtt catcaagcca attctcgaga agatggacgg aaccgaggaa    3600
   ctgcttgtca agctcaatcg agaggatctg cttcggaagc aacgaacctt cgacaacggc    3660
   agcattcctc atcagatcca cctcggtgag ctgcacgcca ttcttcgacg tcaggaagac    3720
   ttctacccct ttctcaagga caaccgagag aagatcgaga agattcttac ctttcgaatc    3780
   ccctactatg ttggtcctct tgccagagga aactctcgat ttgcttggat gactcgaaag    3840
   tccgaggaaa ccatcactcc ctggaacttc gaggaagtcg tggacaaggg tgcctctgca    3900
   cagtccttca tcgagcgaat gaccaacttc gacaagaatc tgcccaacga gaaggttctt    3960
   cccaagcatt cgctgctcta cgagtacttt acagtctaca acgaactcac caaagtcaag    4020
   tacgttaccg agggaatgcg aaagcctgcc ttcttgtctg gcgaacagaa gaaagccatt    4080
   gtcgatctcc tgttcaagac caaccgaaag gtcactgtta agcagctcaa ggaggactac    4140
   ttcaagaaaa tcgagtgttt cgacagcgtc gagatttccg gagttgagga ccgattcaac    4200
   gcctctttgg gcacctatca cgatctgctc aagattatca aggacaagga ttttctcgac     4260
   aacgaggaaa acgaggacat tctggaggac atcgtgctca ctcttaccct gttcgaagat     4320
   cgggagatga tcgaggaacg actcaagaca tacgctcacc tgttcgacga caaggtcatg     4380
   aaacaactca agcgacgtag atacaccggc tggggaagac tttcgcgaaa gctcatcaac     4440
   ggcatcagag acaagcagtc cggaaagacc attctggact ttctcaagtc cgatggcttt     4500
   gccaaccgaa acttcatgca gctcattcac gacgattctc ttaccttcaa ggaggacatc     4560
   cagaaggcac aagtgtccgg tcagggcgac agcttgcacg aacatattgc caacctggct     4620
   ggttcgccag ccatcaagaa aggcattctc cagactgtca aggttgtcga cgagctggtg     4680
   aaggtcatgg gacgtcacaa gcccgagaac attgtgatcg agatggccag agagaaccag     4740
   acaactcaaa agggtcagaa aaactcgcga gagcggatga agcgaatcga ggaaggcatc     4800
   aaggagctgg gatcccagat tctcaaggag catcccgtcg agaacactca actgcagaac     4860
   gagaagctgt atctctacta tctgcagaat ggtcgagaca tgtacgtgga tcaggaactg     4920
   gacatcaatc gtctcagcga ctacgatgtg gaccacattg tccctcaatc ctttctcaag     4980
   gacgattcta tcgacaacaa ggtccttaca cgatccgaca agaacagagg caagtcggac     5040
   aacgttccca gcgaagaggt ggtcaaaaag atgaagaact actggcgaca gctgctcaac     5100
   gccaagctca ttacccagcg aaagttcgac aatcttacca aggccgagcg aggcggtctg     5160
   tccgagctcg acaaggctgg cttcatcaag cgtcaactcg tcgagaccag acagatcaca     5220
   aagcacgtcg cacagattct cgattctcgg atgaacacca agtacgacga gaacgacaag     5280
   ctcatccgag aggtcaaggt gattactctc aagtccaaac tggtctccga tttccgaaag     5340
   gactttcagt tctacaaggt gcgagagatc aacaattacc accatgccca cgatgcttac     5400
   ctcaacgccg tcgttggcac tgcgctcatc aagaaatacc ccaagctcga aagcgagttc     5460
   gtttacggcg attacaaggt ctacgacgtt cgaaagatga ttgccaagtc cgaacaggag     5520
   attggcaagg ctactgccaa gtacttcttt tactccaaca tcatgaactt tttcaagacc     5580
   gagatcacct tggccaacgg agagattcga aagagaccac ttatcgagac caacggcgaa     5640
   actggagaga tcgtgtggga caagggtcga gactttgcaa ccgtgcgaaa ggttctgtcg     5700
   atgcctcagg tcaacatcgt caagaaaacc gaggttcaga ctggcggatt ctccaaggag     5760
   tcgattctgc ccaagcgaaa ctccgacaag ctcatcgctc gaaagaaaga ctgggatccc     5820
   aagaaatacg gtggcttcga ttctcctacc gtcgcctatt ccgtgcttgt cgttgcgaag     5880
   gtcgagaagg gcaagtccaa aaagctcaag tccgtcaagg agctgctcgg aattaccatc     5940
   atggagcgat cgagcttcga gaagaatccc atcgacttct tggaagccaa gggttacaag     6000
   gaggtcaaga aagacctcat tatcaagctg cccaagtact ctctgttcga actggagaac     6060
   ggtcgaaagc gtatgctcgc ctccgctggc gagctgcaga agggaaacga gcttgccttg     6120
   ccttcgaagt acgtcaactt tctctatctg gcttctcact acgagaagct caagggttct     6180
   cccgaggaca acgaacagaa gcaactcttc gttgagcagc acaaacatta cctcgacgag     6240
   attatcgagc agatttccga gttttcgaag cgagtcatcc tggctgatgc caacttggac     6300
   aaggtgctct ctgcctacaa caagcatcgg gacaaaccca ttcgagaaca ggcggagaac     6360
   atcattcacc tgtttactct taccaacctg ggtgctcctg cagctttcaa gtacttcgat     6420
   accactatcg accgaaagcg gtacacatcc accaaggagg ttctcgatgc caccctgatt     6480
   caccagtcca tcactggcct gtacgagacc cgaatcgacc tgtctcagct tggtggcgac     6540
   tccagagccg atcccaagaa aaagcgaaag gtctaagcgg ccgcaagtgt ggatggggaa     6600
   gtgagtgccc ggttctgtgt gcacaattgg caatccaaga tggatggatt caacacaggg     6660
   atatagcgag ctacgtggtg gtgcgaggat atagcaacgg atatttatgt ttgacacttg     6720
   agaatgtacg atacaagcac tgtccaagta caatactaaa catactgtac atactcatac     6780
   tcgtacccgg gcaacggttt cacttgagtg cagtggctag tgctcttact cgtacagtgt     6840
   gcaatactgc gtatcatagt ctttgatgta tatcgtattc attcatgtta gttgcgtacg     6900
   agccggaagc ataaagtgta aagcctgggg tgcctaatga gtgagctaac tcacattaat     6960
   tgcgttgcgc tcactgcccg ctttccagtc gggaaacctg tcgtgccagc tgcattaatg     7020
   aatcggccaa cgcgcgggga gaggcggttt gcgtattggg cgctcttccg cttcctcgct     7080
   cactgactcg ctgcgctcgg tcgttcggct gcggcgagcg gtatcagctc actcaaaggc     7140
   ggtaatacgg ttatccacag aatcagggga taacgcagga aagaacatgt gagcaaaagg     7200
   ccagcaaaag gccaggaacc gtaaaaaggc cgcgttgctg gcgtttttcc ataggctccg     7260
   cccccctgac gagcatcaca aaaatcgacg ctcaagtcag aggtggcgaa acccgacagg     7320
   actataaaga taccaggcgt ttccccctgg aagctccctc gtgcgctctc ctgttccgac     7380
   cctgccgctt accggatacc tgtccgcctt tctcccttcg ggaagcgtgg cgctttctca     7440
   tagctcacgc tgtaggtatc tcagttcggt gtaggtcgtt cgctccaagc tgggctgtgt     7500
   gcacgaaccc cccgttcagc ccgaccgctg cgccttatcc ggtaactatc gtcttgagtc     7560
   caacccggta agacacgact tatcgccact ggcagcagcc actggtaaca ggattagcag     7620
   agcgaggtat gtaggcggtg ctacagagtt cttgaagtgg tggcctaact acggctacac     7680
   tagaaggaca gtatttggta tctgcgctct gctgaagcca gttaccttcg gaaaaagagt     7740
   tggtagctct tgatccggca aacaaaccac cgctggtagc ggtggttttt ttgtttgcaa     7800
   gcagcagatt acgcgcagaa aaaaaggatc tcaagaagat cctttgatct tttctacggg     7860
   gtctgacgct cagtggaacg aaaactcacg ttaagggatt ttggtcatga gattatcaaa     7920
   aaggatcttc acctagatcc ttttaaatta aaaatgaagt tttaaatcaa tctaaagtat     7980
   atatgagtaa acttggtctg acagttacca atgcttaatc agtgaggcac ctatctcagc      8040
   gatctgtcta tttcgttcat ccatagttgc ctgactcccc gtcgtgtaga taactacgat      8100
   acgggagggc ttaccatctg gccccagtgc tgcaatgata ccgcgagacc cacgctcacc      8160
   ggctccagat ttatcagcaa taaaccagcc agccggaagg gccgagcgca gaagtggtcc      8220
   tgcaacttta tccgcctcca tccagtctat taattgttgc cgggaagcta gagtaagtag      8280
   ttcgccagtt aatagtttgc gcaacgttgt tgccattgct acaggcatcg tggtgtcacg      8340
   ctcgtcgttt ggtatggctt cattcagctc cggttcccaa cgatcaaggc gagttacatg      8400
   atcccccatg ttgtgcaaaa aagcggttag ctccttcggt cctccgatcg ttgtcagaag      8460
   taagttggcc gcagtgttat cactcatggt tatggcagca ctgcataatt ctcttactgt      8520
   catgccatcc gtaagatgct tttctgtgac tggtgagtac tcaaccaagt cattctgaga      8580
   atagtgtatg cggcgaccga gttgctcttg cccggcgtca atacgggata ataccgcgcc      8640
   acatagcaga actttaaaag tgctcatcat tggaaaacgt tcttcggggc gaaaactctc      8700
   aaggatctta ccgctgttga gatccagttc gatgtaaccc actcgtgcac ccaactgatc      8760
   ttcagcatct tttactttca ccagcgtttc tgggtgagca aaaacaggaa ggcaaaatgc      8820
   cgcaaaaaag ggaataaggg cgacacggaa atgttgaata ctcatactct tcctttttca      8880
   atattattga agcatttatc agggttattg tctcatgagc ggatacatat ttgaatgtat      8940
   ttagaaaaat aaacaaatag gggttccgcg cacatttccc cgaaaagtgc cacctgacgc      9000
   gccctgtagc ggcgcattaa gcgcggcggg tgtggtggtt acgcgcagcg tgaccgctac      9060
   acttgccagc gccctagcgc ccgctccttt cgctttcttc ccttcctttc tcgccacgtt      9120
   cgccggcttt ccccgtcaag ctctaaatcg ggggctccct ttagggttcc gatttagtgc      9180
   tttacggcac ctcgacccca aaaaacttga ttagggtgat ggttcacgta gtgggccatc      9240
   gccctgatag acggtttttc gccctttgac gttggagtcc acgttcttta atagtggact      9300
   cttgttccaa actggaacaa cactcaaccc tatctcggtc tattcttttg atttataagg      9360
   gattttgccg atttcggcct attggttaaa aaatgagctg atttaacaaa aatttaacgc      9420
   gaattttaac aaaatattaa cgcttacaat ttccattcgc cattcaggct gcgcaactgt      9480
   tgggaagggc gatcggtgcg ggcctcttcg ctattacgcc agctggcgaa agggggatgt      9540
   gctgcaaggc gattaagttg ggtaacgcca gggttttccc agtcacgacg ttgtaaaacg      9600
   acggccagtg aattgtaata cgactcacta tagggcgaat tgggtaccgg gccccccctc      9660
   gaggtcgatg gtgtcgataa gcttgatatc gaattcatgt cacacaaacc gatcttcgcc      9720
   tcaaggaaac ctaattctac atccgagaga ctgccgagat ccagtctaca ctgattaatt      9780
   ttcgggccaa taatttaaaa aaatcgtgtt atataatatt atatgtatta tatatataca      9840
   tcatgatgat actgacagtc atgtcccatt gctaaataga cagactccat ctgccgcctc      9900
   caactgatgt tctcaatatt taaggggtca tctcgcattg tttaataata aacagactcc      9960
   atctaccgcc tccaaatgat gttctcaaaa tatattgtat gaacttattt ttattactta     10020
   gtattattag acaacttact tgctttatga aaaacacttc ctatttagga aacaatttat     10080
   aatggcagtt cgttcattta acaatttatg tagaataaat gttataaatg cgtatgggaa     10140
   atcttaaata tggatagcat aaatgatatc tgcattgcct aattcgaaat caacagcaac     10200
   gaaaaaaatc ccttgtacaa cataaatagt catcgagaaa tatcaactat caaagaacag     10260
   ctattcacac gttactattg agattattat tggacgagaa tcacacactc aactgtcttt     10320
   ctctcttcta gaaatacagg tacaagtatg tactattctc attgttcata cttctagtca     10380
   tttcatccca catattcctt ggatttctct ccaatgaatg acattctatc ttgcaaattc     10440
   aacaattata ataagatata ccaaagtagc ggtatagtgg caatcaaaaa gcttctctgg     10500
   tgtgcttctc gtatttattt ttattctaat gatccattaa aggtatatat ttatttcttg     10560
   ttatataatc cttttgttta ttacatgggc tggatacata aaggtatttt gatttaattt     10620
   tttgcttaaa ttcaatcccc cctcgttcag tgtcaactgt aatggtagga aattaccata     10680
   cttttgaaga agcaaaaaaa atgaaagaaa aaaaaaatcg tatttccagg ttagacgttc     10740
   cgcagaatct agaatgcggt atgcggtaca ttgttcttcg aacgtaaaag ttgcgctccc     10800
   tgagatattg tacatttttg cttttacaag tacaagtaca tcgtacaact atgtactact     10860
   gttgatgcat ccacaacagt ttgttttgtt tttttttgtt tttttttttt ctaatgattc     10920
   attaccgcta tgtataccta cttgtacttg tagtaagccg ggttattggc gttcaattaa     10980
   tcatagactt atgaatctgc acggtgtgcg ctgcgagtta cttttagctt atgcatgcta     11040
   cttgggtgta atattgggat ctgttcggaa atcaacggat gctcaatcga taaaaaacaa     11100
   aaaaaaaagc accgactcgg tgccactttt tcaagttgat aacggactag ccttatttta     11160
   acttgctatt tctagc                                                     11176
<210> 25
   <211> 655
   <212> DNA
   <213> Artificial sequence
<220>
   <223> can1 upstream homology arm
<400> 25
   gggaagcctt gctacgttag gagaagacgc acggcgatga tacgggtacc cctcatgaca        60
   tcaatatccg ctgcccctct tgccagcaag gcgtcagcag gtgctttttt cgctattttc       120
   accagaccac agcctttttc cttgtgtctc atcttggatt ccttcaaagg caactcaccg       180
   cacctccgag tcgtgtgaac aatgtaataa taggctattg acttttttcc cacctgttta      240
   gcgccaaacc caaagcgctt ttcgccccca ctgcagcccg atggaaggca catatggcaa       300
   gggaaaagtc ttcaggtaat acatgcctgc tgcaactata tgtactctga ctcattccct       360
   cagacgtggg tcatagacag ctgttttaaa ccgggcaaat caatctctgt cgcacaggta       420
   tttctgccct tcaaaaccag gttgccacat cagattccat caaagttttt cagactaact       480
   tcaatcttaa acggcatctc acaacaagcg aattggacgg aaaaaaagcg tctatcatta       540
   ccggcaccta tccacactaa gacagtacta aaggacgacg ctccccacga aacgacgttt       600
   cgaccttaac gaccctgccg tctccatcca tccgaccact cccgacgctc tctcc             655
<210> 26
   <211> 29
   <212> DNA
   <213> Artificial sequence
<220>
   <223> Can1 upstream forward
<400> 26
   gggaagcttg ctacgttagg agaagacgc 29
<210> 27
   <211> 37
   <212> DNA
   <213> Artificial sequence
<220>
   <223> Can1 upstream reverse
<400> 27
   ggagagagcg tcgggagtgg tcggatggat ggagacg 37
<210> 28
   <211> 658
   <212> DNA
   <213> Artificial sequence
<220>
   <223> Can1 downstream homology arm
<400> 28
   cgtctccatc catccgacca ctcccgacgc tctctcctgg agcaaaccac tcttaccaag      60
   catatagcat atataataac gtattgaatt tattaactga ttgaattgag agtaaagcca     120
   gtagcgttgt acggctgtag ctttttagaa aagtggcaga tgagcgatgg tggatatgaa     180
   agtaccttta cggcatgtag cgacacaaga tcgcttccaa gaactcgaca ttcaagccca     240
   gctcgtacaa gaaaatgaac tagccaatca tatgaactag cacattgaag tcaccgcatc     300
   atctctgttg gaaacgacgc gcatgtactc gtgcgtagta aatccgtatc tgtacactcg     360
   aaagattaca gtatgtagta gtagcatgac taacgatgta acgtccaaat aacgctctgt     420
   gcctactcct gtagatgcat tagaccacct gctaacgtct acacgttatg tccgttagct     480
   ccaagattgc acttttccct caaagactct gctgggttac gtcatggtct ctttcgggtc     540
   tctggtccgt tctctgcccg cccatatccg cccaggctgc tacgatacag gataagctca     600
   taagcttaga ttatttttcc ggaatgacat cacgatgcag tggtggaagg atgtatgg       658
<210> 29
   <211> 37
   <212> DNA
   <213> Artificial sequence
<220>
   <223> Can1 downstream homology arm forward primer
<400> 29
   cgtctccatc catccgacca ctcccgacgc tctctcc 37
<210> 30
   <211> 22
   <212> DNA
   <213> Yarrowia lipolytica
<400> 30
   ccatacatcc ttccaccact gc 22
<210> 31
   <211> 1276
   <212> DNA
   <213> Artificial sequence
<220>
   <223> Can1 editing template clonign fragment
<400> 31
   gggaagcctt gctacgttag gagaagacgc acggcgatga tacgggtacc cctcatgaca      60
   tcaatatccg ctgcccctct tgccagcaag gcgtcagcag gtgctttttt cgctattttc     120
   accagaccac agcctttttc cttgtgtctc atcttggatt ccttcaaagg caactcaccg     180
   cacctccgag tcgtgtgaac aatgtaataa taggctattg acttttttcc cacctgttta    240
   gcgccaaacc caaagcgctt ttcgccccca ctgcagcccg atggaaggca catatggcaa     300
   gggaaaagtc ttcaggtaat acatgcctgc tgcaactata tgtactctga ctcattccct     360
   cagacgtggg tcatagacag ctgttttaaa ccgggcaaat caatctctgt cgcacaggta     420
   tttctgccct tcaaaaccag gttgccacat cagattccat caaagttttt cagactaact     480
   tcaatcttaa acggcatctc acaacaagcg aattggacgg aaaaaaagcg tctatcatta     540
   ccggcaccta tccacactaa gacagtacta aaggacgacg ctccccacga aacgacgttt     600
   cgaccttaac gaccctgccg tctccatcca tccgaccact cccgacgctc tctcctggag     660
   caaaccactc ttaccaagca tatagcatat ataataacgt attgaattta ttaactgatt     720
   gaattgagag taaagccagt agcgttgtac ggctgtagct ttttagaaaa gtggcagatg     780
   agcgatggtg gatatgaaag tacctttacg gcatgtagcg acacaagatc gcttccaaga     840
   actcgacatt caagcccagc tcgtacaaga aaatgaacta gccaatcata tgaactagca     900
   cattgaagtc accgcatcat ctctgttgga aacgacgcgc atgtactcgt gcgtagtaaa     960
   tccgtatctg tacactcgaa agattacagt atgtagtagt agcatgacta acgatgtaac    1020
   gtccaaataa cgctctgtgc ctactcctgt agatgcatta gaccacctgc taacgtctac    1080
   acgttatgtc cgttagctcc aagattgcac ttttccctca aagactctgc tgggttacgt    1140
   catggtctct ttcgggtctc tggtccgttc tctgcccgcc catatccgcc caggctgcta    1200
   cgatacagga taagctcata agcttagatt atttttccgg aatgacatca cgatgcagtg    1260
   gtggaaggat gtatgg                                                    1276
<210> 32
   <211> 2686
   <212> DNA
   <213> Artificial sequence
<220>
   <223> pUC18
<400> 32
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgccaa gcttgcatgc ctgcaggtcg     420
   actctagagg atccccgggt accgagctcg aattcgtaat catggtcata gctgtttcct     480
   gtgtgaaatt gttatccgct cacaattcca cacaacatac gagccggaag cataaagtgt     540
   aaagcctggg gtgcctaatg agtgagctaa ctcacattaa ttgcgttgcg ctcactgccc     600
   gctttccagt cgggaaacct gtcgtgccag ctgcattaat gaatcggcca acgcgcgggg     660
   agaggcggtt tgcgtattgg gcgctcttcc gcttcctcgc tcactgactc gctgcgctcg     720
   gtcgttcggc tgcggcgagc ggtatcagct cactcaaagg cggtaatacg gttatccaca     780
   gaatcagggg ataacgcagg aaagaacatg tgagcaaaag gccagcaaaa ggccaggaac     840
   cgtaaaaagg ccgcgttgct ggcgtttttc cataggctcc gcccccctga cgagcatcac     900
   aaaaatcgac gctcaagtca gaggtggcga aacccgacag gactataaag ataccaggcg     960
   tttccccctg gaagctccct cgtgcgctct cctgttccga ccctgccgct taccggatac    1020
   ctgtccgcct ttctcccttc gggaagcgtg gcgctttctc atagctcacg ctgtaggtat    1080
   ctcagttcgg tgtaggtcgt tcgctccaag ctgggctgtg tgcacgaacc ccccgttcag    1140
   cccgaccgct gcgccttatc cggtaactat cgtcttgagt ccaacccggt aagacacgac    1200
   ttatcgccac tggcagcagc cactggtaac aggattagca gagcgaggta tgtaggcggt    1260
   gctacagagt tcttgaagtg gtggcctaac tacggctaca ctagaaggac agtatttggt    1320
   atctgcgctc tgctgaagcc agttaccttc ggaaaaagag ttggtagctc ttgatccggc    1380
   aaacaaacca ccgctggtag cggtggtttt tttgtttgca agcagcagat tacgcgcaga    1440
   aaaaaaggat ctcaagaaga tcctttgatc ttttctacgg ggtctgacgc tcagtggaac    1500
   gaaaactcac gttaagggat tttggtcatg agattatcaa aaaggatctt cacctagatc    1560
   cttttaaatt aaaaatgaag ttttaaatca atctaaagta tatatgagta aacttggtct    1620
   gacagttacc aatgcttaat cagtgaggca cctatctcag cgatctgtct atttcgttca    1680
   tccatagttg cctgactccc cgtcgtgtag ataactacga tacgggaggg cttaccatct    1740
   ggccccagtg ctgcaatgat accgcgagac ccacgctcac cggctccaga tttatcagca    1800
   ataaaccagc cagccggaag ggccgagcgc agaagtggtc ctgcaacttt atccgcctcc    1860
   atccagtcta ttaattgttg ccgggaagct agagtaagta gttcgccagt taatagtttg    1920
   cgcaacgttg ttgccattgc tacaggcatc gtggtgtcac gctcgtcgtt tggtatggct    1980
   tcattcagct ccggttccca acgatcaagg cgagttacat gatcccccat gttgtgcaaa    2040
   aaagcggtta gctccttcgg tcctccgatc gttgtcagaa gtaagttggc cgcagtgtta    2100
   tcactcatgg ttatggcagc actgcataat tctcttactg tcatgccatc cgtaagatgc    2160
   ttttctgtga ctggtgagta ctcaaccaag tcattctgag aatagtgtat gcggcgaccg    2220
   agttgctctt gcccggcgtc aatacgggat aataccgcgc cacatagcag aactttaaaa    2280
   gtgctcatca ttggaaaacg ttcttcgggg cgaaaactct caaggatctt accgctgttg    2340
   agatccagtt cgatgtaacc cactcgtgca cccaactgat cttcagcatc ttttactttc    2400
   accagcgttt ctgggtgagc aaaaacagga aggcaaaatg ccgcaaaaaa gggaataagg    2460
   gcgacacgga aatgttgaat actcatactc ttcctttttc aatattattg aagcatttat    2520
   cagggttatt gtctcatgag cggatacata tttgaatgta tttagaaaaa taaacaaata    2580
   ggggttccgc gcacatttcc ccgaaaagtg ccacctgacg tctaagaaac cattattatc    2640
   atgacattaa cctataaaaa taggcgtatc acgaggccct ttcgtc                   2686
<210> 33
   <211> 3901
   <212> DNA
   <213> Artificial sequence
<220>
   <223> pRF80
<400> 33
   agcttgctac gttaggagaa gacgcacggc gatgatacgg gtacccctca tgacatcaat      60
   atccgctgcc cctcttgcca gcaaggcgtc agcaggtgct tttttcgcta ttttcaccag     120
   accacagcct ttttccttgt gtctcatctt ggattccttc aaaggcaact caccgcacct     180
   ccgagtcgtg tgaacaatgt aataataggc tattgacttt tttcccacct gtttagcgcc     240
   aaacccaaag cgcttttcgc ccccactgca gcccgatgga aggcacatat ggcaagggaa     300
   aagtcttcag gtaatacatg cctgctgcaa ctatatgtac tctgactcat tccctcagac     360
   gtgggtcata gacagctgtt ttaaaccggg caaatcaatc tctgtcgcac aggtatttct     420
   gcccttcaaa accaggttgc cacatcagat tccatcaaag tttttcagac taacttcaat     480
   cttaaacggc atctcacaac aagcgaattg gacggaaaaa aagcgtctat cattaccggc     540
   acctatccac actaagacag tactaaagga cgacgctccc cacgaaacga cgtttcgacc     600
   ttaacgaccc tgccgtctcc atccatccga ccactcccga cgctctctcc tggagcaaac     660
   cactcttacc aagcatatag catatataat aacgtattga atttattaac tgattgaatt     720
   gagagtaaag ccagtagcgt tgtacggctg tagcttttta gaaaagtggc agatgagcga     780
   tggtggatat gaaagtacct ttacggcatg tagcgacaca agatcgcttc caagaactcg     840
   acattcaagc ccagctcgta caagaaaatg aactagccaa tcatatgaac tagcacattg     900
   aagtcaccgc atcatctctg ttggaaacga cgcgcatgta ctcgtgcgta gtaaatccgt     960
   atctgtacac tcgaaagatt acagtatgta gtagtagcat gactaacgat gtaacgtcca    1020
   aataacgctc tgtgcctact cctgtagatg cattagacca cctgctaacg tctacacgtt    1080
   atgtccgtta gctccaagat tgcacttttc cctcaaagac tctgctgggt tacgtcatgg      1140
   tctctttcgg gtctctggtc cgttctctgc ccgcccatat ccgcccaggc tgctacgata      1200
   caggataagc tcataagctt gcatgcctgc aggtcgactc tagaggatcc ccgggtaccg      1260
   agctcgaatt cgtaatcatg gtcatagctg tttcctgtgt gaaattgtta tccgctcaca      1320
   attccacaca acatacgagc cggaagcata aagtgtaaag cctggggtgc ctaatgagtg      1380
   agctaactca cattaattgc gttgcgctca ctgcccgctt tccagtcggg aaacctgtcg      1440
   tgccagctgc attaatgaat cggccaacgc gcggggagag gcggtttgcg tattgggcgc      1500
   tcttccgctt cctcgctcac tgactcgctg cgctcggtcg ttcggctgcg gcgagcggta      1560
   tcagctcact caaaggcggt aatacggtta tccacagaat caggggataa cgcaggaaag      1620
   aacatgtgag caaaaggcca gcaaaaggcc aggaaccgta aaaaggccgc gttgctggcg      1680
   tttttccata ggctccgccc ccctgacgag catcacaaaa atcgacgctc aagtcagagg      1740
   tggcgaaacc cgacaggact ataaagatac caggcgtttc cccctggaag ctccctcgtg      1800
   cgctctcctg ttccgaccct gccgcttacc ggatacctgt ccgcctttct cccttcggga      1860
   agcgtggcgc tttctcatag ctcacgctgt aggtatctca gttcggtgta ggtcgttcgc      1920
   tccaagctgg gctgtgtgca cgaacccccc gttcagcccg accgctgcgc cttatccggt      1980
   aactatcgtc ttgagtccaa cccggtaaga cacgacttat cgccactggc agcagccact      2040
   ggtaacagga ttagcagagc gaggtatgta ggcggtgcta cagagttctt gaagtggtgg      2100
   cctaactacg gctacactag aaggacagta tttggtatct gcgctctgct gaagccagtt      2160
   accttcggaa aaagagttgg tagctcttga tccggcaaac aaaccaccgc tggtagcggt      2220
   ggtttttttg tttgcaagca gcagattacg cgcagaaaaa aaggatctca agaagatcct      2280
   ttgatctttt ctacggggtc tgacgctcag tggaacgaaa actcacgtta agggattttg      2340
   gtcatgagat tatcaaaaag gatcttcacc tagatccttt taaattaaaa atgaagtttt      2400
   aaatcaatct aaagtatata tgagtaaact tggtctgaca gttaccaatg cttaatcagt      2460
   gaggcaccta tctcagcgat ctgtctattt cgttcatcca tagttgcctg actccccgtc      2520
   gtgtagataa ctacgatacg ggagggctta ccatctggcc ccagtgctgc aatgataccg      2580
   cgagacccac gctcaccggc tccagattta tcagcaataa accagccagc cggaagggcc      2640
   gagcgcagaa gtggtcctgc aactttatcc gcctccatcc agtctattaa ttgttgccgg      2700
   gaagctagag taagtagttc gccagttaat agtttgcgca acgttgttgc cattgctaca      2760
   ggcatcgtgg tgtcacgctc gtcgtttggt atggcttcat tcagctccgg ttcccaacga      2820
   tcaaggcgag ttacatgatc ccccatgttg tgcaaaaaag cggttagctc cttcggtcct      2880
   ccgatcgttg tcagaagtaa gttggccgca gtgttatcac tcatggttat ggcagcactg      2940
   cataattctc ttactgtcat gccatccgta agatgctttt ctgtgactgg tgagtactca      3000
   accaagtcat tctgagaata gtgtatgcgg cgaccgagtt gctcttgccc ggcgtcaata      3060
   cgggataata ccgcgccaca tagcagaact ttaaaagtgc tcatcattgg aaaacgttct      3120
   tcggggcgaa aactctcaag gatcttaccg ctgttgagat ccagttcgat gtaacccact      3180
   cgtgcaccca actgatcttc agcatctttt actttcacca gcgtttctgg gtgagcaaaa      3240
   acaggaaggc aaaatgccgc aaaaaaggga ataagggcga cacggaaatg ttgaatactc      3300
   atactcttcc tttttcaata ttattgaagc atttatcagg gttattgtct catgagcgga      3360
   tacatatttg aatgtattta gaaaaataaa caaatagggg ttccgcgcac atttccccga      3420
   aaagtgccac ctgacgtcta agaaaccatt attatcatga cattaaccta taaaaatagg      3480
   cgtatcacga ggccctttcg tctcgcgcgt ttcggtgatg acggtgaaaa cctctgacac      3540
   atgcagctcc cggagacggt cacagcttgt ctgtaagcgg atgccgggag cagacaagcc      3600
   cgtcagggcg cgtcagcggg tgttggcggg tgtcggggct ggcttaacta tgcggcatca      3660
   gagcagattg tactgagagt gcaccatatg cggtgtgaaa taccgcacag atgcgtaagg      3720
   agaaaatacc gcatcaggcg ccattcgcca ttcaggctgc gcaactgttg ggaagggcga      3780
   tcggtgcggg cctcttcgct attacgccag ctggcgaaag ggggatgtgc tgcaaggcga      3840
   ttaagttggg taacgccagg gttttcccag tcacgacgtt gtaaaacgac ggccagtgcc      3900
   a                                                                      3901
<210> 34
   <211> 1210
   <212> DNA
   <213> Artificial sequence
<220>
   <223> Can1 polynucleotide modification (editing) template
<400> 34
   gctacgttag gagaagacgc acggcgatga tacgggtacc cctcatgaca tcaatatccg      60
   ctgcccctct tgccagcaag gcgtcagcag gtgctttttt cgctattttc accagaccac     120
   agcctttttc cttgtgtctc atcttggatt ccttcaaagg caactcaccg cacctccgag     180
   tcgtgtgaac aatgtaataa taggctattg acttttttcc cacctgttta gcgccaaacc     240
   caaagcgctt ttcgccccca ctgcagcccg atggaaggca catatggcaa gggaaaagtc     300
   ttcaggtaat acatgcctgc tgcaactata tgtactctga ctcattccct cagacgtggg     360
   tcatagacag ctgttttaaa ccgggcaaat caatctctgt cgcacaggta tttctgccct     420
   tcaaaaccag gttgccacat cagattccat caaagttttt cagactaact tcaatcttaa     480
   acggcatctc acaacaagcg aattggacgg aaaaaaagcg tctatcatta ccggcaccta     540
   tccacactaa gacagtacta aaggacgacg ctccccacga aacgacgttt cgaccttaac     600
   gaccctgccg tctccatcca tccgaccact cccgacgctc tctcctggag caaaccactc     660
   ttaccaagca tatagcatat ataataacgt attgaattta ttaactgatt gaattgagag     720
   taaagccagt agcgttgtac ggctgtagct ttttagaaaa gtggcagatg agcgatggtg     780
   gatatgaaag tacctttacg gcatgtagcg acacaagatc gcttccaaga actcgacatt     840
   caagcccagc tcgtacaaga aaatgaacta gccaatcata tgaactagca cattgaagtc     900
   accgcatcat ctctgttgga aacgacgcgc atgtactcgt gcgtagtaaa tccgtatctg     960
   tacactcgaa agattacagt atgtagtagt agcatgacta acgatgtaac gtccaaataa    1020
   cgctctgtgc ctactcctgt agatgcatta gaccacctgc taacgtctac acgttatgtc    1080
   cgttagctcc aagattgcac ttttccctca aagactctgc tgggttacgt catggtctct    1140
   ttcgggtctc tggtccgttc tctgcccgcc catatccgcc caggctgcta cgatacagga    1200
   taagctcata                                                           1210
<210> 35
   <211> 3719
   <212> DNA
   <213> Artificial sequence
<220>
   <223> CAN1 locus
<400> 35
   tcgtaatttg agtagttttt ggggccctat acctctctca tgttcacttc ctccctcctt      60
   atctaatctc gtccaattac caaagactgc aaagttttgc ttgccaagac agggcgtcat     120
   ccagcctggc tgacagcaga tgttgacagc gatgcataat cgtgagtggg ctccatatgt     180
   aaaatcagta acagggcaga ccgcgtattg gggtcccacc gctgcgccgt ccttctcaga     240
   tgccctatat atgtttgtct cttgtgtgtg gtggctggac ccactctaac tcaacctctg     300
   gctcccagac cttttcagac agactctgct acgagcctcg cgcccccccc atgaaccata     360
   gaatagaata gctacgttag gagaagacgc acggcgatga tacgggtacc cctcatgaca     420
   tcaatatccg ctgcccctct tgccagcaag gcgtcagcag gtgctttttt cgctattttc     480
   accagaccac agcctttttc cttgtgtctc atcttggatt ccttcaaagg caactcaccg     540
   cacctccgag tcgtgtgaac aatgtaataa taggctattg acttttttcc cacctgttta     600
   gcgccaaacc caaagcgctt ttcgccccca ctgcagcccg atggaaggca catatggcaa     660
   gggaaaagtc ttcaggtaat acatgcctgc tgcaactata tgtactctga ctcattccct     720
   cagacgtggg tcatagacag ctgttttaaa ccgggcaaat caatctctgt cgcacaggta     780
   tttctgccct tcaaaaccag gttgccacat cagattccat caaagttttt cagactaact     840
   tcaatcttaa acggcatctc acaacaagcg aattggacgg aaaaaaagcg tctatcatta     900
   ccggcaccta tccacactaa gacagtacta aaggacgacg ctccccacga aacgacgttt     960
   cgaccttaac gaccctgccg tctccatcca tccgaccaca atggaaaaga cattttcaaa    1020
   cgattaccca ccctccggga ctgaggccca catccacatc aaccacacgg cccactcgga    1080
   tgactcagag gaggtgccct cgcacaagga aaattacaac accagtggcc acgacctgga    1140
   ggagtccgac ccggataacc atgtcggtga gaccctcgag gtcaagcgag gtctcaagat    1200
   gcgacacatc tccatgatct cgcttggagg aaccattggt accggtctct tcattggtac    1260
   cggaggagct ctccagcagg ccggtccctg tggcgccctc gtcgcctacg tgttcatggc    1320
   caccattgtc tactctgttg ccgagtctct tggagaactg gctacgtaca ttcccatcac    1380
   cggctccttt gccgtcttta ctacccgata tctgtcacag tcgtttggtg cctccatggg    1440
   ctggctatac tggttctcgt gggcgatcac cttcgccatc gagctcaaca ccattggtcc    1500
   cgtgattgag tactggactg acgccgttcc tactgctgcc tggattgcca tcttcttcgt    1560
   catcctcact accatcaact tcttccccgt gggcttctat ggcgaagtcg agttctgggt    1620
   ggcctccgtg aaggtcattg ccatcattgg atggctcatc tacgcgctct gcatgacgtg    1680
   tggagcaggt gtaacaggtc ctgtgggatt cagatactgg aaccaccccg gacccatggg    1740
   agacggaatc tggaccgacg gcgtgcccat tgtgcgaaac gcgcccggtc gacgattcat    1800
   gggatggctc aattcgctcg ttaacgccgc cttcacctac cagggctgtg agctggtcgg    1860
   agtcactgcc ggtgaggccc agaaccccag aaagtccgtc cctcgagcca tcaaccgagt    1920
   ctttgctcga atttgcatct tctacattgg ctctatcttc ttcatgggca tgctcgtgcc    1980
   ctttaacgac cccaagctga ccgatgactc ctccgtcatc gcctcctctc cttttgttat    2040
   tgccattatc aactctggca ccaaggtgct ccctcacatt ttcaacgccg tcattctcat    2100
   caccctgatt tcggcaggaa actccaacgt ctacattggc tcgcgagtgg tctacgccct    2160
   ggctgactcc ggaaccgcac caaagttctt caagcgaacc accaagaagg gagtgccgta    2220
   cgtggcagtc tgcttcacct cggcgtttgg tctgctggcc ttcatgtctg tgtccgagtc      2280
   gtcgtccact gtcttcgact ggttcatcaa catctccgct gtggccggcc tcatctgttg      2340
   ggccttcatc tctgcctccc acatccgatt catgcaagtg cttaagcaca gagggatctc      2400
   cagagatacg ctgcccttca aggcacgatg gcagccattc tactcatggt acgcgctcgt      2460
   ctccatcatc ttcatcactc tcatccaggg cttcacgtcc ttctggcact ttaccgccgc      2520
   caagttcatg actgcataca tctccgtcat tgtctgggtc ggtttgtaca ttatcttcca      2580
   gtgtctgttc cgatgcaagt tccttatccc tattgaggat gtggacattg acaccggccg      2640
   acgagagatt gacgacgatg tgtgggagga gaagatcccc acaaagtggt acgagaagtt      2700
   ttggaatatt attgcataag aagatcgggg attcccgacg ctctctcctg gagcaaacca      2760
   ctcttaccaa gcatatagca tatataataa cgtattgaat ttattaactg attgaattga      2820
   gagtaaagcc agtagcgttg tacggctgta gctttttaga aaagtggcag atgagcgatg      2880
   gtggatatga aagtaccttt acggcatgta gcgacacaag atcgcttcca agaactcgac      2940
   attcaagccc agctcgtaca agaaaatgaa ctagccaatc atatgaacta gcacattgaa      3000
   gtcaccgcat catctctgtt ggaaacgacg cgcatgtact cgtgcgtagt aaatccgtat      3060
   ctgtacactc gaaagattac agtatgtagt agtagcatga ctaacgatgt aacgtccaaa      3120
   taacgctctg tgcctactcc tgtagatgca ttagaccacc tgctaacgtc tacacgttat      3180
   gtccgttagc tccaagattg cacttttccc tcaaagactc tgctgggtta cgtcatggtc      3240
   tctttcgggt ctctggtccg ttctctgccc gcccatatcc gcccaggctg ctacgataca      3300
   ggataagctc ataagcttag attatttttc cggaatgaca tcacgatgca gtggtggaag      3360
   gatgtatggg gaatgctttt gacgacgcaa tatttaccga aaagctctga aaactcataa      3420
   agtagacatc gatggttcta gtagtgatta tgaacatttt caaccaaatt tgagccttgg      3480
   atctcgagag agaagaacag tacaagtacc gtagtaaatc actcatggat ataaccaaac      3540
   gttaatttcc aaattcatca atctcgttta cttcagtcat cactactgta acggtgcttg      3600
   aatcgagtgc gtaaaacatg tcgaatgaag aacgaagaaa cgacctttac aacatttgcg      3660
   tgcttatatt acgtcttccg acgacactgt agatgtaccg cccgcttcgt actgtatcc       3719
<210> 36
   <211> 20
   <212> DNA
   <213> Artificial sequence
<220>
   <223> editing template forward primer
<400> 36
   agcttgctac gttaggagaa 20
<210> 37
   <211> 20
   <212> DNA
   <213> Artificial sequence
<220>
   <223> editing template reverse primer
<400> 37
   tatgagctta tcctgtatcg 20
<210> 38
   <211> 20
   <212> DNA
   <213> Artificial sequence
<220>
   <223> Can1 locus Forward
<400> 38
   ggaaggcaca tatggcaagg 20
<210> 39
   <211> 21
   <212> DNA
   <213> Artificial sequence
<220>
   <223> Can1 locus reverse
<400> 39
   gtaagagtgg tttgctccag g 21
<210> 40
   <211> 2125
   <212> DNA
   <213> Yarrowia lipolytica
<400> 40
   ggaaggcaca tatggcaagg gaaaagtctt caggtaatac atgcctgctg caactatatg      60
   tactctgact cattccctca gacgtgggtc atagacagct gttttaaacc gggcaaatca     120
   atctctgtcg cacaggtatt tctgcccttc aaaaccaggt tgccacatca gattccatca     180
   aagtttttca gactaacttc aatcttaaac ggcatctcac aacaagcgaa ttggacggaa     240
   aaaaagcgtc tatcattacc ggcacctatc cacactaaga cagtactaaa ggacgacgct     300
   ccccacgaaa cgacgtttcg accttaacga ccctgccgtc tccatccatc cgaccacaat     360
   ggaaaagaca ttttcaaacg attacccacc ctccgggact gaggcccaca tccacatcaa     420
   ccacacggcc cactcggatg actcagagga ggtgccctcg cacaaggaaa attacaacac     480
   cagtggccac gacctggagg agtccgaccc ggataaccat gtcggtgaga ccctcgaggt     540
   caagcgaggt ctcaagatgc gacacatctc catgatctcg cttggaggaa ccattggtac     600
   cggtctcttc attggtaccg gaggagctct ccagcaggcc ggtccctgtg gcgccctcgt     660
   cgcctacgtg ttcatggcca ccattgtcta ctctgttgcc gagtctcttg gagaactggc     720
   tacgtacatt cccatcaccg gctcctttgc cgtctttact acccgatatc tgtcacagtc     780
   gtttggtgcc tccatgggct ggctatactg gttctcgtgg gcgatcacct tcgccatcga     840
   gctcaacacc attggtcccg tgattgagta ctggactgac gccgttccta ctgctgcctg     900
   gattgccatc ttcttcgtca tcctcactac catcaacttc ttccccgtgg gcttctatgg     960
   cgaagtcgag ttctgggtgg cctccgtgaa ggtcattgcc atcattggat ggctcatcta    1020
   cgcgctctgc atgacgtgtg gagcaggtgt aacaggtcct gtgggattca gatactggaa    1080
   ccaccccgga cccatgggag acggaatctg gaccgacggc gtgcccattg tgcgaaacgc    1140
   gcccggtcga cgattcatgg gatggctcaa ttcgctcgtt aacgccgcct tcacctacca    1200
   gggctgtgag ctggtcggag tcactgccgg tgaggcccag aaccccagaa agtccgtccc    1260
   tcgagccatc aaccgagtct ttgctcgaat ttgcatcttc tacattggct ctatcttctt    1320
   catgggcatg ctcgtgccct ttaacgaccc caagctgacc gatgactcct ccgtcatcgc    1380
   ctcctctcct tttgttattg ccattatcaa ctctggcacc aaggtgctcc ctcacatttt    1440
   caacgccgtc attctcatca ccctgatttc ggcaggaaac tccaacgtct acattggctc    1500
   gcgagtggtc tacgccctgg ctgactccgg aaccgcacca aagttcttca agcgaaccac    1560
   caagaaggga gtgccgtacg tggcagtctg cttcacctcg gcgtttggtc tgctggcctt    1620
   catgtctgtg tccgagtcgt cgtccactgt cttcgactgg ttcatcaaca tctccgctgt    1680
   ggccggcctc atctgttggg ccttcatctc tgcctcccac atccgattca tgcaagtgct    1740
   taagcacaga gggatctcca gagatacgct gcccttcaag gcacgatggc agccattcta    1800
   ctcatggtac gcgctcgtct ccatcatctt catcactctc atccagggct tcacgtcctt    1860
   ctggcacttt accgccgcca agttcatgac tgcatacatc tccgtcattg tctgggtcgg    1920
   tttgtacatt atcttccagt gtctgttccg atgcaagttc cttatcccta ttgaggatgt    1980
   ggacattgac accggccgac gagagattga cgacgatgtg tgggaggaga agatccccac    2040
   aaagtggtac gagaagtttt ggaatattat tgcataagaa gatcggggat tcccgacgct    2100
   ctctcctgga gcaaaccact cttac                                          2125
<210> 41
   <211> 392
   <212> DNA
   <213> Artificial sequence
<220>
   <223> Can1 locus deletion
<400> 41
   ggaaggcaca tatggcaagg gaaaagtctt caggtaatac atgcctgctg caactatatg      60
   tactctgact cattccctca gacgtgggtc atagacagct gttttaaacc gggcaaatca     120
   atctctgtcg cacaggtatt tctgcccttc aaaaccaggt tgccacatca gattccatca     180
   aagtttttca gactaacttc aatcttaaac ggcatctcac aacaagcgaa ttggacggaa     240
   aaaaagcgtc tatcattacc ggcacctatc cacactaaga cagtactaaa ggacgacgct     300
   ccccacgaaa cgacgtttcg accttaacga ccctgccgtc tccatccatc cgaccactcc     360
   cgacgctctc tcctggagca aaccactctt ac                                   392
<210> 42
   <211> 62
   <212> DNA
   <213> Artificial sequence
<220>
   <223> Copy number analysis fragment
<400> 42
   agcgccaaac ccaaagcgct tttcgccccc actgcagccc gatggaaggc acatatggca      60
   ag                                                                    62
<210> 43
   <211> 17
   <212> DNA
   <213> Artificial sequence
<220>
   <223> Can1 copy number F
<400> 43
   agcgccaaac ccaaagc 17
<210> 44
   <211> 20
   <212> DNA
   <213> Artificial sequence
<220>
   <223> Can1 copy number R
<400> 44
   cttgccatat gtgccttcca 20
<210> 45
   <211> 21
   <212> DNA
   <213> Artificial sequence
<220>
   <223> Can1 copy number probe
<400> 45
   cttttcgccc ccactgcagc c 21
<210> 46
   <211> 69
   <212> DNA
   <213> Yarrowia lipolytica
<400> 46
   tgaccgtcct tggagatacc agcctcgaac tcaccaacac caccagcaat gatgaggatg 60
   gcacagtcg 69
<210> 47
   <211> 20
   <212> DNA
   <213> Artificial sequence
<220>
   <223> TEF1 forward
<400> 47
   cgactgtgcc atcctcatca 20
<210> 48
   <211> 21
   <212> DNA
   <213> Artificial sequence
<220>
   <223> TEF1 reverse
<400> 48
   tgaccgtcct tggagatacc a 21
<210> 49
   <211> 21
   <212> DNA
   <213> Artificial sequence
<220>
   <223> TEF1 probe
<400> 49
   tgctggtggt gttggtgagt t 21